# FASCICULE DE BREVET EUROPEEN

(11) **EP 0 986 759 B1**
(45) Date de publication et mention de la délivrance du brevet: **26.04.2006**
(21) Numéro de dépôt: 98929488.9
(22) Date de dépôt: 04.06.1998
(51) Int. Cl.: G01N 33/58

(54) **UTILISATION D' UNE PROTEINE FLUORESCENTE POUR LA DETECTION DE L' INTERACTION ENTRE UN RECEPTEUR MEMBRANAIRE ET SON LIGAND**
VERWENDUNG EINES FLUORESZENZPROTEINS ZUM NACHWEIS VON MEMBRANE REZEPTOREN-LIGAND INTERAKTIONEN
USE OF A FLUORESCENT PROTEIN FOR DETECTING THE INTERACTION BETWEEN A MEMBRANE RECEPTOR AND ITS LIGAND

(30) Priorité: 05.06.1997 FR 9706977
(43) Date de publication de la demande: 22.03.2000
(73) Titulaire: CENTRE NATIONAL DE LA RECHERCHE SCIENTIFIQUE (CNRS), 75016 Paris (FR)
(72) Inventeur: GALZI, Jean-Luc, F-67100 Strasbourg (FR); ALIX, Philippe, F-14650 Carpiquet (FR)
(74) Mandataire: Grosset-Fournier, Chantal Catherine
(86) Numéro de dépôt international: PCT/FR1998/001136
(87) Numéro de publication internationale: WO 1998/055873

(56) Documents cités:
- EP-A- 0 552 108
- WO-A-91/01305
- WO-A-96/41166
- WO-A-97/28261
- US-A- 5 342 789
- US-A- 5 439 797
- US-A- 5 625 048
- MIYAWAKI A ET AL: "Fluorescent indicators for Ca-2+ based on green fluorescent proteins and calmodulin." NATURE (LONDON) 388 (6645). 1997. 882-887. ISSN: 0028-0836, XP002058386
- ROMOSER V A ET AL: "Detection in living cells of Ca-2+-dependent changes in the fluorescence emission of an indicator composed of two green fluorescent protein variants linked by a calmodulin - binding sequence: A new class of fluorescent indicators." JOURNAL OF BIOLOGICAL CHEMISTRY 272 (20). 1997. 13270-13274. ISSN: 0021-9258, XP002058387
- PETUSHKOV V N ET AL: "Direct measurement of excitation transfer in the protein complex of bacterial luciferase hydroxyflavin and the associated yellow fluorescence proteins from Vibrio fischeri Y1." BIOCHEMISTRY 35 (25). 1996. 8413-8418. ISSN: 0006-2960, XP002058388
- MITRA R D ET AL: "Fluorescence resonance energy transfer between blue-emitting and red-shifted excitation derivatives of the green fluorescent protein." GENE (AMSTERDAM) 173 (1). 1996. 13-17. ISSN: 0378-1119, XP004042847
- PROTASEVICH ET AL BIOCHEMISTRY vol. 36, 1997, pages 2017 - 2024
- FALLON ET AL STRUCTURE vol. 11, 2003, pages 1303 - 1307
- CHATTOPADHYAYA ET AL JOURNAL OF MOLECULAR BIOLOGY vol. 228, 1992, pages 1177 - 1192
- BREJC ET AL NATURE vol. 411, 2001, pages 269 - 276
- PALCZEWSKI ET AL SCIENCE vol. 289, 2000, pages 739 - 745
- LACOWICZ: 'Principles of fluorescence spectroscopy', 1999, KLUWER ACADEMIC / PLENUM PUBLISHERS, NEW YORK

## Description

L'invention concerne l'utilisation d'une protéine fluorescente pour la détection d'interactions entre un récepteur membranaire et son ligand.

De nombreux médicaments et substances naturelles exercent leur action en interagissant avec des protéines régulatrices appelées récepteurs, impliquées dans de nombreuses fonctions physiologiques des organismes, et les altérations de leurs fonctions sont la cause de nombreuses pathologies. L'accessibilité des récepteurs aux agents pharmacologiques endogènes ou exogènes, naturels ou synthétiques, depuis l'extérieur de la cellule conduit à les considérer comme des cibles de choix pour la recherche de molécules biologiquement actives, en particulier de molécules présentant des pouvoirs thérapeutiques potentiels.

Afin d'identifier de nouveaux outils pharmacologiques et produits médicamenteux, plusieurs tests de criblage de molécules biologiquement actives, ont été développés :
- le test SPA ou test de proximité par scintillation (scintillation proximity assay) (Udenfirend *et al.* Anal. Biochem. (1987) 161:494-500, brevet américain n° 4,568,649 ; brevet européen 0,154,734 et brevet japonais n° 60-196668) ne parvient pas à s'affranchir de l'utilisation de molécules radioactives, et donc de toutes les nuisances associées à la manipulation, l'utilisation et le stockage de radioéléments ;
- le test de mesure par transfert d'énergie de fluorescence utilisant le couple europium - allophycocyanine (Mathis. clin. Chem. (1993), 39:1953-1959) requiert l'utilisation d'allophycocyanine purifiée et nécessite son greffage sur une protéine cible elle aussi purifiée, ce qui n'est pas toujours possible surtout lorsque la protéine cible n'est pas abondante ;
- les tests fonctionnels faisant intervenir la régulation de gènes rapporteurs codant pour des protéines luminescentes, ou pourvues d'une activité enzymatique dosée par colorimétrie, sont des mesures très indirectes de l'interaction entre un ligand et son récepteur, peuvent être source de faux-positifs, font intervenir des cascades d'amplification qui interfèrent avec la mesure quantitative des interactions entre le récepteur et ses ligands, et ne sont applicables que sur des système biologiques couplés à une transcription des gènes (Broach, J.R. & Thorner, J. 1996, Nature 384 supp. 14-16) ;
- un test de mesure par fluorescence permettant la détection de la liaison d'un messager secondaire intracellulaire (le nucléotide cyclique AMPc) développé par greffage d'un site de liaison du messager secondaire sur la protéine fluorescente verte (GFP) de méduse *Aequorea victoria* (Thastrup *et al.* WO96/23898). Ce test qui permet la détection de molécules biologiquement actives se limite aux analogues de messagers secondaires affectant des processus intracellulaires.

Le gène codant pour une protéine fluorescente issue de la méduse *Aequorea victoria,* la protéine fluorescente verte (green fluorescent protein ou GFP) (Prasher *et al.* 1992, Gene 111:229-233), a été récemment déchiffré. La GFP est une protéine monomérique. Elle acquiert ses propriétés de fluorescence par un mécanisme autocatalytique de formation du fluorophore. L'expression, endogène ou hétérologue, de la GFP ne requiert qu'un gène et ne nécessite l'addition d'aucun groupe prosthétique. La GFP a été exprimée de manière hétérologue dans des cellules et organismes aussi divers que les bactéries, les levures, les cellules eucaryotes animales et végétales. La structure de la GFP (Ormö *et al.* 1996, Science 273:1392-1395; Yang *et al.* 1996, Nature Biotechnology, 14:1246-1251) permet son greffage sur d'autres polypeptides, soit du côté amino-terminal soit du côté carboxy terminal sans préjudice ni vis-à-vis de son taux d'expression, ni vis-à-vis de la formation du fluorophore. La GFP a ainsi pu être couplée, par fusion de gènes, à des protéines solubles ou membranaires.. Enfin, les codons contenus dans le gène naturel ont été remplacés par les codons préférés des organismes hôtes (EGFP [Cormack *et al.* 1996, Gene 173:33-38] par exemple, pour l'expression dans les cellules eucaryotes animales) et diverses mutations permettent de modifier ses spectres d'absorption et d'émission de lumière et rendent possible des détections multiples dans un système d'expression unique.

La demande internationale WO 91/01305 décrit l'utilisation de GFP comme gène rapporteur de l'expression d'une autre protéine, mais ne mentionne pas la mesure de transfert d'énergie de fluorescence, ni la mesure des interactions entre une protéine cible et son ligand.

L'article de Mitra et al. (1996, *Gene,* 173: 13-17) concerne l'utilisation d'un polypeptide contenant un site de clivage par une enzyme protéolytique, marqué génétiquement par deux GFP de couleurs différentes disposées de part et d'autre du site de clivage. L'occurrence d'une coupure par l'enzyme dans ce site provoque une diminution du signal de transfert d'énergie détecté entre les deux GFP. Le procédé décrit dans cet article ne concerne pas la mesure de transfert d'énergie entre deux molécules.

La demande internationale WO 96/41166 concerne l'utilisation d'un ion hydrophobe capable de se transloquer d'une face d'une membrane biologique à l'autre face de cette membrane en fonction du potentiel membranaire. On le visualise sur une seule des deux faces par transfert d'énergie de fluorescence avec une autre molécule incapable de se transloquer. Cependant, ce procédé n'utilise pas de GFP et ne mesure pas d'interactions entre une protéine cible et l'un de ses ligands.

L'invention a pour but la préparation de protéines cibles membranaires récepteurs, rendu(e)s fluorescent(e)s par fusion avec une protéine fluorescente, d'une part, de leurs ligands marqués, d'autre part, et leur utilisation pour la détection d'interactions entre les récepteurs fluorescents et leurs ligands marqués et l'identification de nouvelles molécules biologiquement actives.

L'un des autres aspects de l'invention est un procédé simple à mettre en oeuvre, rapide, sensible, permettant d'effectuer des mesures quantitatives, à l'équilibre et en temps réel, d'interactions non covalentes entre une protéine cible membranaire et son ligand.

L'un des autres aspects de l'invention est de proposer un procédé généralisable à de nombreuses protéines cibles membranaires et à leurs ligands.

L'un des autres aspects de l'invention est de proposer un procédé ne nécessitant ni la purification de la protéine cible membranaire, ni celle du ligand.

L'un des aspects de l'invention est de proposer un procédé non polluant puisqu'il n'utilise pas de radioactivité, économique puisqu'il utilise la lumière visible (pas de quartz) sur les équipements disponibles et ne nécessitant aucune filtration.

L'un des autres aspects est de proposer un procédé automatisable.

L'invention a pour objet la construction de d'ADNc contenant une séquence codant pour
1) un récepteur membranaire, dans lequel un ou plusieurs acides aminés sont substitués, insérés ou délétés, fusionnée avec l'ADNc codant pour la GFP, ou l'un de ses mutants, dans lequel un ou plusieurs acides aminés sont substitués, insérés ou délétés afin de maintenir la phase de lecture et d'obtenir un polypeptide hybride.

L'invention a également pour objet des cellules contenant une séquence d'ADN codant pour un récepteur, dans lequel un ou plusieurs acides aminés sont substitués, insérés ou délétés, fusionnée avec l'ADNc codant pour la GFP, ou l'un de ses mutants, dans lequel un ou plusieurs acides aminés sont substitués, insérés ou délétés afin de maintenir la phase de lecture et capable d'exprimer la séquence d'ADN considérée.

L'invention a également pour objet la production de ligands pharmacologiquement actifs contenant un groupe chimique fluorescent lié par une réaction chimique dans lequel le groupe fluorescent est soit donneur d'énergie pour la GFP ou l'un de ses mutants, soit accepteur d'énergie de la GFP ou de l'un de ses mutants.

L'invention a également pour objet la culture de cellules contenant l'hybride protéine-GFP dans des conditions permettant
1) l'expression du polypeptide hybride,
2) la détection de fluorescence de la cellule.

L'invention a également pour objet l'incubation de cellules exprimant l'hybride protéine-GFP avec le ligand fluorescent, la mesure des changements de fluorescence soit d'émission du donneur, soit d'émission de l'accepteur, soit d'excitation de l'accepteur, révélant l'interaction protéine ligand, ainsi que l'addition d'une molécule suspectée biologiquement active avec le ligand fluorescent et la mesure des altérations du signal de transfert d'énergie par rapport à l'incubation des susdites cellules avec le ligand fluorescent.

L'invention a également pour objet la construction d'ADNc contenant une séquence codant pour
1) un polypeptide, notamment un ligand, dans lequel un ou plusieurs acides aminés sont substitués, insérés ou délétés, fusionné avec l'ADNc codant pour la GFP, notamment son mutant S65T ou S65C, dans lequel un ou plusieurs acides aminés sont substitués, insérés ou délétés afin de maintenir la phase de lecture et d'obtenir un polypeptide hybride.

L'invention a également pour objet des cellules contenant une séquence d'ADN codant pour un polypeptide, notamment un ligand, dans lequel un ou plusieurs acides aminés sont substitués, insérés ou délétés, fusionné avec l'ADNc codant pour la GFP, notamment son mutant S65T ou S65C, dans lequel un ou plusieurs acides aminés sont substitués, insérés ou délétés afin de maintenir la phase de lecture et capable d'exprimer la séquence d'ADN considérée.

L'invention a également pour objet la culture de cellules contenant l'hybride ligand-GFP dans des conditions permettant
1) l'expression du polypeptide hybride,
2) la détection de fluorescence de la cellule,
3) l'isolement de l'hybride ligand-GFP.

L'invention a également pour objet l'incubation de cellules exprimant l'hybride protéine-GFP avec l'hybride ligand-GFP, notamment le mutant S65T ou S65C de la GFP et la mesure les changements de fluorescence soit d'émission du donneur (protéine-GFP), soit d'émission de l'accepteur (ligand-GFP), soit d'excitation de l'accepteur, révélant l'interaction protéine ligand et l'addition d'une molécule suspectée biologiquement active avec l'hybride ligand-GFP et la mesure des altérations du signal de transfert d'énergie par rapport à l'incubation des cellules exprimant l'hybride protéine-GFP et l'hybride ligand-GFP.

Ces différents aspects sont obtenus par l'utilisation d'une protéine fluorescente choisie parmi les protéines fluorescentes issues ou dérivées de protéines autofluorescentes de cnidaires, notamment choisie parmi :
- la protéine fluorescente verte (GFP), ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence, pour la détection et la quantification d'interactions non covalentes entre une protéine cible marquée par la GFP ou l'un des variants ci-dessus définis ou l'un des fragments ci-dessus définis et l'un de ses ligands marqué par un marqueur constitué :
   - soit par une molécule susceptible d'absorber la lumière émise par la protéine fluorescente,
   - soit par une substance fluorescente,
   cette détection et quantification ayant lieu par transfert d'énergie de fluorescence:
   . entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'excitation de la GFP, ou de l'un des susdits variants, ou de l'un des susdits fragments, ou
   . entre la GFP ou l'un de ses variants définis ci-dessus, ou l'un des fragments définis ci-dessus, et la susdite molécule susceptible d'absorber la lumière émise par la protéine fluorescente.

L'invention a notamment pour objet l'utilisation d'une protéine fluorescente choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
- la protéine fluorescente verte (GFP), ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence, pour la détection et la quantification d'interactions non covalentes entre une protéine cible marquée par voie génétique par la GFP ou l'un des variants ci-dessus définis ou l'un des fragments ci-dessus définis et l'un de ses ligands marqué par un marqueur constitué :
   - soit par une molécule susceptible d'absorber la lumière émise par la protéine fluorescente,
   - soit par une substance fluorescente,
   cette détection et quantification ayant lieu par transfert d'énergie de fluorescence:
   . entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'excitation de la GFP, ou de l'un des susdits variants, ou de l'un des susdits fragments, ou
   . entre la GFP ou l'un de ses variants définis ci-dessus, ou l'un des fragments définis ci-dessus, et la susdite molécule susceptible d'absorber la lumière émise par la protéine fluorescente.

La présente invention comporte les avantages suivants: La sensibilité de la détection de la cible et/ou du ligand obtenue par fusion avec la protéine fluorescente est telle que des concentrations de l'ordre du picomolaire sont aisément détectables. Par le fait que la fluorescence est introduite sur la cible (parfois aussi sur le ligand) par manipulation génétique, il résulte qu'il n'est pas nécessaire de disposer de la protéine que l'on souhaite rendre fluorescente, ce qui permet de détecter des protéines dont l'abondance naturelle empêche tout marquage chimique par manque de quantités manipulables (protéine non isolée ou non isolable) de celle-ci.

Le marquage par voie génétique offre en outre deux avantages supplémentaires, à savoir, quantité et homogénéité. Par opposition au marquage chimique qui peut survenir dans certains cas de manière partielle et inhomogène, la marquage par voie génétique est quantitatif et survient à une position prédéterminée et connue. Il en résulte que toutes les molécules sont marquées et qu'elles sont toutes marquées de manière identique.

L'invention consiste à détecter des interactions non covalentes entre récepteur membranaire et l'un de ses ligands, par transfert d'énergie de fluorescence, et à utiliser ce procédé dans le criblage de molécules biologiquement actives, notamment dans le domaine des récepteurs. La protéine est rendue fluorescente par fusion de son ADNc avec l'ADNc codant pour la GFP, le ligand est rendu fluorescent soit par greffage chimique d'un groupe fluorescent, soit par fusion de son gène avec celui de la GFP. L'interaction entre la protéine et son ligand entraîne des modifications du spectre de fluorescence de la GFP et/ou du ligand, qui peuvent être enregistrés en temps réel ou à l'équilibre.

L'interaction non covalente entre une protéine cible et son ligand correspond à la formation d'un complexe entre la protéine cible et le ligand dans lequel la protéine cible et le ligand interagissent l'une avec l'autre, et sont retrouvés intacts après suppression de l'interaction

Une série de tests permettant de déterminer la nature non covalente, spécifique et saturable de l'interaction est notamment décrite dans les articles suivants : Levitski, A. 1980, in Cellular receptors for hormones and neurotransmitters, Eds Schulster, D. & Levitski, A., John Wiley & Sons Ltd.; Horovitz, H. and Levitski, A. 1987, Proc. Natl. Acad. Sci. 84:6654-6658; Receptor Biochemistry and Methodology, volume 3, Ventre, J.C. & Harrison, L.C. Eds, Alan R. Liss, INC, New York, 1987.

Dans le contexte présent, les termes suivants sont définis ainsi :
- protéine autofluorescente : protéine synthétique ou naturelle dans laquelle le chromophore se forme par une réaction autocatalytique entre des acides aminés de la protéine sans nécessiter l'addition d'un groupe prosthétique et dont les propriétés de fluorescence sont intrinsèques au monomère,
- "polypeptide hybride" : indique un polypeptide qui est une fusion d'au moins une partie de deux protéines, dans l'invention, à titre d'exemple au moins une partie de la GFP avec une partie d'une protéine cible ou une partie d'un ligand polypeptidique dé la protéine cible,
- "compétiteur" : toute molécule se liant sur la protéine cible au même site que le ligand fluorescent,
- "substance biologiquement active" : toute substance susceptible d'interférer avec l'interaction d'un ligand fluorescent et de la protéine fluorescente spécifique en modifiant les paramètres cinétiques ou thermodynamiques de l'interaction.

On appelle ligand toute molécule qui interagit avec une autre molécule de manière non covalente et réversible.

L'expression "transfert d'énergie de fluorescence" correspond à un processus physique, dépendant de la distance, par lequel de l'énergie est transmise de manière non radiative d'un chromophore excité, le donneur, à un autre chromophore, l'accepteur, par interaction dipôle-dipôle (Förster 1951 in Fluoreszenz organischer Verbindung. Vandenhoek and Rupprecht, Gôttingen; Wu and Brand 1994 Anal. Biochem. 218:1-13; Clegg 1995, Current Opinion in Biotechnol. 6:103-110). Le transfert d'énergie peut être observé soit par une diminution de l'amplitude de l'émission du donneur, soit par une augmentation de l'amplitude de l'excitation et de l'émission de l'accepteur.

Dans le cas de l'application du transfert d'énergie à des échantillons biologiques en interaction non covalentes, le signal de transfert ne peut pas persister si les conditions expérimentales ne permettent pas l'interaction entre le ligand fluorescent et la protéine cible fluorescente. De même, si l'un des deux partenaires interagissant n'est pas fluorescent, les éventuelles variations de fluorescence observées pour l'autre partenaire ne pourront pas être attribuées à un processus de transfert d'énergie.

Les termes changement ou variation de fluorescence, définis dans le contexte du transfert d'énergie, se réfèrent à toutes modifications de 1) l'amplitude du signal de fluorescence du donneur, de 2) l'amplitude du spectre d'excitation ou 3) l'amplitude du signal d'émission du donneur. Les variations ou changements de fluorescence ne doivent pas être observés si l'un des deux partenaires n'est pas fluorescent ou si l'interaction entre les partenaires fluorescents est inhibée, par exemple par un excès d'un agent compétiteur.

De façon plus précise, la réaction de transfert d'énergie de fluorescence requiert deux groupes fluorescents, l'un appelé donneur et l'autre accepteur. Cette réaction se produit lorsque deux conditions sont réunies :
1) le spectre d'absorption de l'accepteur et le spectre d'émission du donneur doivent se recouvrir, au moins en partie ; le recouvrement se calcule à partir de données expérimentales et d'une équation donnant une valeur en cm³M⁻¹ (Lakey *et al.* 1991, J. Mol. Biol. 218:639-653) ;
2) le donneur et l'accepteur doivent être proches dans l'espace (de 10 à 100 angströms) afin que le transfert d'énergie puisse avoir lieu.

La première condition a pour conséquence le fait que l'excitation du donneur entraîne alors de manière concomitante une diminution de l'amplitude de l'émission du donneur et l'apparition d'un signal d'émission de l'accepteur. Ceci permet de détecter les interactions entre le donneur et l'accepteur et/ou de mesurer leur distance.

L'expression "proches dans l'espace" signifie que la distance entre le donneur et l'accepteur est inférieure ou égale à 2 Ro, Ro représentant le rayon de Forster (op.cit.) (Lakey, J.H. *et al.* 1991, J. Mol. Biol. 218:639-653).

Si l'accepteur n'est pas fluorescent, mais présente un spectre d'excitation recouvrant au moins en partie le spectre d'émission du donneur, le transfert d'énergie pourra être détecté sous la forme d'une réduction d'amplitude de l'émission du donneur.

L'invention concerne l'utilisation d'un ligand marqué par un marqueur constitué :
- soit par une molécule susceptible d'absorber la lumière émise par la protéine fluorescente,
- soit par une substance fluorescente, pour la détection et la quantification d'interactions non covalentes entre une protéine cible et le susdit ligand, ladite protéine cible étant marquée par voie génétique par une protéine fluorescente choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :

- la protéine fluorescente verte (GFP), ou
- des variants dérivés de la GFP, par addition , délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence, cette détection et quantification ayant lieu par transfert d'énergie de fluorescence:
   . entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'excitation de la GFP, ou de l'un des susdits variants, ou de l'un des susdits fragments, ou
   . entre la GFP ou l'un de ses variants définis ci-dessus, ou l'un des fragments définis ci-dessus, et la susdite molécule susceptible d'absorber la lumière émise par la protéine fluorescente.

Selon un mode de réalisation avantageux, la protéine fluorescente est choisie parmi :
- la protéine fluorescente verte (GFP) (*Ward et al.* 1980, Photochem. Photobiol. 31:611-615 ; Chalfie 1995, Photochem. Photobiol. 62:651-656), ou EGFP (Heim & Tsien, Current Biology, 1996, vol. n° 6, p178-182 ; Miyawaki et al., Nature 1997, vol. 388, p882-887),
- la protéine fluorescente cyan (CFP ou ECFP) (Heim & Tsien, Current Biology, 1996, vol. n° 6, p178-182 ; Miyawaki et al., Nature 1997, vol. 388, p882-887),
- la protéine fluorescente jaune (YFP ou EYFP) (Cormack *et al.* 1995, Gene 173:33-38 ; Heim, Cubitt et Tsien 1995, Nature ; Ehrig *et al.* 1995, FEBS Lett. 367:163-166) (Miyawaki et al., Nature 1997, vol. 388, p882-887),
- GFPUV (Crameri *et al.* 1996, Nature Biotechnol. 14:315-319 : Ehrig *et al.* 1995, FEBS Lett. 367:163-166),
ou leurs mutants dans lesquels les codons sont optimisés pour l'expression dans les cellules humaines, bactériennes ou végétales,
ou leurs mutants présentant des longueurs d'excitation ou d'émission plus élevées ou plus faibles que celles associées aux protéines définies ci-dessus, sous réserve que leur coefficient d'extinction moléculaire soit supérieur à environ 14000M⁻¹cm⁻¹ et leur rendement quantique de fluorescence soit supérieur à environ 0,38.

L'expression "codons optimisés" indique la substitution de codons de la protéine sauvage par leurs homologues préférés de l'organisme hôte, sans changement de code donc sans changement de séquence protéique.

La GFP sauvage (WT) de longueur d'onde, d'excitation et d'émission 395/470-509 est décrite dans ref: Ward *et al.* 1980 Photochem. Photobiol. 31:611-615, Chalfie 1995, Photochem. Photobiol. 62:651-656.

La GFP UV présentant les mutations suivantes : F99S, M153T, V163A de longueur d'onde, d'excitation et d'émission respectivement de 395-510 est décrite dans Crameri *et al.* 1996 Nature Biotechnol. 14:315-319. ou avec la mutation T203I et la longueur d'onde respectivement d'excitation et d'émission 400-512 est décrite dans Ehrig *et al.* 1995 FEBS Lett. 367:163-166.

La EGFP présente les mutations suivantes :
F64L S65T H231L

La EYFP présente les mutations suivantes :
S65G V68L S72A T203Y

La ECFP présente les mutations suivantes :
F64L S65T Y66W N146I
M153T V163A N212K

Les différents mutants de GFP peuvent en outre être optimisés (par l'introduction de mutations silencieuse optimisant l'usage de codons spécifiques à chaque espèce) pour l'expression dans ces cellules :
- humaines, ref Haas *et al.* 1996 Curr. Biol. 6:315-323; Yan *et al.* 1996, Nucleic Ac. Res. 24:4592-4593; Zolotukhin *et al.* 1996, J. Virol. 70:4646-4654
- bactériennes : Crameri *et al.* 1996 Nature Biotechnol. 14:315-319, Cormack *et al.* 1996, Gene, 173:33-38 pour *Escherichia coli,*
- végétales: Reichel *et al.* 1996, Proc. Natl. Acad. Sci. 93:5888-5893.

### . GFP :

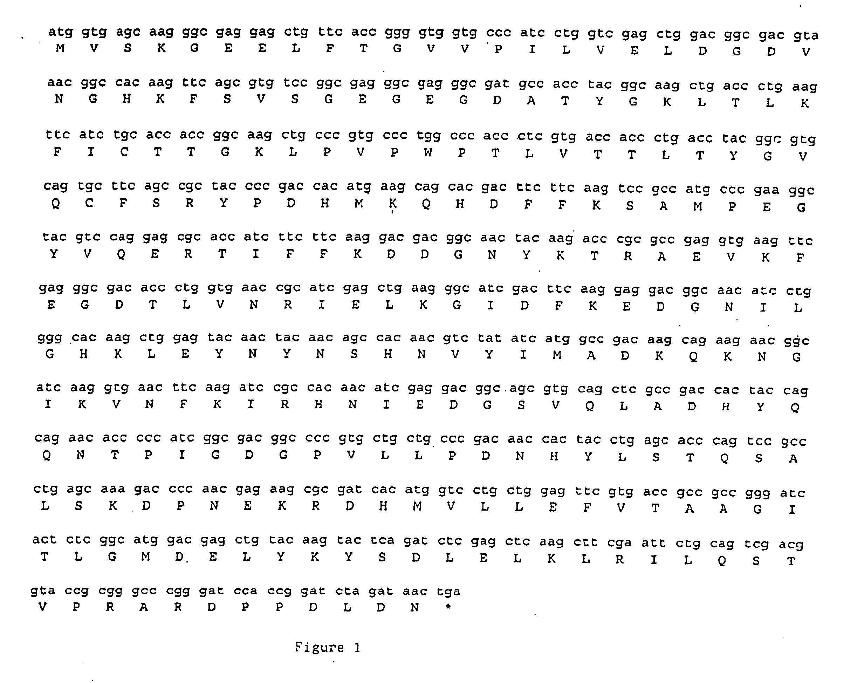

Le terme GFP indique une protéine codée par la séquence nucléotidique donnée en figure 1, et qui une fois exprimée dans des cellules émet une fluorescence. Les GFPs présentant des substitutions, additions ou délétions d'acides aminés influençant soit les propriétés de fluorescence, soit le taux d'expression de la GFP sont appelés mutants de GFP.

On donne ci-après les principales caractéristiques des protéines fluorescentes avantageusement utilisées dans le procédé de l'invention :

| Protéine | λ-excitation maximale | λ-émission | coefficient d'extinction | rendement quantique |
|---|---|---|---|---|
| EYFP | 514 | 527 | 36500 | 0,63 |
| ECFP | 432 | 480 | 18000 | 0,67 |
| GFPUV | 395 | 509 | 21000 | 0,77 |
| EGFP | 489 | 511 | 39000 | 0,66 |

La protéine autofluorescente BFP est de préference exclue car elle ne répond pas aux conditions défines pour les protéines autofluorescente de cnidaire, à savoir coefficient d'extinction moléculaire supérieur à 14000 M⁻¹ cm⁻¹ et rendement quantique de fluorescence supérieur à 0,38.

L'invention concerne également l'utilisation d'une protéine fluorescente (n° 1) telle que définie ci-dessus, dans laquelle le ligand est marqué
* soit par une substance fluorescente, le marquage étant :
   - soit effectué par voie chimique, la substance fluorescente étant alors un composé chimique,
   - soit effectué par voie recombinante, la substance fluorescente étant alors un peptide ou une protéine fluorescente (n° 2), et pouvant être notamment choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :

   - la protéine fluorescente verte (GFP), ou
   - des variants dérivés de la GFP, par addition , délétion ou substitution d'un ou plusieurs acides aminés, sous réserve que ces variants conservent la propriété de fluorescence,
   - ou des fragments de la GFP, ou de fragments des susdits variants, sous réserve que ces fragments conservent la propriété de fluorescence,
* soit par une substance non fluorescente appartenant au groupe des acides violets [Acid Violet 5, CAS 10130-48-0 ; Acid Violet 7, CAS 4321-69-1 ; Acid Violet 17, CAS 4129-84-4], acides rouges [Acid Red 1, CAS 3734-67-6 ; Acid Red 8, CAS 4787-93-3 ; Acid Red 37, CAS 6360-07-2 ; Acid Red 40, CAS 12167-45-2 ; Acid Red 106, CAS 6844-74-2 ; Acid Red 114, CAS 6459-94-5], les alizarines, l'aluminon, l'azocarmine B [CAS 25360-72-9], la fuschine basique [Basic Red 9, CAS 569-61-9], le Bordeaux R [Acid Red 17, CAS 5858-33-3], la Carmine [CAS 1390-65-4].

"CAS" correspond à Chemical Abstracts.
. Marquage :

Par marquage d'une protéine cible ou d'un ligand, on entend:
- pour la protéine cible, la fusion de son gène ou ADNc, ou partie du gène ou de l'ADNc, avec le gène ou ADNc, ou partie du gène ou ADNc, de la GFP;
- pour le ligand, il peut s'agir d'un couplage chimique entre le ligand et un groupe fluorescent, ou bien de la fusion de son gène ou ADNc, ou partie du gène ou de l'ADNc, avec le gène ou ADNc, ou partie du gène ou ADNc, de la GFP.

L'invention concerne l'utilisation d'une protéine fluorescente selon l'invention dans laquelle la protéine cible et le ligand sont marqués par voie génétique, la protéine fluorescente et la substance fluorescente étant respectivement choisies parmi les couples de composés suivants :
GFPUV - EYFP
EYFP - GFPUV
ECFP - EYFP
EYFP - ECFP
ECFP - EGFP
EGFP - ECFP
EGFP - EYFP
EYFP - EGFP
et notamment dans laquelle la protéine cible est marquée par la protéine EYFP ou EGFP et le ligand est marqué par la protéine ECFP, ou la protéine cible est marquée par la protéine ECFP et le ligand est marqué par la protéine EYFP ou EGFP.

L'invention a également pour objet l'utilisation d'une protéine fluorescente choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
- la protéine fluorescente verte (GFP), ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence,
pour la détection et la quantification d'interactions non covalentes entre une protéine cible marquée par la GFP ou l'un des variants ci-dessus définis ou l'un des fragments ci-dessus définis et l'un de ses ligands marqué par une substance fluorescente, cette détection et quantification ayant lieu par transfert d'énergie de fluorescence entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'onde d'excitation de la GFP, ou de l'un des susdits variants, ou de l'un des susdits fragments.

Selon un mode de réalisation avantageux de l'invention, la protéine fluorescente est EGFP et dans laquelle :
- soit la EGFP est donneur d'énergie de fluorescence et le marqueur absorbant la lumière émise par la EGFP est une substance fluorescente ou non, et le marqueur étant choisi parmi des substances, dont le spectre d'excitation chevauche le spectre d'émission de la EGFP, et notamment dans le cas où le marqueur est une substance fluorescente, il est choisi parmi : le 4,4-difluoro-4-bora-3a,4a-diaza-s-indacene (Bodipy), l'éosine, l'érythrosine, la tétraméthylrhodamine, la sulforhodamine 101 commercialisée par Molecular probe sous la dénomination Texas Red, et leurs dérivés permettant d'une part le greffage et, d'autre part, dont le spectre d'excitation recouvre le spectre d'émission de EGFP,
   et dans le cas où le marqueur n'est pas une substance fluorescente, il est choisi parmi le groupe des acides violets [Acid Violet 5, CAS 10130-48-0 ; Acid Violet 7, CAS 4321-69-1 ; Acid Violet 17, CAS 4129-84-4], des acides rouges [Acid Red 1, CAS 3734-67-6 ; Acid Red 8, CAS 4787-93-3 ; Acid Red 37, CAS 6360-07-2 ; Acid Red 40, CAS 12167-45-2 ; Acid Red 106, CAS 6844-74-2 ; Acid Red 114, CAS 6459-94-5], les alizarines, l'aluminon, l'azocarmine B [CAS 25360-72-9], la fuschine basique [Basic Red 9, CAS 569-61-9], le Bordeaux R [Acid Red 17, CAS 5858-33-3], la Carmine [CAS 1390-65-4],
- soit la EGFP est accepteur d'énergie de fluorescence et la substance fluorescente est donneur d'énergie de fluorescence et est choisie parmi des substances, dont le spectre d'émission chevauche le spectre d'excitation de la EGFP, et notamment parmi : les coumarines, la fluorescamine, le 6-(N-méthylanilino)naphtalène, (mansyl) et leurs dérivés permettent d'une part le greffage et, d'autre part, dont le spectre d'émission recouvre le spectre d'excitation de EGFP,
- ou soit la protéine fluorescente est ECFP et est donneur d'énergie de fluorescence et la substance fluorescente est accepteur d'énergie et est choisi parmi la fluoresceïne et le 7-nitrobenz-2-oxa-1,3-diazole,
- ou soit la protéine fluorescente est ECFP et est accepteur d'énergie de fluorescence et la substance fluorescente est donneur d'énergie et est choisie parmi le pyrène ou la coumarine ou leurs dérivés permettant d'une part le greffage, et, d'autre part, dont le spectre d'émission chevauche le spectre d'excitation de la ECFP.

S'agissant de la protéine cible, elle peut être choisie parmi :
- les récepteurs membranaires couplés à la protéine G, notamment dans Supplement Trends in Pharmacological Sciences, 1997 (*Receptor and ion Channel Nomenclature),*
- les récepteurs des facteurs de croissance, notamment ceux qui sont structurellement reliés au récepteur de l'insuline (Yarden, Y. and Ullrich, A. 1988, Biochemistry 27:3113-3119) ou au récepteur de l'interféron γ (Brisco, J. *et al.* 1996, Phylos. Trans. R. Soc. Lond. B. Biol. Sci. 351:167-171 ; Ihle, J.N. 1995, Nature 372:591-594),
- les récepteurs canaux, notamment dans Supplement Trends in Pharmacological Sciences, 1997 (*Receptor and ion Channel Nomenclature*),

Selon un mode de réalisation avantageux, la protéine cible est choisie parmi les récepteurs membranaires couplés à la protéine G.

Dans ce qui précède et ce qui suit, on désigne par "récepteur" toute molécule de nature protéique susceptible d'entrer en interaction non covalente avec un agent pharmacologique. Préférentiellement dans l'invention un récepteur de neurotransmetteur, d'hormones, de facteur de croissance etc.., capable de produire, après interaction avec un ligand pharmacologique, une réponse de transduction de signal mesurable *in vivo* et/ou *in vitro.*

Par réponse de transduction de signal, on désigne toute réponse, ou inhibition de réponse, mesurable *in vivo* et/ou *in vitro,* résultant de l'interaction d'un récepteur avec ses agent pharmacologiques spécifiques et conduisant à des activations, ou inhibitions, du métabolisme cellulaire par effet sur des messagers secondaires, des enzymes, ou des courants ioniques.

S'agissant de la réponse de transduction de signal pour les récepteurs couplés aux protéines G, le test général consiste à déterminer l'activation de la protéine G par mesure de la fixation de GTP (Befort *et al.* 1996 Neurochem. Res. 11:1301-1307). D'autres mesures plus spécifiques font par exemple intervenir des déterminations de concentrations intracellulaires d'AMPc, d'inositol phosphates, de calcium, des mesures d'activation de la transcription de gènes ou d'activité oncogénique, suivant le type de couplage spécifique du récepteur considéré.

Pour les récepteurs-canaux, les mesures les plus directes sont des déterminations de courants ioniques (Hille, B. 1992 in Ion channels of excitable membranes, Sinauer Associates, Sunderlands, Massachussets). D'autres mesures peuvent, par exemple, faire intervenir des détermination des transcription de gènes ou des activations d'enzymes.

Pour les récepteurs de facteurs de croissance, les tests généraux sont ceux de la prolifération, de différentiation ou de la survie cellulaire, fréquemment aussi des tests de phosphorylations de substrats spécifiques (Honneger *et al.* 1988, EMBO J. 7:3053-3060) de chaque récepteur et repérage par des anticorps spécifiques de phosphoaminoacides.

A titre d'exemples de récepteurs membranaires couplés aux protéines G, on peut citer les récepteurs de purines et nucléotides, des amines biogéniques des peptides et protéines, des eicosanoides, des lipides et dérivés, des acides aminés excitateurs et des ions, des molécules olfactives ainsi que les récepteurs orphelins (ci-après une liste assez exhaustive).

A titre d'exemple de récepteurs de facteurs de croissance, on peut citer les cytokines, le facteur de croissance épidermique, l'insuline, le facteur de croissance dérivé des plaquettes, le facteur de croissance de transformation.

A titre de récepteurs canaux, on peut citer notamment les récepteurs de l'ATP, de la sérotonine, du GABA, de la glycine, de l'acétylcholine, du glutamate.

A titre d'exemple de récepteurs nucléaires, on peut citer notamment les récepteurs des hormones thyroidiennes, des oestrogènes, des glucocorticoïdes, des rétinoïdes.

A titre de ligands des récepteurs couplés à la protéine G on peut citer :
● Purines et Nucléotides
   . Adénosine
   . cAMP
   . ATP
   . UTP
   . ADP
● Amines Biogéniques (et ligands naturels reliés)
   - 5-hydroxytryptamine
   - Acétylcholine
   - Dopamine
   - Adrénaline
   - Histamine
   - Mélatonine
   - Noradrénaline
   - Tyramine/Octopamine
   - autres composés reliés
● Peptides
   . Hormone adrénocorticotrophique (ACTH)
   . Hormone stimulatrice de mélanocyte (MSH)
   . Mélanocortines
   . Neurotensine (NT)
   .Bombésine et peptides voisins
   . Endothélines
   . Cholecystokinine
   . Gastrine
   . Neurokinine B (NKB)
   . Récepteur des tachykinines
   . Substance K (NKA)
   . Substance P (SP)
   . Neuropeptide Y (NPY)
   . Facteur de libération de la thyrotropine
   . Nociceptine
   . Bradykinine
   . Angiotensine II
   . Beta-endorphine
   . C5a anaphalatoxine
   . Calcitonine
   . Chemokines (également appelés intercrines)
   . Facteur de libération corticotrophique (CRF)
   . Dynorphine
   . Endorphine
   . Peptides formylés
   . Follitropine (FSH)
   . Phéromones de maturation fongique
   . Galanine
   . Récepteur du polypeptide inhibiteur gastrique (GIP)
   . Peptides analogues du glucagon (GLPs)
   . Glucagon
   . Hormone de libération de gonadotropine (GmRH)
   . Hormone de libération de l'hormone de croissance (GHRM)
   . Hormone diurétique d'insecte
   . Interleukine
   . Leutropine (LH/HCG)
   . MET -enképhaline
   . Peptides opioides
   . Oxytocine
   . Hormone parathyroide (PTH) et (PTHrP)
   . Peptides activant l'adényl cyclase pituitaire (PACAP)
   . Secretine
   . Somatostatine
   . Thrombine
   . Thyrotropine (TSH)
   . Peptide instestinal vasoactif (VIP)
   . Vasopressine
   . Vasotocine
● Eicosanoides
   . IP - Prostacyclines
   . PG - Prostaglandines
   . TX - Thromboxanes
● Composés à base de rétinal
   . 11-cis retinal de vertébré
   . 11-cis retinal d'invertébré
● Lipides et composés à bases de lipides
   . Cannabinoides
   . Anandamide
   . Acide lysophosphatidique
   . Facteur d'activation des plaquettes
   . Leukotriènes
● Amino acides excitateurs et ions
   . Ion Calcium
   . Glutamate
● Récepteurs orphelins
   . Récepteurs olfactifs

Le ligand peut être soit un agoniste, soit un antagoniste.

Par "agoniste", on entend toute molécule mimant l'effet du ligand endogène naturel, par exemple le neurotransmetteur, le facteur de croissance, l'hormone.

Par "antagoniste", on entend toute molécule inhibant l'effet de l'agoniste en se liant sur la même protéine cible que ce dernier.

L'invention concerne un procédé de détection et de quantification d'interactions non covalentes entre un récepteur et l'un de ses ligands, caractérisé en ce que :
- on prépare des cellules soit des fragments de cellules contenant une séquence d'ADN comprenant le gène codant pour une protéine fluorescente fusionnée avec le gène de la protéine cible, la fusion entre le gène de la protéine fluorescente et le gène de la susdite protéine cible étant telle que les propriétés de la protéine cible récepteur, ne sont pas modifiées par la présence de la protéine fluorescente, à savoir :
   * l'interaction entre la protéine cible récepteur, et le ligand n'est pas modifiée,
   * la fonction de transduction de la réponse n'est pas modifiée, la protéine fluorescente étant choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
- la protéine fluorescente verte (GFP), ou
- des variants dérivés de la GFP, par addition , délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence,
- on met en présence les susdites cellules ou les susdits fragments de cellules avec un ligand de la susdite protéine cible récepteur, marqué par un marqueur constitué :
   - soit par une molécule susceptible d'absorber la lumière émise par la protéine fluorescente,
   - soit par une substance fluorescente,
   et soit la protéine fluorescente étant donneur d'énergie de fluorescence et le marqueur étant accepteur d'énergie de fluorescence, ou soit la protéine fluorescente étant accepteur d'énergie de fluorescence et le marqueur étant une substance fluorescente donneur d'énergie de fluorescence, et
- on irradie à une longueur d'onde permettant soit d'exciter la protéine fluorescente, soit d'exciter la substance fluorescente,
- les susdites étapes de mise en présence et d'irradiation pouvant être effectuées soit simultanément, soit l'une après l'autre, ou
- on met en présence les susdites cellules ou les susdits fragments de cellules avec un ligand de la susdite protéine, notamment du susdit récepteur, marqué par un marqueur, les cellules ou le ligand ayant été irradiés préalablement à leur mise en présence,
- on détecte soit une diminution d'amplitude de l'émission du donneur et/ou un signal d'émission caractéristique de l'émission de l'accepteur.

. Cellules, fragments et protéine purifiée :

Par cellule, on entend toute cellule eucaryote ou procaryote, animale ou végétale dans laquelle on introduit le gène codant pour une protéine de fusion entre un récepteur (décrits) et la GFP ou ses mutants ou fragments.

Par fragment de cellule, on entend toute fraction, membranaire ou non membranaire, obtenue a partir des cellules et contenant la protéine de fusion récepteur-GFP.

Par protéine purifiée, on entend protéine de fusion partiellement ou totalement purifiée.

Selon un mode de réalisation avantageux, le procédé utilise l'ADNc du variant EGFP de la protéine de fluorescence verte fusionnée à un récepteur en association avec un ligand dudit récepteur marqué par le Bodipy 530-550. Le système d'expression préféré est la cellule mammifère ou la levure.

L'invention a également pour objet un procédé de détection et de quantification d'interactions non covalentes entre une protéine cible, récepteur, et l'un de ses ligands, caractérisé en ce que :
- on prépare une protéine fluorescente fusionnée avec une protéine cible dont on veut déterminer l'interaction protéine-ligand, la fusion entre la protéine fluorescente et la susdite protéine cible étant telle que les propriétés de la protéine, notamment du récepteur, ne sont pas modifiées par la présence de la protéine fluorescente, à savoir :
   * l'interaction entre la protéine cible, notamment le récepteur, et le ligand n'est pas modifiée,
   ^{*} la fonction de transduction de la réponse n'est pas modifiée,
   la protéine fluorescente étant choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
   - la protéine fluorescente verte (GFP), ou
   - des variants dérivés de la GFP, par addition , délétion ou substitution d'un ou plusieurs acides aminés, sous réserve que ces variants conservent la propriété de fluorescence,
   - ou des fragments de la GFP, ou de fragments des susdits variants, sous réserve que ces fragments conservent la propriété de fluorescence,
   - on met en présence la susdite protéine fluorescente fusionnée avec la protéine cible avec un ligand de la susdite protéine récepteur, ce ligand étant marqué par un marqueur constitué :

   - soit par une molécule susceptible d'absorber la lumière émise par la protéine fluorescente,
   - soit par une substance fluorescente,
   et soit la protéine fluorescente étant donneur d'énergie de fluorescence et le marqueur étant accepteur d'énergie de fluorescence, ou soit la protéine fluorescente étant accepteur d'énergie de fluorescence et le marqueur est une substance fluorescente donneur d'énergie de fluorescence, et
- on irradie à une longueur d'onde permettant soit d'exciter la protéine fluorescente, soit d'exciter la substance fluorescente,
- les susdites étapes de mise en présence et d'irradiation pouvant être effectuées soit simultanément, soit l'une après l'autre, ou
- on met en présence la susdite protéine fluorescente fusionnée avec la protéine cible avec un ligand de la susdite protéine récepteur, ce ligand étant marqué par un marqueur constitué :

- soit par une molécule susceptible d'absorber la lumière émise par la protéine fluorescente,
- soit par une substance fluorescente,
   la protéine fluorescente fusionnée avec la protéine cible ou le ligand ayant été irradiés préalablement à leur mise en présence,
- on détecte soit une diminution d'amplitude de l'émission du donneur et/ou un signal d'émission caractéristique d'émission de l'accepteur.

La protéine cible et notamment le récepteur modifié par fusion avec la GFP devra, outre exhiber la fluorescence de la GFP, conserver des propriétés pharmacologiques compatibles avec sa définition de récepteur spécifique des composés le caractérisant. En particulier, il devra pouvoir lier les même ligands que le récepteur sauvage.

Dans le procédé de l'invention, la mise en présence de la protéine fluorescente fusionnée avec la protéine cible et du ligand, ou de cellules ou fragments de cellules (contenant la susdite protéine fluorescente fusionnée avec la protéine cible) et du ligand conduit à un mélange.

Le mélange peut être effectué dans n'importe quel ordre d'addition. L'ordre préférentiel sera l'addition de ligand fluorescent à une solution de cellules ou de protéine, si la GFP est donneur, l'inverse si la GFP est accepteur.

Le mélange peut être effectué dans un appareil de mélange rapide combiné à un système de détection de fluorescence pour avoir accès à des mesures en temps réel de l'interaction récepteur ligand et de ses altérations par des molécules biologiquement actives.

. Introduction d'ADN dans les cellules :

Les cellules animales., végétales, d'insectes, les levures, les bactéries ou les champignons constituant le système d'expression choisi pourront soit être transfectées, transformées, électroporées, ou infectées, de manière stable ou transitoire selon les protocoles décrits dans les manuels de laboratoire, par exemple Current Protocols in Molecular Biology, eds Ausabel *et al.,* John Wiley and sons.

L'invention a également pour objet un procédé dans lequel on introduit une étape supplémentaire :
- avant ou après, ou simultanément à l'étape de mise en présence de la protéine fluorescente fusionnée avec la protéine cible et d'un ligand de la susdite protéine, ce ligand étant marqué par un marqueur, ou
- avant, ou après, ou simultanément à l'étape de mise en présence de cellules ou de fragments de cellules et d'un ligand de la susdite protéine, marqué par un marqueur,
cette étape supplémentaire consistant :
- soit à mettre la susdite protéine fluorescente fusionnée avec la protéine cible en présence du susdit ligand non marqué et simultanément du susdit ligand marqué,
- soit à mettre en présence les susdites cellules ou les susdits fragments de cellules en présence simultanée du susdit ligand non marqué et du susdit ligand marqué,
- on détecte soit une diminution d'amplitude de l'émission du donneur et/ou un signal d'émission caractéristique de l'émission de l'accepteur, respectivement dans le cas de l'utilisation du ligand marqué et dans le cas de l'utilisation simultanée du ligand marqué et du ligand non marqué,
- et on compare soit les diminutions d'amplitude de l'émission du donneur respectivement obtenues et/ou les signaux d'émission caractéristiques de l'émission de l'accepteur respectivement obtenus.

Dans ce cas, il y a compétition entre le ligand marqué et le ligand non marqué, vis à vis de la protéine cible. L'intérêt d'un tel procédé est de pouvoir être adapté sur des automates pour identifier de nouvelles molécules capables d'interagir avec la protéine cible et se comportant soit comme agoniste soit comme antagoniste vis-à-vis de la réponse biologique. L'analyse, à différentes concentrations de la molécule non marquée, de l'inhibition de l'interaction entre la protéine cible et son ligand fluorescent permet par ailleurs de déterminer les paramètres d'affinité de la molécule non marquée pour la protéine cible.

L'invention a également pour objet un procédé, dans lequel la protéine fluorescente est EGFP et dans lequel :
- soit la EGFP est donneur d'énergie de fluorescence et le marqueur est accepteur d'énergie de fluorescence et est choisie parmi des substances, dont le spectre d'excitation chevauche le spectre d'émission de la EGFP, et notamment dans le cas où le marqueur est une substance fluorescente, il est choisi parmi : le 4,4-difluoro-4-bora-3a,4a-diaza-s-indacene (Bodipy), l'éosine, l'érythrosine, la tétraméthylrhodamine, la sulforhodamine 101 commercialisée par Molecular probe sous la dénomination Texas Red, et leurs dérivés permettent d'une part le greffage et, d'autre part, dont le spectre d'excitation recouvre le spectre d'émission de EGFP,
   et dans le cas où le marqueur n'est pas une substance fluorescente, il est choisi parmi le groupe des acides violets [Acid Violet 5, CAS 10130-48-0 ; Acid Violet 7, CAS 4321-69-1 ; Acid Violet 17, CAS 4129-84-4], acides rouges [Acid Red 1, CAS 3734-67-6 ; Acid Red 8, CAS 4787-93-3 ; Acid Red 37, CAS 6360-07-2 ; Acid Red 40, CAS 12167-45-2 ; Acid Red 106, CAS 6844-74-2 ; Acid Red 114, CAS 6459-94-5], les alizarines, l'aluminon, l'azocarmine B [CAS 25360-72-9], la fuschine basique [Basic Red 9, CAS 569-61-9], le Bordeaux R [Acid Red 17, CAS 5858-33-3], la Carmine [CAS 1390-65-4],
- soit la EGFP est accepteur d'énergie de fluorescence et la substance fluorescente est donneur d'énergie de fluorescence et est choisie parmi des substances, dont le spectre d'émission chevauche le spectre d'excitation de la EGFP, et notamment parmi : les coumarines, la fluorescamine, le 6-(N-méthylanilino)naphtalène, (mansyl) et leurs dérivés permettent d'une part le greffage et, d'autre part, dont le spectre d'émission recouvre le spectre d'excitation de EGFP.

L'invention a également pour objet un procédé dans lequel la protéine dont on veut déterminer l'interaction protéine-ligand est choisie parmi:
- les récepteurs membranaires couplés à la protéine G, notamment dans Supplement Trends in Pharmacological Sciences, 1997 (*Receptor and ion Channel Nomenclature*),
- les récepteurs des facteurs de croissance, notamment ceux qui sont structurellement reliés au récepteur de l'insuline (Yarden, Y. and Ullrich, A. 1988, Biochemistry 27:3113-3119) ou au récepteur de l'interféron γ (Brisco, J. *et al.* 1996, Phylos. Trans. R. Soc. Lond. B. Biol. Sci. 351:167-171 ; Ihle, J.N. 1995, Nature 377:591-594),
- les récepteurs canaux, notamment dans Supplement Trends in Pharmacological Sciences, 1997 (*Receptor and ion Channel Nomenclature*),

L'invention a également pour objet un procédé dans lequel la protéine fluorescente est la EGFP et la substance marquée est le Bodipy et dans lequel on détecte soit la diminution d'amplitude d'émission de la EGFP, soit le signal d'émission du Bodipy résultant du transfert d'énergie, la longueur d'onde d'irradiation correspondant à la longueur d'onde d'excitation de la EGFP.

L'invention a également pour objet un procédé dans lequel la protéine fluorescente est la EGFP et la substance marquée est une coumarine et dans lequel on détecte soit la diminution d'amplitude de la coumarine, soit le signal d'émission de la EGFP résultant du transfert d'énergie, la longueur d'onde d'irradiation correspondant à la longueur d'onde d'excitation de la coumarine.

L'invention concerne également un procédé dans lequel la protéine fluorescente est fusionnée du côté N-terminal et la protéine cible, notamment le récepteur, est fusionné du côté C-terminal.

L'invention concerne également un procédé dans lequel la protéine fluorescente est fusionnée du côté C-terminal et la protéine cible, notamment le récepteur, est fusionné du côté N-terminal.

L'invention a également pour objet un procédé dans lequel la protéine fluorescente est insérée dans la protéine cible à un endroit ne correspondant pas à un site de liaison protéine cible-ligand, notamment dans le cas des récepteurs couplés à la protéine G, cette insertion ayant lieu dans la première ou la troisième boucle intracellulaire du récepteur, sous réserve que l'insertion ne détruise ni les propriétés du récepteur, ni la fluorescence de la protéine fluorescente.

L'invention concerne également un procédé dans lequel les cellules sont des cellules de mammifères, notamment les cellules HEK 293 adhérentes ou en suspension, cellules CHO, cellules COS, lignées lymphocytaires, fibroblastes, etc.., ou des cellules de levure, notamment *pichia* telle que *pichia pastoris, saccharomyces* telle que *saccharomyces cerevisia, saccharomyces kluyveri, Hansenula* telle que *hansenula polymorpha,* ou des cellules d'insectes infectées par un virus tel que *baculovirus,* notamment cellules TNI ou sf9, ou des champignons, notamment les souches de *Aspergillus* (*A. oryzae, A. nidulans, A. niger*), *Neurospora, Fusarium, Trichoderma.*

L'utilisation de *Aspergillus* pour l'expression de protéines est décrite dans EP 0,272,277 ou EP 0,230,023 ou EP 0,184,438, ou des cellules de plantes, notamment *Arabidopsis* (*A. thaliana*), ou des protoplastes de citrange (*Citrus sinensis*) est décrite dans Haseloff, J. and Amos, B. 1995, Trends in Genetics 11:328-329.

S'agissant de la lignée cellulaire utilisée, elle dépend du récepteur; il est en effet souhaitable de choisir une lignée n'exprimant pas déjà de manière naturelle le récepteur choisi.

L'invention a également pour objet un procédé dans lequel un signal est détectable, dans un appareil de fluorimétrie conventionnel ou dans un appareil de mélange rapide équipé d'un système de détection de fluorescence, après mélange du donneur et de l'accepteur et peut être aboli par l'addition d'une substance non fluorescente de même spécificité pharmacologique, et notamment dans lequel le rapport signal/bruit est supérieur à environ 2.

Pour fixer les idées et à titre d'exemple, le rapport signal/bruit est d'environ 100 avec le couple BODIPY-EGFP sur l'équipement utilisé (décrit dans les exemples) et avec le récepteur de la substance qu'on désignera par NK2R marquée par la EGFP (NK2R-EGFP) et son ligand, la substance K (NKA) marquée par le BODIPY (NKA-BO).

Concernant le signal et le rapport signal sur bruit, il peut être défini comme suit :

Si le mélange du donneur et de l'accepteur est accompagné d'un changement de fluorescence, soit du donneur, soit de l'accepteur, ce changement doit être inhibé et renversé par une substance non fluorescente (de même spécificité pharmacologique) et qui de plus définit la spécificité du phénomène pharmacologique observé. Pour certaines applications (réponse de type oui-non) un faible rapport signal sur bruit peut suffire.

S'agissant de la protéine cible, il est possible de faire des mesures avec des cellules entières (contenant l'ADN codant pour la protéine cible), des fragments de cellules (membranes pour des récepteurs membranaires) des échantillons de protéines cibles solubilisés (récepteurs membranaires) ou purifiés (cf. exemples).

L'invention a également pour objet l'utilisation d'un protéine fluorescente choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
- la protéine fluorescente verte (GFP), ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence,
pour la détection et la quantification d'interactions non covalentes entre une protéine cible constituée par un récepteur couplé aux protéines G et une protéine G, en vue d'identifier les molécules biologiquement actives vis-à-vis du récepteur, susceptibles de former une interaction non covalente réversible avec ledit récepteur, ledit récepteur étant marqué par voie génétique par la protéine fluorescente et la protéine G étant marquée par un marqueur constitué :
- soit par une molécule susceptible d'absorber la lumière émise par la protéine fluorescente,
- soit par une substance fluorescente pouvant être notamment choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
   - la protéine fluorescente verte (GFP), ou
   - des variants dérivés de la GFP, par addition , délétion ou substitution d'un ou plusieurs acides aminés, sous réserve que ces variants conservent la propriété de fluorescence,
   - ou des fragments de la GFP, ou de fragments des susdits variants, sous réserve que ces fragments conservent la propriété de fluorescence,
      cette détection et quantification ayant lieu par transfert d'énergie de fluorescence entre le récepteur marqué par la GFP ou l'un de ses variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'excitation de la GFP, ou de l'un des susdits variants, ou de l'un des susdits fragments, ou
      . entre la GFP ou l'un de ses variants définis ci-dessus, ou l'un des fragments définis ci-dessus, et la susdite molécule susceptible d'absorber la lumière émise par la protéine fluorescente.

L'invention a également pour objet l'utilisation d'une protéine G marquée par un marqueur constitué:
- soit par une molécule susceptible d'absorber la lumière émise par la protéine fluorescente,
- soit par une substance fluorescente pouvant être notamment choisie parmi les protéines fluorescentes issues ou dérivées de protéines auto fluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
   - la protéine fluorescente verte (GFP), ou
   - des variants dérivés de la GFP, par addition , délétion ou substitution d'un ou plusieurs acides aminés, sous réserve que ces variants conservent la propriété de fluorescence,
   - ou des fragments de la GFP, ou de fragments des susdits variants, sous réserve que ces fragments conservent la propriété de fluorescence,
   pour la détection et la quantification d'interactions non covalentes entre une protéine cible constituée par un récepteur couplé aux protéines G et la susdite protéine G, en vue d'identifier les molécules biologiquement actives vis-à-vis, du récepteur, susceptibles de former une interaction non covalente réversible avec ledit récepteur, ledit récepteur étant marqué par voie génétique par une protéine fluorescente choisie parmi les protéines fluorescentes issues ou dérivées de protéines autofluorescentes de cnidaires dont le coefficient d'extinction moléculaire est supérieur à environ 14000M⁻¹cm⁻¹ et le rendement quantique de fluorescence est supérieur à environ 0,38, notamment choisie parmi :
   - la protéine fluorescente verte (GFP), ou
   - des variants dérivés de la GFP, par addition, délétion ou substitution d'un ou plusieurs acides aminés, sous réserve que ces variants conservent la propriété de fluorescence,
   - ou des fragments de la GFP, ou de fragments des susdits variants, sous réserve que ces fragments conservent la propriété de fluorescence,
   cette détection et quantification ayant lieu par transfert d'énergie de fluorescence:
   . entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'excitation de la GFP, ou de l'un des susdits variants, ou de l'un des susdits fragments, ou
   . entre la GFP ou l'un de ses variants définis ci-dessus, ou l'un des fragments définis ci-dessus, et la susdite molécule susceptible d'absorber la lumière émise par la protéine fluorescente.

Selon un mode de réalisation avantageux du procédé de l'invention, la protéine fluorescente est choisie parmi EGFP, ECFP, EYFP, ou leurs mutants pour lesquels le coefficient d'extinction moléculaire est supérieur à environ 14000 M⁻¹cm⁻¹ et le rendement quantique est supérieur à environ 0,38.

Selon un mode de réalisation avantageux le récepteur est choisi parmi :
- les récepteurs couplés aux protéines G, notamment ceux décrits dans Supplément Trends in Pharmacological Sciences, 1997 (*Receptor and ion Channel Nomenclature*),
- les séquences codant pour des récepteurs couplés aux protéines G putatifs dont les molécules biologiquement actives vis-à-vis d'eux-mêmes sont à identifier, notamment choisies parmi les séquences de récepteur orphelins disponibles dans les banques de séquence Genbank, EMBL et banques affiliées.

Selon un autre mode de réalisation avantageux, la protéine G est choisie parmi les protéines G décrites dans Journal of Receptor Research, vol 13, pp19-26, 1993 ou Angewandte Chemie, ed. Engl. vol 34, pp1406-1419, 1995.

L'invention concerne également un procédé de détection et de quantification d'interactions non covalentes entre une protéine cible constituée par un récepteur couplé aux protéines G et une protéine G, en vue d'identifier les molécules biologiquement actives vis-à-vis du récepteur, susceptibles de former une interaction non covalente réversible avec ledit récepteur , caractérisé en ce que :
- on prépare des cellules soit des fragments de cellules exprimant une séquence d'ADN comprenant le gène codant pour une protéine fluorescente fusionnée avec le gène du récepteur couplé aux protéines G, la fusion entre le gène de la protéine fluorescente et le gène du susdit récepteur étant telle que les propriétés du récepteur ne sont pas modifiées par la présence de la protéine fluorescente, à savoir :
   * l'interaction entre le récepteur et la protéine G n'est pas modifiée,
   * l'interaction entre le récepteur et la molécule biologiquement active n'est pas modifiée,
   * la fonction de transduction de la réponse n'est pas modifiée, la protéine fluorescente étant choisie parmi les protéines fluorescentes issues ou dérivées de protéines autofluorescentes de cnidaires dont le coefficient d'extinction moléculaire est supérieur à environ 14000M⁻¹cm⁻¹ et le rendement quantique de fluorescence est supérieur à environ 0,38, notamment choisie parmi :
      - la protéine fluorescente verte (GFP), ou
      - des variants dérivés de la GFP, par addition , délétion ou substitution d'un ou plusieurs acides aminés, sous réserve que ces variants conservent la propriété de fluorescence,
      - ou des fragments de la GFP, ou de fragments des susdits variants, sous réserve que ces fragments conservent la propriété de fluorescence, la protéine G étant marquée par un marqueur constitué :

      - soit par une molécule susceptible d'absorber la lumière émise par la protéine fluorescente,
      - soit par une substance fluorescente,
      - la protéine fluorescente et le susdit marqueur étant tels qu'ils peuvent transférer l'énergie de l'un à l'autre, la protéine fluorescente pouvant être donneur d'énergie ou le susdit marqueur pouvant être donneur d'énergie,
   l'interaction entre le récepteur marqué par la protéine fluorescente et la protéine G marquée par un marqueur susdéfini étant détectée par transfert d'énergie de fluorescence.

L'invention concerne également un procédé d'identification et éventuellement de quantification d'interactions entre un récepteur et une molécule non fluorescente biologiquement active vis-à-vis dudit récepteur, susceptibles de former une interaction non covalente réversible avec ledit récepteur, par mise en oeuvre du procédé défini ci-dessus, dans lequel une molécule non fluorescente biologiquement active est ajoutée à des cellules, ou fragments de cellules, exprimant l'ADN codant pour le récepteur marqué par la protéine fluorescente et pour la protéine G marquée par le marqueur, caractérisé en ce que :
- une molécule non fluorescente biologiquement active et agoniste déclenche une transduction de signal détectée par variation de transfert d'énergie entre le récepteur marqué par la protéine fluorescente et la protéine G marquée par le marqueur ;
- une molécule non fluorescente biologiquement active antagoniste inhibe la transduction de signal provoquée par un agoniste et détectée par variation de transfert d'énergie de fluorescence entre le récepteur marqué par la protéine fluorescente et la protéine G marquée par le marqueur.

L'invention a pour objet des cellules ou fragment de cellules contenant une séquence d'ADN comprenant le gène codant pour une protéine fluorescente fusionnée avec le gène d'une protéine cible, la protéine fluorescente étant choisie parmi les protéines fluorescentes issues ou dérivées de protéines autofluorescentes de cnidaires, dont le coefficient d'extinction moléculaire est supérieur à environ 14000M⁻¹cm⁻¹et le rendement quantique de fluorescence est supérieur à environ 0,38, la fusion entre le gène de la protéine fluorescente et le gène de la susdite protéine cible étant telle que
* les propriétés de la protéine cible ne sont pas modifiées par la présente de la protéine fluorescente, à savoir
* l'interaction entre la protéine cible et le ligand n'est pas modifiée,
* la fonction de transduction de la réponse n'est pas modifiée,
sous réserve que :
* lorsque la protéine cible est le récepteur des glucocorticoïdes de rat fusionné en N-terminal avec successivement une séquence de purification comportant 6 histidines, un épitope hémaglutinine et une protéine fluorescente et est exprimée dans la lignée cellulaire 1471.1 (Htun *et al.* 1996, PNAS 93:4845-4850), la protéine fluorescente est différente de la GFP (768 paires de bases du plasmide TU65 avec la mutation S65T)(Chalfie *et al.* 1994, Science 263:802-805, avec la mutation S65T),
* lorsque la protéine cible est le récepteur humain des glucocorticoïdes tronqué de ses 131 premiers amino acides, fusionné en C-terminal d'une protéine fluorescente dans les sites Sal I et BamH I et est exprimée dans les cellules Cos-1 (Ogawa *et al.* 1995, PNAS 92:11899-11903), ladite protéine fluorescente est différente de la GFP telle que décrite dans l'article de Inouye S. et Tsuji, F. I., 1994, Febs Letters, 341:277-280,
* lorsque la protéine cible est la sous-unité NMDA R1 de rat exprimé dans les cellules HEK 293 (selon Marshall *et al.* 1995, Neuron 14:211-21.5) fusionnée en C-terminal avec une protéine fluorescente, la protéine fluorescente est différente de celle constituée par les acides aminés 2-238 de la GFP sauvage (Chalfie *et al.* 1994, Science 263:802-805),
* lorsque la protéine cible est un récepteur ou un fragment de récepteur de messagers secondaires intracellulaires, la protéine fluorescente est différente de la GFP et de ses dérivés (WO96/23898).

L'invention a également pour objet des cellules ou fragment de cellules contenant une séquence d'ADN comprenant le gène codant pour une substance fluorescente fusionnée avec le gène d'un ligand, la substance fluorescente étant choisie parmi les protéines fluorescentes issues ou dérivées de protéines autofluorescentes de cnidaires, dont le coefficient d'extinction moléculaire est supérieur à 14000 M⁻¹cm⁻¹ et le rendement quantique de fluorescence est supérieur à 0,38, la fusion entre le gène de la substance fluorescente et le.gène du ligand étant tel que
* les propriétés du ligand ne sont pas modifiées par la présence de la substance fluorescente, à savoir
* l'interaction entre la protéine cible et le ligand n'est pas modifiée,
* la fonction de transduction de la réponse n'est pas modifiée.

La présente invention a également pour objet des cellules ou fragments de cellules tels que définis ci-dessus, caractérisés en ce que le ligand est constitué par la protéine G,
- et comprenant le gène codant pour une protéine fluorescente fusionnée avec le gène d'une protéine cible constituée par un récepteur, la protéine fluorescente étant choisie parmi les protéines fluorescentes issues ou dérivées de protéines autofluorescentes de cnidaires, telles que définies ci-dessus,
- la fusion entre le gène de la substance fluorescente et le gène de la susdite protéine G, et la fusion entre le gène de la protéine fluorescente et le gène du récepteur étant telle que
   * les propriétés du récepteur ne sont pas modifiées par la présence de la protéine fluorescente, à savoir
   * la fonction de transduction de la réponse n'est pas modifiée, sous réserve que:
      * lorsque la protéine cible est le récepteur des glucocorticoïdes de rat fusionné en N-terminal avec successivement une séquence de purification comportant 6 histidines, un épitope hémaglutinine et une protéine fluorescente et est exprimée dans la lignée cellulaire 1471.1, la protéine fluorescente est différente de la GFP (768 paires de bases du plasmide TU65 avec la mutation S65T),
      * lorsque la protéine cible est le récepteur humain des glucocorticoïdes tronqué de ses 131 premiers amino acides, fusionné en C-terminal d'une protéine fluorescente dans les sites Sal I et BamH I et est exprimée dans les cellules Cos-1, ladite protéine fluorescente est différente de la GFP telle que décrite dans l'article de Inouye S. et Tsuji, F. I., 1994, Febs Letters, Vol. 341, p.277-280,
      * lorsque la protéine cible est la sous-unité NMDA R1 de rat exprimé dans les cellules HEK 293 fusionnée en C-terminal avec une
- protéine fluorescente, la protéine fluorescente est différente de celle constituée par les acides aminés 2-238 de la GFP sauvage,
   * lorsque la protéine cible est un récepteur ou un fragment de récepteur de messagers secondaires intracellulaires, la protéine fluorescente est différente de la GFP et de ses dérivés.

L'invention a également pour objet une trousse ou nécessaire pour la détection et la quantification d'interactions non covalentes entre une protéine cible membranaire marquée par une protéine fluorescente et l'un de ses ligands marqué par un marqueur constitué :
- soit par une molécule susceptible d'absorber la lumière émise par la protéine fluorescente,
- soit par une substance fluorescente,
laquelle protéine fluorescente est choisie parmi les protéines fluorescentes issues ou dérivées de protéines autofluorescentes de cnidaires, dont le coefficient d'extinction moléculaire est supérieur à environ 14000M⁻¹cm⁻¹ et le rendement quantique de fluorescence est supérieur à environ 0,38, notamment choisie parmi :
- la protéine fluorescente verte (GFP), ou
- des variants dérivés de la GFP, par addition , délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence et son ligand marqué par une substance fluorescente, ladite trousse comprenant :

- la protéine cible fusionnée avec une protéine fluorescente ou une lignée cellulaire stable susceptible d'exprimer la protéine fusionnée avec une protéine fluorescente ou un plasmide contenant la séquence nucléique codant pour ladite protéine cible fusionnée avec une protéine fluorescente telle que définie ci-dessus,
- le ligand marqué par le susdit marqueur,
- les tampons et milieux nécessaires au transfert d'énergie entre la susdite protéine et le susdit ligand.

L'invention a pour objet une trousse ou nécessaire pour la détection et la quantification d'interactions non covalentes entre une protéine cible membranaire marquée par une protéine fluorescente (n° 1) et l'un de ses ligand marqué par une substance fluorescente correspondant à une protéine fluorescente (n° 2), la protéine fluorescente (n° 1) étant choisie parmi la protéine fluorescente EYFP ou EGFP et le ligand étant marqué par la protéine fluorescente (n° 2) ECFP ou la protéine fluorescente
(n° 1) étant ECFP et le ligand étant marqué par la protéine fluorescente (n° 2) EYFP ou EGFP, ladite trousse comprenant :
- soit un plasmide contenant un acide nucléique codant pour la protéine cible fusionnée avec une protéine fluorescente (n° 1), et
   * un plasmide contenant un acide nucléique codant pour le ligand fusionné avec une protéine fluorescente (n° 2), ou
   * un ligand fusionné avec une protéine fluorescente (n° 2), obtenu par voie recombinante et purifié,
- soit une lignée cellulaire stable susceptible d'exprimer la protéine cible fusionnée avec une protéine fluorescente (n° 1), et
   * une lignée cellulaire stable susceptible d'exprimer le ligand fusionné avec une protéine fluorescente (n° 2), ou
   * un ligand fusionné avec une protéine fluorescente (n° 2), obtenu par voie recombinante et purifié,
- les tampons et milieux nécessaires au transfert d'énergie entre la susdite protéine et le susdit ligand.

L'invention a pour objet une trousse ou nécessaire pour la détection et la quantification d'interactions non covalentes entre une protéine cible constituée par un récepteur couplé à la protéine G marquée par une protéine fluorescente (n° 1) et la protéine G marquée par une substance fluorescente correspondant à une protéine fluorescente (n° 2), la protéine fluorescente (n° 1) étant choisie parmi la protéine fluorescente EYFP ou EGFP et la protéine G étant marquée par la protéine fluorescente (n° 2) ECFP ou la protéine fluorescente (n° 1) étant ECFP et la protéine G étant marquée par la protéine fluorescente (n° 2) EYFP ou EGFP, ladite trousse comprenant :
- soit un plasmide contenant un acide nucléique codant pour le récepteur fusionné avec une protéine fluorescente (n° 1), et
   * un plasmide contenant un acide nucléique codant pour la protéine G fusionnée avec une protéine fluorescente (n° 2), ou
   * la protéine G fusionnée avec une protéine fluorescente (n° 2), obtenue par voie recombinante et purifiée,
- soit une lignée cellulaire stable susceptible d'exprimer le récepteur fusionné avec une protéine fluorescente (n° 1), et
   * une lignée cellulaire stable susceptible d'exprimer la protéine G fusionnée avec une protéine fluorescente (n° 2), ou
   * la protéine G fusionnée avec une protéine fluorescente (n° 2), obtenue par voie recombinante et purifiée,
- les tampons et milieux nécessaires au transfert d'énergie entre le susdit récepteur et la susdite protéine G.

### DESCRIPTION DETAILLEE DE L'INVENTION

Dans sa réalisation préférée, le développement de l'invention utilise l'ADNc codant pour la protéine fluorescente verte (Prasher *et al.* Gene 1992, 111:229-233; GenBank Accession N° M62653) de la méduse *Aequorea victoria,* préférentiellement les mutants GFPUV et RSGFP de cette protéine fluorescente optimisés pour leur expression dans les organismes hôtes préférés, les cellules mammifères.

L'ADNc peut être modifié pour coder pour un variant dans lequel un ou plusieurs acides aminés sont substitués, insérés ou délétés afin de permettre sa fusion, en N ou en C-terminal avec le gène codant pour une protéine cible.

La protéine cible récepteur, peut être choisie parmi :
1) les récepteurs de neurotransmetteurs couplés à des protéines G structurellement reliés aux récepteurs adrénergiques et récepteurs métabotropiques du glutamate tels que présentés dans la liste actualisées annuellement et éditée comme supplément sous le nom : "Receptor and Ion Channel Nomenclature" par Elsevier Trends journals, dans Trends in Pharmacological Sciences.
2) les récepteurs-canaux structurellement reliés aux récepteurs nicotiniques, au récepteurs de glutamate et au récepteur de l'ATP, tels que présentés dans la liste actualisées annuellement et éditée comme supplément sous le nom: "Receptor and Ion Channel Nomenclature" par Elsevier Trends journals dans Trends in Pharmacological Sciences.
3) les récepteurs nucléaires possédant un domaine d'interaction avec l'ADN structurellement reliés au récepteur des stéroïdes (Mangelsdorf *et al.* 1995, Cell, 83:835-839, Wurtz, J.L. *et al.* 1996, Nature Struct. Biol. 3:206).
4) les récepteurs de la membrane plasmique à activité tyrosine kinase structurellement reliés au récepteur de finsuline (Yarden, Y. and Ullrich, A. 1988, Biochemistry 27:3113-3119).
5) les récepteurs membranaires couplés aux protéines tyrosine kinases (STATs, TYK2, Jak) structurellement reliés au récepteur de l'interféron γ (Brisco, J. *et al.* 1996, Phylos. Trans. R. Soc. Lond. B. Biol. Sci. 351:167-171 ; Ihle, J.N. 1995, Nature 377:591-594).

Dans le cas où la fusion est effectuée entre la EGFP et un récepteur couplé aux protéines G (groupe 1), la fusion peut être notamment effectuée :
1) du coté N-terminal du récepteur, et donc du côté C-terminal de la EGFP,
2) du coté C-terminal du récepteur et donc du côté N-terminal de la EGFP,
3) dans la séquence du récepteur, en particulier dans la première ou la troisième boucle intracellulaire, éventuellement en introduisant une ou plusieurs copies d'une séquence espaceur, notamment -GGGGS-.

Dans le cas où la fusion est effectuée entre la EGFP et un récepteur-canal (groupe 2), la fusion peut être notamment effectuée:
1) dans la région homologue à la "région immunogénique majeure" de la sous-unité α du récepteur nicotinique de Torpille (résidus 67-76), éventuellement en introduisant une ou plusieurs copies d'une séquence espaceur, notamment -GGGGS-.

Dans le cas ou la fusion est effectuée entre la EGFP et un récepteur nucléaire (groupe 3), la fusion peut être notamment effectuée:
1) du coté N-terminal du récepteur, et donc du côté C-terminal de la EGFP,
2) du coté N-terminal du récepteur, tronqué dans sa partie N-terminale en amont du domaine de liaison à l'ADN, et donc du côté C-terminal de la EGFP.

Dans le cas où la fusion est effectuée entre la EGFP et un récepteur soit à activité tyrosine kinase, soit couplé à une tyrosine kinase (groupes 4 et 5), la fusion peut être notamment effectuée:
1) du coté N-terminal du récepteur, et donc du coté C-terminal de la EGFP,

Tout gène codant pour une protéine fluorescente, notamment la GFP, couplée à un récepteur, et dérivant d'organismes exprimant la GFP ou des protéines similaires pourraient être utilisés dans cette invention.

Les séquences d'ADN codant pour la GFP et les protéines cibles, notamment des récepteurs, peuvent être d'origine génomique ou être des ADNc, et peuvent être obtenus à partir de l'ADN de n'importe quelle espèce animale ou végétale, eucaryote ou procaryote, par exemple en préparant des banques génomiques ou des banques d'ADNc et en criblant ces banques pour identifier les séquences codantes par hybridation avec des sondes oligonucléotidiques par les techniques standard (Current Protocols in Molecular Biology, op. cit.).

Les constructions d'ADN codant pour la GFP et les protéines cibles peuvent aussi être obtenues par synthèse totale par les méthodes standards, notamment la méthode des phosphoramidites (Beaucage and caruthers, Tett. Lett. 1981, 22:1859-1869) et l'emploi d'appareils de synthèse d'ADN automatisés, le polynucléotide obtenus étant ensuite purifiés, ligués et clonés dans les vecteurs appropriés. Pour la plupart des applications, les gènes codant pour la GFP et les protéines cibles seront préférentiellement obtenus par criblage de banques, tandis que les bras espaceurs ainsi que les oligonucleotides requis pour la mutagenèse seront préférentiellement obtenus par synthèse.

Les constructions d'ADN pourront être de nature mixte, synthétique et génomique, par ligation de fragments synthétiques avec des élément d'ADN génomique, selon des procédures standard (Current Protocols in Molecular Biology, op.cit.).

Les constructions d'ADN peuvent aussi être obtenues par PCR (polymerase chain reaction) en employant des amorces spécifiques, comme par exemple décrit dans PCR protocol 1990, Academic press, San Diego, California, USA.

Enfin, les constructions d'ADN peuvent être modifiées par d'autres méthodes incluant par exemple des réactions chimiques, de la mutagenèse aléatoire ou dirigée, par insertion, délétion ou substitution de nucléotides, ces modifications pouvant altérer des propriétés de l'une ou l'autre protéine, notamment, la GFP et les protéines cibles.

Les constructions d'ADN peuvent être insérées dans un vecteur recombinant. Ce vecteur peut être n'importe quel vecteur approprié pour les procédures employées avec des vecteurs recombinants. Le choix du vecteur sera souvent effectué en fonction de la cellules hôte dans laquelle a construction d'ADN voudra être introduite. Le vecteur pourra ainsi être un vecteur capable de se répliquer de manière autonome, c'est-à-dire extrachromosomal, et indépendante de la réplication chromosomale, par exemple un plasmide. Alternativement, le vecteur pourra être élaboré de manière à intégrer tout ou partie de l'ADN qu'il contient dans le génome de la cellules hôte, et se répliquera en même temps que le(s) chromosome(s) dans lequel il sera intégré.

Le vecteur est préférentiellement un vecteur d'expression dans lequel la GFP fusionnée à la protéine cible ou la GFP fusionnée au ligand est sous le contrôle d'autres segments d'ADN requis pour la transcription. En général, le vecteur d'expression dérive d'ADN plasmidique ou viral ou peut contenir des éléments de l'un et de l'autre.

Le terme "sous le contrôle" indique que les segments d'ADN sont disposés sur le vecteur de manière à fonctionner de concert pour servir l'objectif voulu, par exemple, la transcription est initiée dans le promoteur et se poursuit tout au long de la séquence codant pour la protéine cible fusionnée à la GFP ou le ligand fusionné à la GFP.

Le promoteur peut être n'importe quelle séquence d'ADN susceptible de promouvoir une activité transcriptionnelle dans la cellule hôte choisie et peut être dérivé de gène homologues ou hétérologues à la cellule hôte.

Des exemples du promoteurs convenant pour l'expression de la protéine cible fusionnée avec le GFP ou du ligand fusionné avec la GFP dans des cellules mammifères sont le promoteur du virus simien SV40 (Subramani *et al.* 1981, Mol Cell. Biol. 1:854-864), le promoteur du virus du sarcome de Rous (RSV), le promoteur du cytomegalovirus (CMV) ou le promoteur tardif majeur de l'adénovirus (AdMLP).

### Exemples de promoteurs pour cellules d'insectes :

Promoteur de la polyhédrine (US 4,745,051; Vasuvedan et *al.* 1992, FEBS Lett. 311:7-11) le promoteur P10 (Vlack *et al.* 1988, J. Gen. Virol. 69:765-776) le promoteur du gène précoce 1 du baculovirus (US 5,155,037; US 5,162,222).

### Exemples de promoteurs pour levures :

Promoteurs des gènes de la glycolyse (Hitzeman *et al.* J. Biol. Chem. 1980, 255:12073-12080; Alber et Kawasaki, J. Mol. Appl. Gen. 1982, 1:419-434) des gènes des alcool deshydrogénases (Young *et al.* dans Genetic Engineering of microorganisms for chemicals (Hollaender et *al.* eds), Plenum Press, NY 1982).

### Exemples de promoteurs pour bactéries:

Des exemples de promoteurs pour l'expression dans la bactérie peuvent être des promoteurs constitutifs comme le promoteur de la polymérase T7, ou des promoteurs inductibles comme par exemple le promoteur pL du phage lambda (Current Protocols in Molecular Biology, op.cit.).

### Exemples de promoteurs pour champignons filamenteux

Les promoteurs utilisables sont par exemple le promoteur ADH3 (McKnight *et al.* EMBO J. 1985, 4:2093-2099) ou le promoteur tpiA. D'autres promoteurs utiles peuvent être dérivés des gènes codant pour l'aspartate protéinase de *Rhizomucor miehei,* l'alpha-amylase neutre de *Aspergillus niger,* de l'acétamidase de *Aspergillus nidulans,* de la TAKA amylase de *Aspergillus oryzae* ou le promoteur de la glucoamylase de *Aspergillus awamori.*

Le vecteur pourra par ailleurs contenir :
- des séquences de polyadénylation, comme par exemple celles de SV40 ou de la région Elb 5 de l'adénovirus,
- des séquences activatrices (enhancer) de la transcription (l'activateur de SV40),
- des séquences de réplication comme par exemple les séquences de réplication de SV40 ou du virus Epstein Barr, pour les cellules mammifères ou l'origine et les gènes de réplication REP 1-3 du plasmide 2 *µ*, pour les levures,
- des marqueurs de sélection, à savoir des gènes conférant une résistance à un antibiotique (néomycine, zéocine, hygromycine, ampicilline, kanamycine, tétracycline, chloramphénicol...) ou permettant la compensation d'un défaut (gène codant pour la dihydrofolate réductase permettant la résistance au méthotrexate, ou gène TPI de S. pombe décrit par Russell, 1985, Gene, 40:125-130).

La cellule hôte peut être n'importe quelle cellule capable d'exprimer la construction d'ADN insérée dans un vecteur approprié.

Les cellules peuvent être notamment des bactéries, des levures, des champignons et des cellules eucaryotes supérieures comme par exemple des cellules mammifères.

Des exemples de cellules bactériennes capables d'exprimer les constructions d'ADN sont :
- des bactéries grampositives telles que les souches de Bacillus comme *B. subtilis, B. licheniformis, B. lentus, B. brevis, B. strearothermophilus, B. thurigiensis* ou des souches de *Streptomyces* comme *S. lividans, S murinus,*
- des bactéries gramnégatives telles *Escherichia coli.*

La transformation des bactéries peut être effectuée par transformation protoplastique ou par transformation de bactéries compétentes (Current Protocols in Molecular Biology, op.cit.).

### Exemples de cellules eucaryotes:

Lignées cellulaires HEK 293, HeLa, cultures primaires, cellules COS (e.g. ATCC CRL 1650), BHK (e.g. ATCC CRL 1632) CHO (e.g. ATCC CCL 61).

Les méthodes d'introduction de l'ADN dans ces cellules (transfection, lipofection, électroporation etc. sont décrites dans Current Protocols in Molecular Biology, op.cit.

Exemples de cellules de levures :
*Saccharomyces, S. cerevisiae, S. kluyveri,*
*Kluiveromyces, K. lactis,*
*Hatisenula, H. polymorpha,*
*Pichia, P. pastoris,*
transformées par introduction d'ADN hétérologue selon les protocoles décrits dans Current Protocols in Molecular Biology, op.cit.

Les cellules transformées sont sélectionnées par un phénotype déterminé par un marqueur de résistance, généralement à une drogue, ou par leur capacité à proliférer en l'absence d'un nutriment particulier.

### Exemples de champignons filamenteux:

Les souches *Aspergillus* (*A. oryzae, A. nidulans, A. niger*), *Neurospora, Fusarium, Trichoderma.* L'utilisation de *Aspergillus* pour l'expression de protéines est décrite dans EP 272 277 ou EP 230 023 ou EP 184 438.

### Exemples de cellules d'insectes :

On peut citer les lignées de *Lepidoptera* e.g. *Spodoptera frugiperda* (Sf9) ou *Trichoplusia ni* (Tni). Les méthodes de transformation (infection en particulier) sont décrites dans Current Protocols in Molecular Biology (op.cit.).

### LES LIGANDS

Les ligands interagissant avec la protéine cible peuvent être de n'importe quelle origine (naturelle, synthétique, semi-synthétique, recombinante), et de n'importe quelle structure (chimique, peptidique, protéique). Ils peuvent être naturellement fluorescents (ou porteurs d'un chromophore) ou peuvent nécessiter soit une réaction chimique permettant le greffage d'un groupe fluorescent (ou d'un précurseur de groupe fluorescent) ou d'un chromophore, soit une construction d'ADN conduisant à la fusion du ligand avec la GFP et permettant l'expression du ligand ainsi rendu fluorescent.

Des exemples de réactions chimiques sont:
- couplage d'amines ou de thiols avec des réactifs de type halogénure d'alkyle, halogénures d'aryles, halogénures d'acyles, halogénures d'acides, le groupe isothiocyanate, le groupe maléimide, les époxydes, dans un solvant organique en présence d'une base ou en milieu aqueux,
- couplage d'acides avec des amines activées par des groupes tels que les succinimides.

Selon le procédé de l'invention, la fluorescence des cellules transformées peut être mesurée dans un spectrofluorimètre à l'aide duquel les propriétés spectrales des cellules, en suspension ou adhérentes, peuvent être déterminées par l'acquisition de leurs spectres d'excitation et d'émission. Les interactions avec le ligand fluorescent sont ensuite détectées par les changements des spectres d'excitation et/ou d'émission du donneur et de l'accepteur d'énergie, et les ligands sont définis comme pharmacologiquement significatifs si leurs interactions avec la protéine cible sont inhibées par l'addition d'un excès de ligand non fluorescent empêchant l'interaction entre la protéine cible fluorescente et le ligand fluorescent.

### DESCRIPTION DES FIGURES :

- La Figure 1 donne la séquence nucléotidique codante de la GFP sauvage (Prasher *et al.* 1992, Gene 111:229-233) de *Aequorea victoria.*

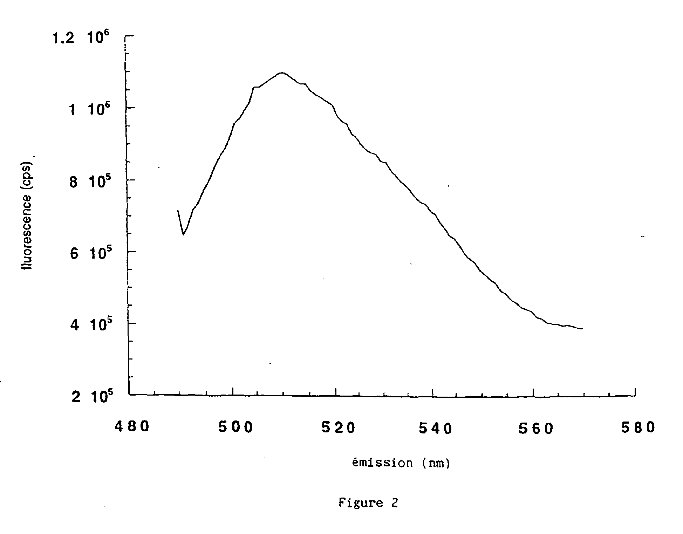

- La Figure 2 représente la fluorescence mesurée lors de l'expression de la construction pMT3-EGFP-C3-SP. En ordonnée, on a présenté la fluorescence exprimée en coups par seconde (cps) et en abscisse la longueur d'onde d'émission. La trace représentée est un spectre d'émission de différence entre la mesure du milieu de culture exprimant la EGFP et le milieu de culture n'exprimant pas la EGFP.

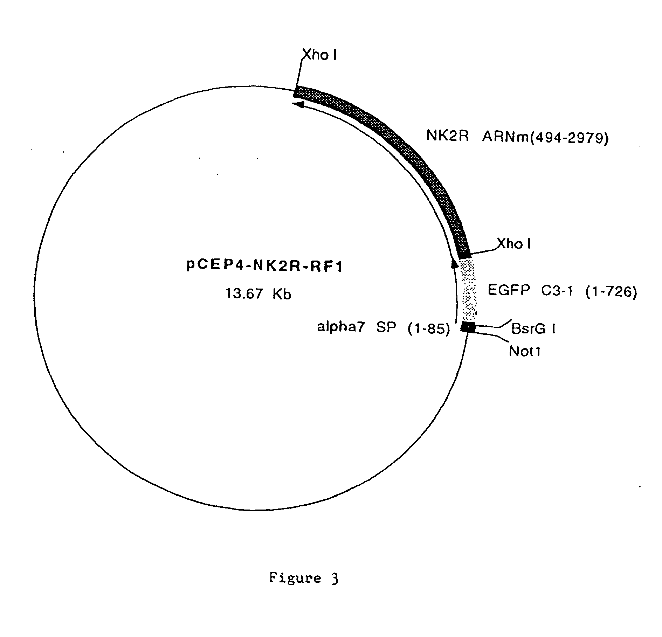

- La Figure 3 représente la construction d'ADN pCEP4-NK2R-RF1 (13,67 kb) et comporte les séquence codant pour le peptide signal du récepteur nicotinique alpha7 (alpha7 SP), la EGFP (EGFP C3-1) et le récepteur NK2R (NK2R ARNm) des tachykinines.

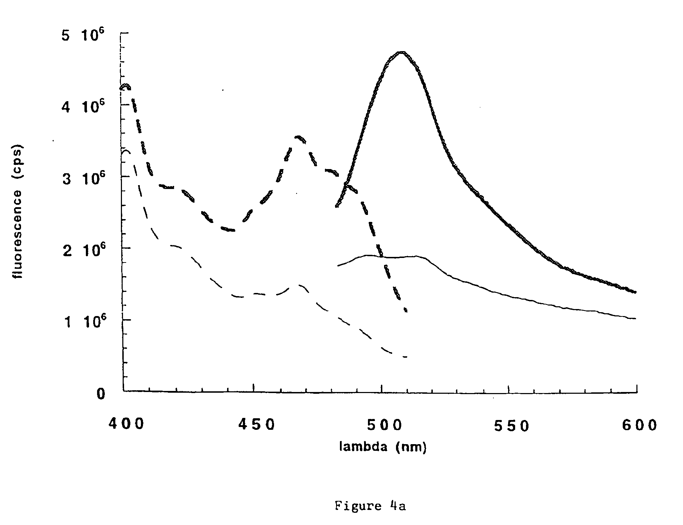

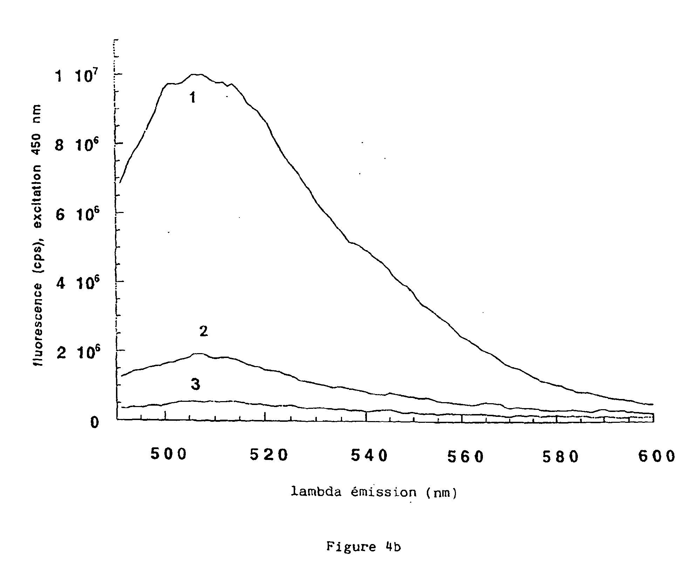

- La Figure 4a représente les spectres d'excitation (émission 540 nm) et d'émission (excitation 450 nm) de cellules HEK293 en suspension exprimant les constructions d'ADN pCEP4-NK2R et pCEP4-NK2R-RF1. En ordonnée, on a présenté la fluorescence exprimée en coups par seconde (cps) et en abscisse la longueur d'onde d'émission.
   Les courbes en pointillés représentent les spectres d'excitation (émission 540 nm) de pCEP4-NK2R (traits fins) et pCEP4-NK2R-RF1 (traits gras).
   Les courbes en trait plein représentent les spectres d'émission (excitation 450 nm) de pCEP4-NK2R (traits fins) et pCEP4-NK2R-RF1 (traits gras).
- La Figure 4b représente le spectre d'émission (excitation 450 nm) de cellules exprimant les récepteurs NK2R-WT (courbe n° 3), NK2R-RF1 (courbe n° 1) et NK2R-RF2 (courbe n° 2). En ordonnée, on a présenté la fluorescence exprimée en coups par seconde (cps) et en abscisse la longueur d'onde d'émission.

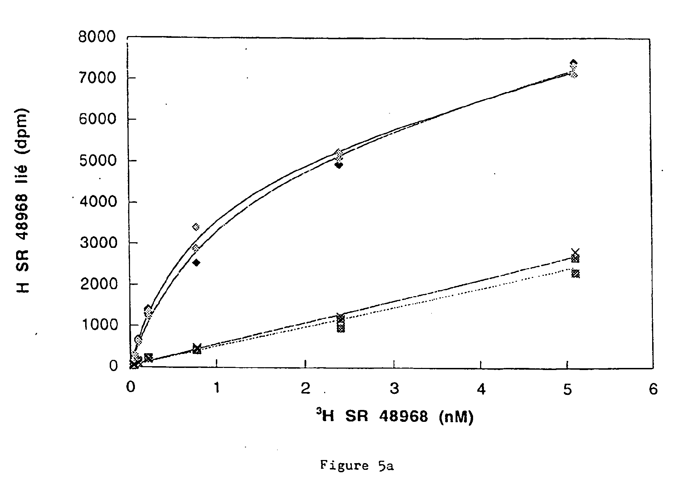

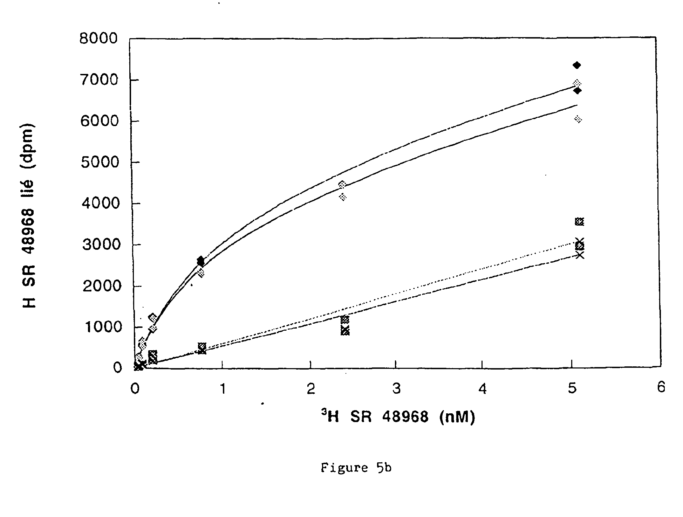

- La Figure 5a représente la liaison de l'antagoniste ³H SR 48968 sur des cellules exprimant le récepteur NK2R sauvage. En ordonnée, on a représenté la quantité de ³H SR 48968 liée (en désintégrations par minute;dpm) et en abscisse, la concentration de ³H SR 48968 ajoutée dans l'échantillon. Les losanges gris et noirs représentent la liaison totale de ³H SR 48968 dans deux expériences différentes, les croix et carrés gris représentent la liaison non spécifique déterminée en présence d'un excès de neurokinine A (10 *µ*M). Les lignes correspondent aux courbes théoriques de liaison d'un ligand à son récepteur ou à des sites de liaison non spécifiques. Les valeurs d'affinité déterminées (KD) sont de 1.05 nM et 0.78 nM dans chacune des deux expériences. Les valeurs de liaison maximale (B max) sont toutes deux de 0.1 pMol/25000 cellules.
- La Figure 5b représente la liaison de l'antagoniste ³H SR 48968 sur des cellules exprimant le récepteur fluorescent NK2R-RF1. En ordonnée, on a représenté la quantité de ³H SR 48968 liée (en désintégrations par minute;dpm) et en abscisse, la concentration de ³H SR 48968 ajoutée dans l'échantillon. Les losanges gris et noirs représentent la liaison totale de ³H SR 48968 dans deux expériences différentes, les croix et carrés gris représentent la liaison non spécifique déterminée en présence d'un excès de neurokinine A (10 *µ*M). Les lignes correspondent aux courbes théoriques de liaison d'un ligand à son récepteur ou à des sites de liaison non spécifiques. Les valeurs d'affinité déterminées (KD) sont de 0.8 nM. et 0.92 nM dans chacune des deux expériences. Les valeurs de liaison maximale (B max) sont respectivement de 0.075 et 0.088 pMol/25000 cellules.

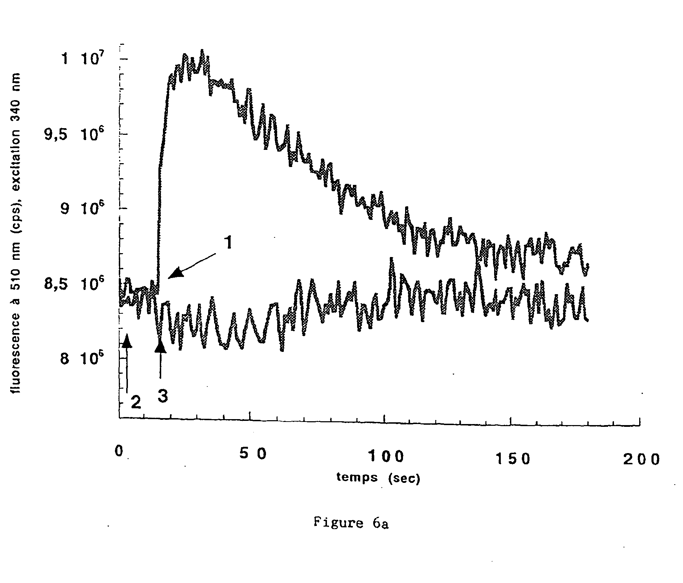

- La Figure 6a représente le test de fonctionalité de réponse de libération de calcium intracellulaire (FURA 2) de cellules exprimant la construction d'ADN pCEP4-NK2R WT. En ordonnée, on a représenté la fluorescence à 510 nm (exprimée en coups par seconde), l'excitation ayant lieu à 340 nm et en abscisse, on a représenté le temps (seconde). Les réponses sont évoquées par l'agoniste neurokinine A (NKA) et inhibées par l'antagoniste cyclo(-Gln-Trp-Phe-Gly-Leu-Met) (cyclopeptide). Dans l'expérience 1, on ajoute 10 nM de NKA. Dans l'expérience (2,3), on ajoute successivement 5 *µ*M de cyclopeptide (2), puis 10 nM de NKA (3).

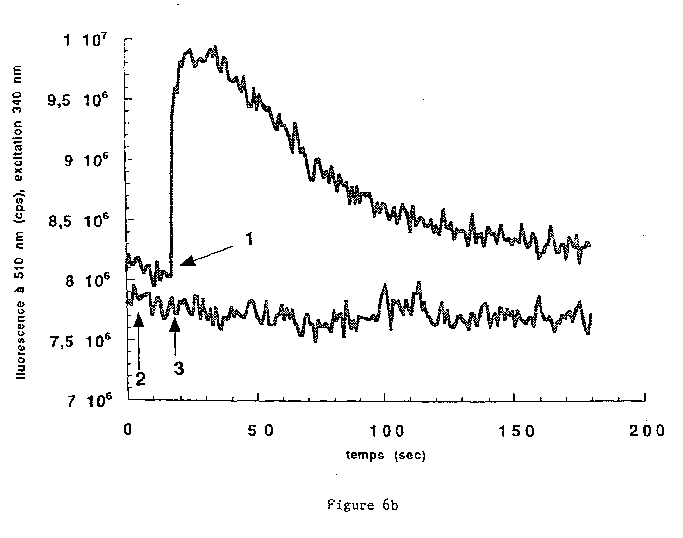

- La Figure 6b représente le test de fonctionnalité de réponse de libération de calcium intracellulaire (FURA 2) de cellules exprimant la construction d'ADN pCEP4-NK2R-RF1. En ordonnée, on a représenté la fluorescence à 510 nm (exprimée en coups par seconde), l'excitation ayant lieu à 340 nm et en abscisse on a représenté le temps (seconde). Les réponses sont évoquées par l'agoniste neurokinine A (NKA) et inhibées par l'antagoniste cyclo(-Gln-Trp-Phe-Gly-Leu-Met) (cyclopeptide). Dans l'expérience 1, on ajoute 10 nM de NKA. Dans l'expérience (2,3), on ajoute successivement 5 *µ*M de cyclopeptide (2), puis 10 nM de NKA (3).

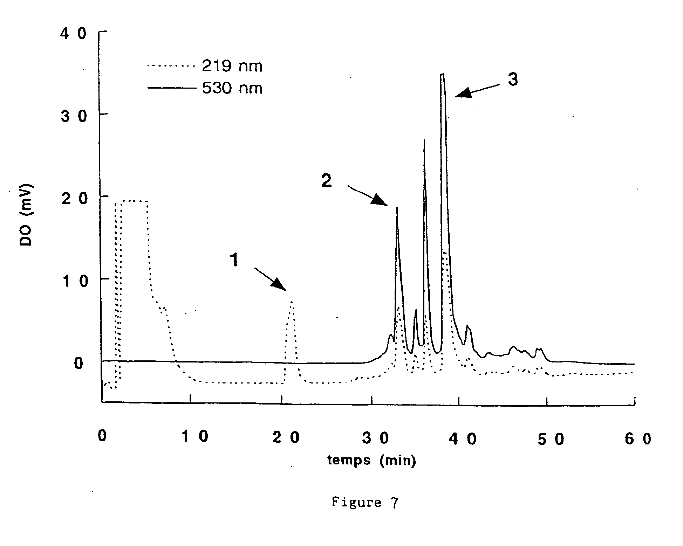

- La Figure 7 représente la purification du peptide NKA BO I par HPLC en phase inverse. En abscisse, on a représenté le temps (minute) et en ordonnée la densité optique (mV). La détection est effectuée à 2 longueurs d'onde : 219 nm (traits pointillés) et 530 nm (traits pleins).
   Les pics identiques 1, 2 et 3 sont respectivement la NKA, le dérivé NKA-BODIPY, le réactif BODIPY-IA.

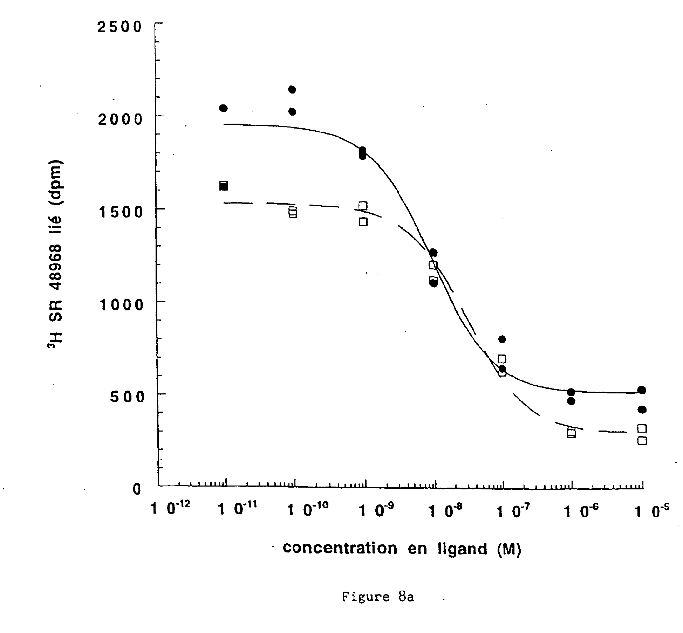

- La Figure 8a représente le déplacement de la liaison de ³H SR48968 sur le récepteur NK2R-WT par NKA et NKA BO 1. En ordonnée, on a représenté la radioactivité (exprimée en désintégrations par minute;dpm) et en abscisse la concentration en ligand (M).
   Les cercles noirs représentent l'expérience de déplacement de ³H SR48968 par la NKA-BO I (KI = 16,15 nM).
   Les carrés blancs représentent l'expérience de déplacement de ³H SR48968 par la NKA (KI = 3,03 nM).

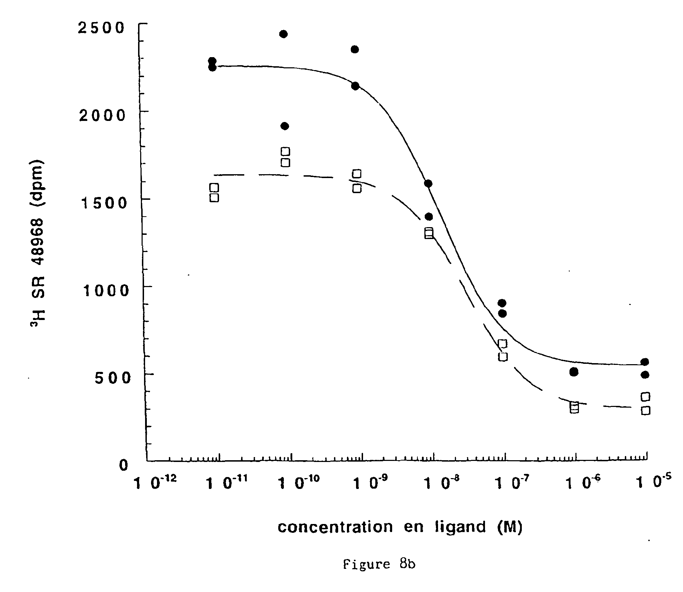

- La Figure 8b représente le déplacement de la liaison de ³H SR48968 sur le récepteur NK2R-RF1 par NKA (ronds noirs : (KI = 2,1 nM) et NKA-BO I (carrés blancs : (KI = 16,08 nM). En ordonnée, on a représenté la radioactivité (exprimée en désintégrations par minute;dpm) et en abscisse la concentration en ligand.

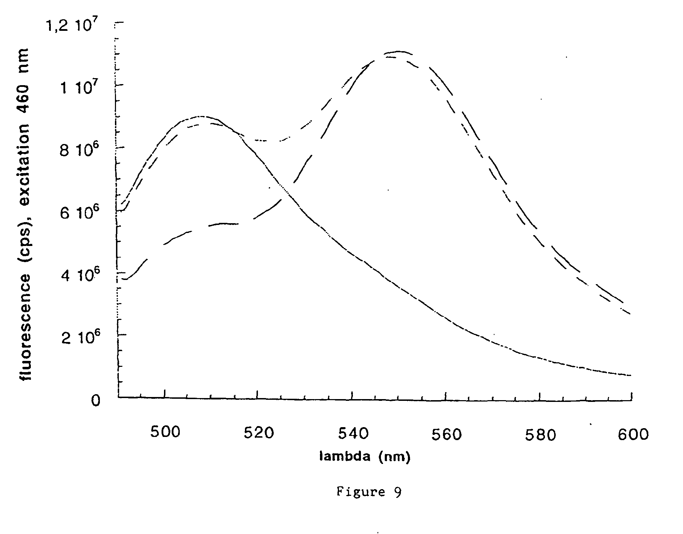

- La Figure 9 représente l'expérience de transfert d'énergie entre le ligand fluorescent NKA-BO I et le récepteur fluorescent NK2R-RF1 déterminée à l'équilibre.
   La courbe en trait plein représente l'émission de fluorescence (entre 490 et 600 nm) des cellules exprimant le récepteur fluorescent NK2R-RF1 (excitation 460 nm) (Etape 1).
   La courbe en grands traits pointillés représente le même spectre d'émission de fluorescence après addition de 100 nM de NKA BO I (Etape 2).
   La courbe en traits pointillés représente le spectre d'émission de fluorescence après addition, sur l'étape 2, de 10 *µ*M de NKA non fluorescente. Les mêmes résultats sont obtenus après addition de 10 *µ*M de cyclo(-Gln-Trp-Phe-Gly-Leu-Met) ou de SR 48968.
   L'addition, en étape 2, de NKA non fluorescente au lieu de NKA BO I est sans effet sur le spectre d'émission de fluorescence. Celui-ci se superpose avec le spectre en trait plein.
   En ordonnée, on a représenté la fluorescence en cps, en abscisse la longueur d'onde en nm.

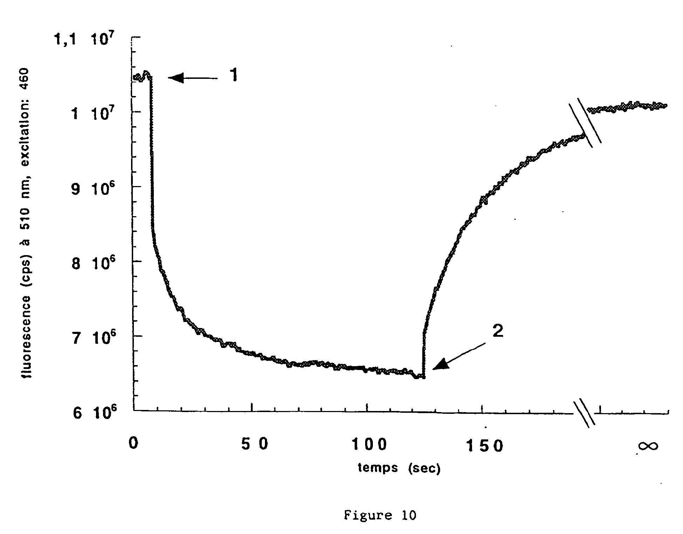

- La Figure 10 représente la mesure en temps réel du transfert d'énergie entre 100 nM de NKA BO 1 et le récepteur NK2R-RF1 (1), et inhibition (2) par un excès de ligand non fluorescent (20 *µ*M de NKA ou de MEN 10,376 ou de cyclopeptide). En abscisse, on a représenté le temps en seconde et en ordonnée la fluorescence (coups par seconde) à 510 nm, l'excitation ayant lieu à 460 nm.
   La mesure est effectuée avec une suspension de 10⁶ cellules/ml.

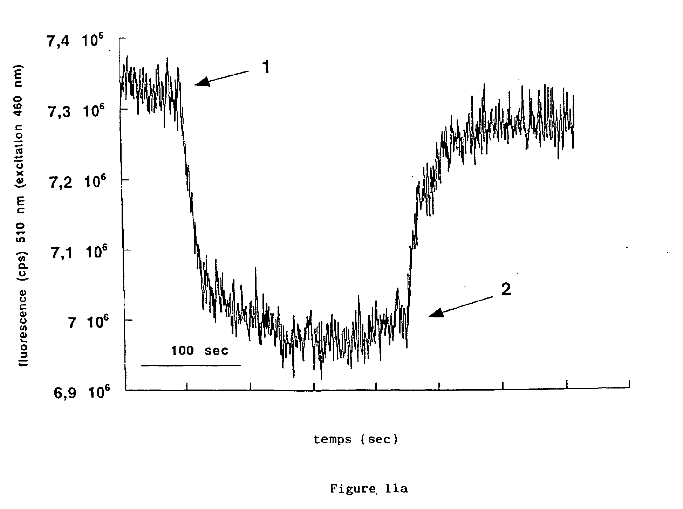

- La Figure 11a représente la mesure en temps réel du transfert d'énergie entre 100 nM de NKA-TR et le récepteur NK2R-RF1 (1), et inhibition par un excès (2) de ligand non fluorescent (100 µM de NKA ou de MEN 10,376 ou de cyclopeptide). En abscisse, on a représenté le temps en seconde et en ordonnée la fluorescence (coups par seconde) à 510 nm, l'excitation ayant lieu à 460 nm.

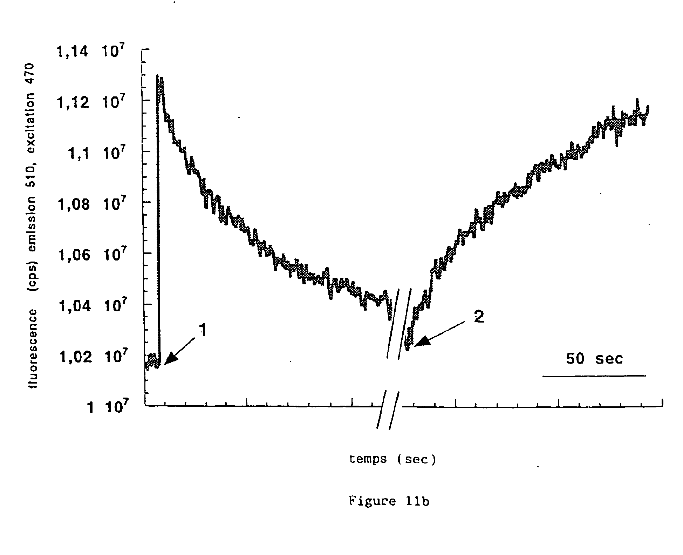

- La Figure 11b représente la mesure en temps réel du transfert d'énergie entre 100 nM de NKA-Eos et le récepteur NK2R-RF1 (1), et inhibition par un excès (2) de ligand non fluorescent (20 *µ*M de NKA ou de MEN 10,376 ou de cyclopeptide). En abscisse, on a représenté le temps en seconde et en ordonnée la fluorescence (coups par seconde) à 510 nm, l'excitation ayant lieu à 470 nm.

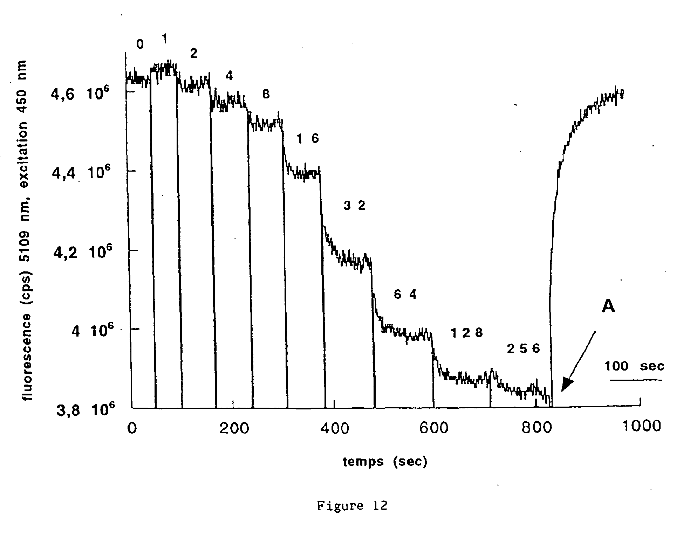

- La Figure 12 représente l'amplitude du signal de fluorescence en fonction de la concentration de NKA BO I ajoutée (0 à 256 nM) à une suspension de cellules (10⁶ cellules/ml) exprimant le récepteur NK2R-RF1 et l'inhibition par un excès (A) de ligand non fluorescent (10 *µ*M de NK ou de MEN 10,376 ou de cyclopeptide). En abscisse, on a représenté le temps en seconde et en ordonnée la fluorescence (coups par seconde) à 510 nm, l'excitation ayant lieu à 450 nm.

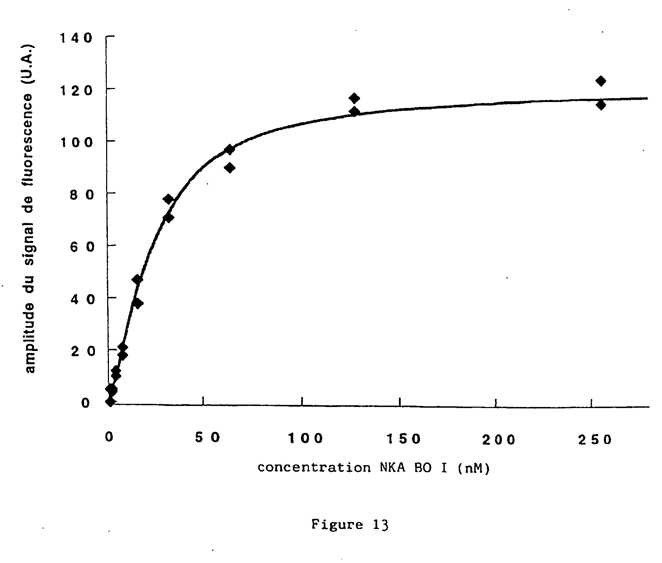

- La Figure 13 représente le traitement des données de la Figure 12. En abscisse, on a représenté la concentration en NKA BO I (nM) et en ordonnée l'amplitude du signal de fluorescence exprimée en unités arbitraires (U.A.). La ligne en trait plein représente la courbe théorique de liaison de la NKA-BO I à ses sites et fournit une valeur d'affinité KD = 24 nM.

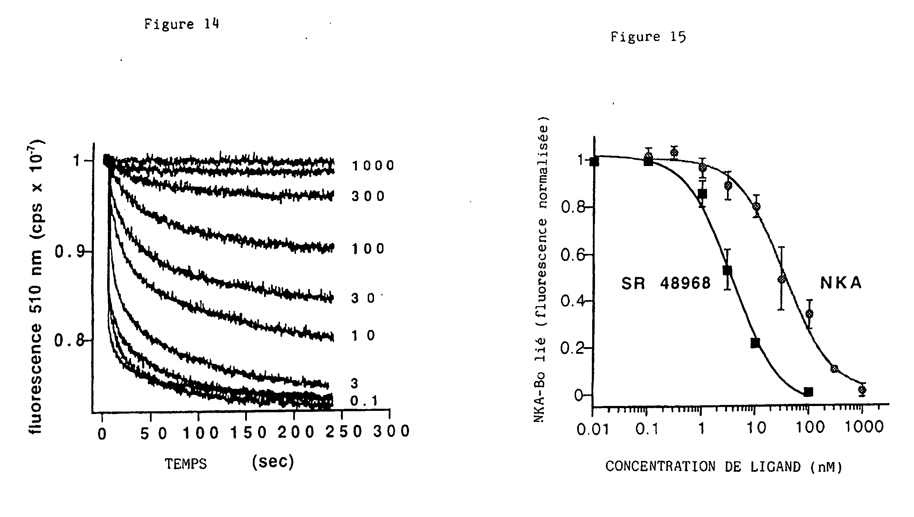

- La Figure 14 représente l'extinction de fluorescence mesurée à 510 nm (excitation à 460 nm) résultant de la liaison de 10 nM NKA-Bo sur le récepteur EGFP-NK2R exprimé à la surface de cellules HEK 293 (suspension à 10° cellules/ml) en absence, et en présence de concentrations croissantes (indiquées sur le graphe en nM) de neurokinine A (NKA). En ordonnée, on représente la fluorescence mesurée en coups par seconde (cps) et en abscisse, le temps en , seconde.
- La Figure 15 correspond à l'expérience décrite dans la figure 14, effectuée en présence de neurokinine A (NKA) ou de SR48968. L'amplitude de fluorescence, normalisée par rapport à l'amplitude de fluorescence mesurée en absence de NKA ou SR48968, est reportée sur un graphe en fonction de la concentration de NKA ou SR48968 utilisée pour tracer une courbe de compétition pour la liaison de NKA-Bo contre la NKA ou le SR48968 sur le récepteur EGFP-NK2R. L'affinité du ligand non fluorescent est déterminée à la valeur d'inhibition de 50 % de la liaison de NKA-Bo qui correspond à l'IC₅₀ du composé non fluorescent.

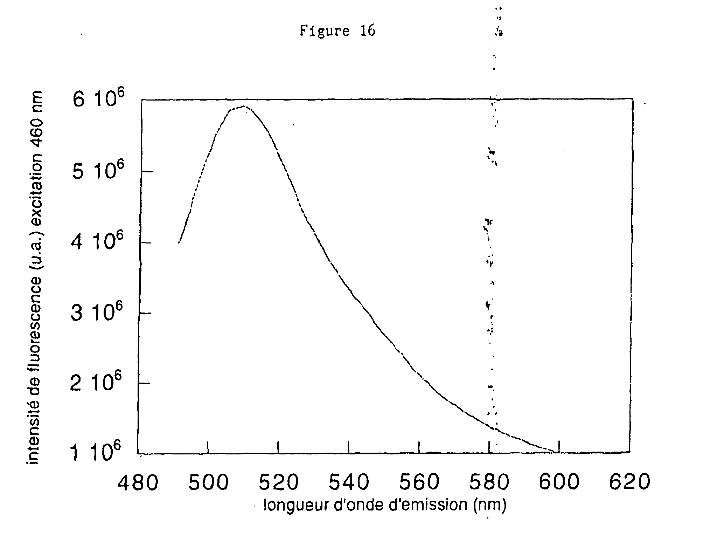

- La Figure 16 représente le spectre d'émission de cellules (à 10⁶ cellules/ml) exprimant la protéine de fusion EGFP-Hind-CCR5. L'excitation est fixée à 460 nm. En ordonnées, on représente la fluorescence exprimée en coups par seconde (cps) et en abscisse, la longueur d'onde d'émission en nm.

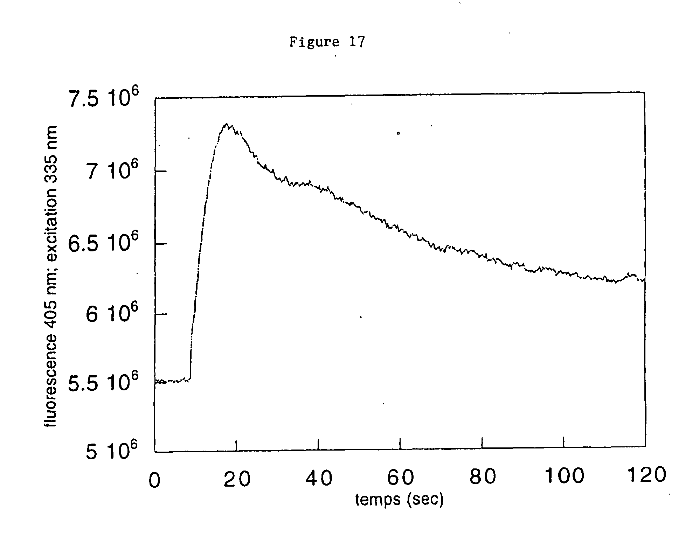

- La Figure 17 représente un test de fonctionnalité de réponse de libération de calcium intracellulaire (INDO-1) de cellules exprimant la construction d'AND EGFP-Hind-CCR5. En ordonnée, on a représenté la fluorescence à 405 nm exprimée en coups par seconde, l'excitation ayant lieu à 335 nm, et en abscisse, on a représenté le temps en seconde. La réponse évoquée par 100 nM de la chimiokine RANTES est montrée.

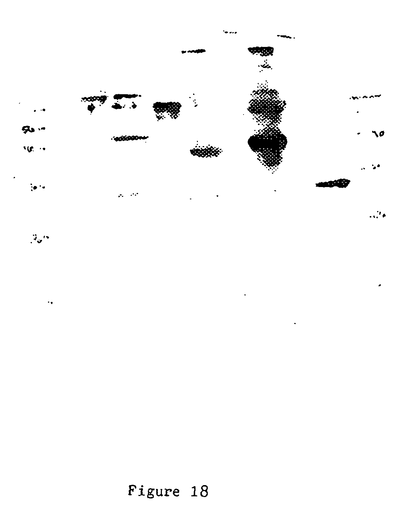

- La Figure 18 représente la révélation de l'expression de la chimiokine fluorescente EGFP-RANTES par immunoblot à l'aide d'un anticorps anti-GFP. Les levures, ou leurs surnageants de culture, exprimant une protéine non fluorescente (le trypsinogène) ou la construction d'ADN codant pour EGFP-RANTES sont déposés sur gel dénaturant de polyacrylamide 12 %. Après électrophorèse, les protéines sont électrotransférées sur membrane de nitrocellulose et la présence de protéines comprenant la GFP est révélée par addition d'anticorps primaire anti-GFP de lapin puis d'anticorps secondaire anti-lapin couplé à la luciférase. Les bandes protéiques contenant la GFP sont révélées par chimioluminescence à l'aide du Kit ECL disponible chez Amersham.

La figure révèle la présence de EGFP-RANTES dans les pistes 3 et 6 (respectivement surnageant de culture de levures, et levures) exprimant la construction d'ADN-EGFP-RANTES (bande de 42 000 daltons). Les pistes 1 et 8 correspondent à la migration de marqueurs de poids moléculaires annotés 20, 30, 40 et 50 kilodaltons. La piste 2 correspond au surnageant de levures contrôle (trypsinogène), la piste 4 correspond au précipité de dialyse de EGFP-RANTES, la piste 5 correspond à la levure contrôle et la piste 7 correspond à la levure exprimant EGFP seule.

### EXEMPLES

### EXEMPLE 1 : CONSTRUCTIONS D'ADN COMPRENANT UNE FUSION ENTRE LA EGFP ET L'EXTREMITE AMINO TERMINALE DU RECEPTEUR NK2R DES TACHYKININES

I) Fusion de la EGFP avec un peptide signal :
   L'ADNc codant pour le EGFP (Figure 1) est fusionné en phase avec la séquence codant pour le peptide signal de la sous-unité alpha 7 de poulet (Genbank accession N°: X522995) codant pour un récepteur nicotinique de l'acétylcholine comme suit:
   On introduit sur les codons codant 1 à 9 de la EGFP un site de restriction pour l'endonucléase BsrG I à l'aide de l'oligonucléotide 5'GGTCGCCACCCTGTACAAGAAGGGCGAGG3', des réactifs fournis dans le kit de mutagenèse RPN 1526 (Sculptor) fourni par la société Amersham, et de simple brin de pEGFP C3 préparé à partir du plasmide pEFGP C3 (Genbank Accession N° U57607) fourni par la société ClonTech. le mutant pEGFP C3-1 obtenu est séquencé, puis cloné en phase avec le peptide signal de alpha7 par ligation de deux fragments: le fragment BsrGI-Xho I de 5225 nt de pJL223 (Eiselé *et al.* 1993, Nature 366:479-483) et le fragment BsrG I - Xho I de 725 nt de pEGFP C3-1. Le plasmide pJL223 contient le gène de la protéine a7-V201-5HT3 entre les sites Not I et Xho I du vecteur pMT3 (Swick, A.G. *et al.* 1992, Proc. Natl. Acad. Sci. 89:1812-1816). la construction obtenue nommée pMT3-EGFP-C3-SP, est transitoirement exprimée dans des cellules HEK 293 (ATCC CRL 1573) après transfection au phosphate de calcium (Cheng et Okayama 1986), afin de vérifier que la construction est correcte. Le spectre d'émission de fluorescence (excitation 450 nm) des surnageants de culture de cellules exprimant pMT3-EGFP C3-SP ou de cellules non transfectées (concentrés 5 fois par centrifugation sur centrikon 10 (Amicon)) sont enregistrés. Sur la figure 2 qui montre la différence entre le spectre de cellules transfectées et non transfectées, on détecte nettement le pic d'émission de la EGFP, ce qui indique que la construction conduit effectivement à l'expression de EGFP sécrétée dans le milieu de culture.
II) Clonage du récepteur NK2R des tachykinines dans les vecteurs de mutagenèse KS et d'expression pCEP4 :
   Le fragment Spe I - Hind III, 2997 nt, du plasmide prTKR1-1 (Pr. S. Nakanishi, Kyoto university, Japon, Biochem. Biophys. Res. Comm. 1989, 165:695-702), contenant l'ADNc codant pour le récepteur NK2R (Genbank accession N°: M31838) de rat, est ligué avec le fragment Spe I - Hind III, 3549 nt, du vecteur pBluescript KS (+) pour conduire au plasmide pKS NK₂R.
   Le fragment Not I - BsrG I, 1369 nt, du plasmide prTKR1-1 est ligué avec le fragment BsrG I - Xho I, 1663 nt, de pKS NK2R et le fragment Not I - Xhol, 10370 nt de pCEP4 pour conduire au plasmide pCEP4-NK₂R.
III) Fusion de la EGFP entre le peptide signal de alpha7 et l'extrémité amino terminal du récepteur NK2R des tachykinines :
   Le fragment Not I - Xho I, 816 nt, de pMT3-EGFP C3-SP est ligué avec le fragment Not I Xho I (digestion partielle Xho I), 12856 nt, de pCEP4-NK₂R pour conduire à la construction pCEP4-NK2R-RF1 (figure 3).

### EXEMPLE 2 : CONSTRUCTION D'ADN COMPRENANT LA FUSION DE LA GFP DANS LES BOUCLES INTRACELLULAIRES I1 ET I3 DU RECEPTEUR NK2R DES TACHYKININES

I) Introduction de sites de clonage de la EGFP dans la boucle il ou i3 du récepteur NK2R :
   L'ADN simple brin de pKS NK2R est mutagénisé comme en 1 a) avec les oligonucléotides contenant les mutations pour les sites de clonage BsrG I et Xho I, permettant l'introduction de la EGFP entre les acides aminés 65 et 66 et contenant les mutations pour les sites de clonage Nhe I, Bgl II et Hind III permettant l'introduction de la EGFP entre les résidus 233 et 234 ou 233 et 238.
   Les plasmides obtenus pKS NK2R-i1 et -i3 .sont introduits dans des bactéries XL1Blue compétentes (transformation), et les échantillons d'ADN plasmidique isolés à partir des colonies résistantes à 1.1 ampicilline sont criblés pour la présence des sites introduits respectivement par les mutations à l'aide des oligonucléotides ilet i3.
II) Clonage de la EGFP dans les boucles i1 et i3 du récepteur NK2R des tachykinines :
   - clonage dans la boucle il: les fragments Hind III - BsrG I, et Hind III - Xho I, de pKS NK2R-il sont ligués avec le fragment BsrG I - Xho I, 725 nt, de pEGFP C3-1. L'insert de 3741 nt codant pour la protéine de fusion est ensuite excisé par les enzymes Spe I et Sal I et ligué avec le vecteur pCEP4 ouvert par les enzymes Nhe 1 et Xho I pour conduire à la construction d'ADN pCEP4 NK2R-RF2 ;
   - clonage dans la boucle i3: deux constructions sont obtenues:
      les fragments Nhe I - Bgl II, 744 nt, de pEGFP C3 sont ligués avec les fragments Not I - Nhe I et Bgl II - Not I de pKS-NK2R-i3, l'insert Spe I - Sal I de 3750 nt ainsi obtenu est ensuite cloné dans le vecteur pCEP4 entre les sites Nhe I et Xho I pour conduire à la construction d'ADN pCEP4-NK2R-RF3.
      les fragments Nhe 1 - Hind III, 757 nt, de pEGFP C3 sont ligués avec les fragments Not I - Nhe I et Hind III - Not I de pKS-NK2Ri3, l'insert Spe I - Sal I de 3770 nt ainsi obtenu est ensuite cloné dans le vecteur pCEP4 entre les sites Nhe 1 et Xho I pour conduire à la construction d'ADN pCEP4-NK2R-RF4.

### EXEMPLE 3 : EXPRESSION DES PROTEINES RECOMBINANTES ET CARACTERISATION FONCTIONNELLE

I) Expression :
   Des cellules HEK 293 sont transfectées, par la méthode de la précipitation au phosphate de calcium (Chen & Okayama 1987, Mol. Cell. Biol. 7:2745-2752, Current Protocols in Molecular Biology, op.cit.), par les constructions d'ADN pCEP4-NK2R-RF1, -RF2, -RF3 et -RF4. Des lignées stables sont établies par sélection des cellules transfectées résistantes à l'hygromycine (100 *µ*g/ml, Clontech). Les cellules sont cultivées en présence d'hygromycine 100 *µ*g/ml dans du milieu MEM (Gibco) supplémenté de 10% de sérum de veau foetal (Seromed) de Pénicilline (100 unités/ml), streptomycine (100 *µ*g/ml) et glutamine (4 mM) (Methods in enzymology, Vol LVIII, 1979).
II) Mesures de l'expression et propriétés de liaison des récepteurs recombinants :
   a) Par fluorimétrie :
      Les expériences de fluorescence sont effectuées dans une cuve de 1 ml pourvue d'un système d'agitation magnétique et placée dans un spectrofluorimètre Fluorolog (SPEX) équipé d'une lampe Xe 450W (Osram) et de monochromateurs Spex 1680 0.22m (excitation) et Spex 1681 0.22 m (émission). Les cellules ou les fragments de membrane sont mis en suspension dans du tampon physiologique: Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (w/v), pH 7,4.
      Les cellules entières sont récoltées après traitement au Versène (PBS, 5 mM EDTA), centrifugées 5 min. à 1.000 g et sont resuspendues à une concentration de 250.000-1.000.000 cellules/ml dans le tampon physiologique.
      La figure 4a montre les spectres d'excitation et émission enregistrés avec des cellules transfectées par les constructions pCEP4-NK2R-WT et pCEP4-NK2R-RF1. Les spectres font clairement apparaître le signal de la EGFP et indiquent que ces constructions sont effectivement exprimées par les cellules HEK 293. La figure 4b montre les spectres émission de fluorescence de cellules exprimant le récepteur sauvage ainsi que les fusions -RF1 et RF2.
   b) Par expérience de liaison :
      L'apparition de sites de liaison des neurokinines à la surface des cellules est déterminée par liaison de 0.1 nM ¹²⁵I NKA ou 1 nM ³H SR48968 dans du PBS sur des cellules entières en suspension (200-500.000 Cellules/ml, 250 *µ*l/essai). Après 30 min. d'incubation à température ambiante, les échantillons sont filtrés sur filtres GF/C (Whatmann) prétraités dans 1 % de lait en poudre dans du PBS (KCl 2.7 mM, KH2PO4 1.47 mM, Nacl 0.137 mM, Na₂HPO₄ 8,06 mM), rincés deux fois au PBS (4 ml), puis comptés dans un cocktail scintillant.
      Les courbes de saturation de la liaison de 3HSR 48 968 sont effectuées dans du tampon Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (w/v), pH 7,4 à 4°C pendant 1 h 30 en présence ou en absence de NKA 1 µM final. Chaque échantillon de 500 µl contient 25 000 cellules, et la fraction de ligand lié est inférieure à 10% de la quantité ajoutée dans chaque tube. La filtration est effectuée en fin d'incubation comme décrit plus haut. les figures 5a et 5b montrent les courbes de liaison (en dpm liés) obtenues avec des concentrations croissantes du ligand 3HSR 48968 sur respectivement des cellules exprimant le récepteur sauvage (NK2R WT) ou la fusion N terminale avec la EGFP (NK2R-RF1). Le ligand exhibe la même affinité pour les deux récepteurs et le nombre de sites de liaisons exprimés à la surface des cellules est comparable.
III) Tests fonctionnels :
   I) Mesure du calcium cytosolique à l'aide du FURA 2 :
      Le chelateur fluorescent du calcium, FURA 2 acétylmethylester (Molecular probes), dissout dans du DMSO est dilué à la concentration de 3 *µ*M dans du tampon Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1 % (w/v), pH 7,4. Le milieu de culture des cellules est aspiré puis remplacé par 7 à 10 ml de la solution de FURA 2 AM. Les cellules sont placées dans l'incubateur à CO₂ pendant 45 min. Le milieu est alors aspiré, remplacé par le tampon Hepes sans FURA et les cellules sont replacées dans l'incubateur à CO₂ pendant 15 min. supplémentaires. Le tampon est aspiré, puis les cellules sont récoltées après avoir été décrochées de la boite par une solution de PBS, 5 mM EDTA pendant 5 min. Après centrifugation à 1000 x g pendant 5 min., le culot de cellules obtenu est resuspendu à raison de 1 x 10⁶ cellules/ml dans le tampon Hepes.
      Les mesures de libération de calcium sont effectuées à 37°C, dans une cuve de spectrofluorimètre contenant par un barreau magnétique d'agitation. La longueur d'onde d'excitation est fixée à 340 nm et la longueur d'onde d'émission est fixée à 510 nm.
      La figure 6, montre les résultats obtenus lors de la stimulation de cellules transfectées avec la construction codant pour le récepteur sauvage (6a) et de cellules transfectées par la construction pCEP4-NK2R-RF1 (6b). Les réponses évoquées par la neurokinine A sont inhibées par les antagonistes NK2R spécifiques SR48968 (Sanofi recherche), MEN 10,376 (Bachem) ou le cyclopeptide cyclo(-Gln-Trp-Phe-Gly-Leu-Met) (Bachem). La neurokinine A n'évoque aucune réponse sur des cellules HEK 293 non transfectées.

### Préparation de fragments de membranes :

Les fragments de membrane sont préparés par homogénéisation des cellules à l'aide d'un homogénéiseur de tissu (potter ou ultraturax) en présence d'un mélange d'inhibiteurs de protéases, à 4°C. Les cellules sont centrifugées à 1000 x G pendant 5 min. puis resuspendues dans du tampon de préparation de membranes: Tris-HCl 50 mM, EDTA-Na 1 mM, DTT 10 mM, contenant le cocktail d'inhibiteurs de protéases, Complete (Boehringer). La suspension est homogénéisée à l'aide d'un potter à 4°C. Après centrifugation à 3000 x G pendant 10 min., le surnageant est collecté, le culot est repris dans le tampon ci-dessus, rehomogénéisé, et centrifugé à nouveau. Les deux surnageants obtenus sont rassemblés et centrifugés à 150 000 x g pendant 30 min. Les culots obtenus sont resuspendus dans du tampon de préparation de membranes à une concentration de 6-10 mg prot/ml.

### EXEMPLE 4 : PREPARATION DE LIGANDS FLUORESCENTS

I) Préparation de neurokinine A fluorescente :
   a) Groupe Bodipy 530/560 :
      La NKA lyophilisée est dissoute dans de la DMF à 10 mM final.
      A 50 *µ*L de cette solution (0.5 *µ*Mol, 50 *µ*L), on ajoute 10 *µ*mol de NEt3 (200 mM dans CH3CN), soit 100 *µ*L, puis 15 *µ*L de BODIPY 530/550 IA [N-(4,4-difluoro-5,7-dimethyl-4-bora-3a,4a-diaza-s-indacene-3-propionyl)-N'-iodoacetylethylenediamine] dissout dans de la DMF à raison de 0.3 *µ*Mol par 10 *µ*L. Le mélange est vortexé, puis laissé à température ambiante. Après 24 h, le produit de la réaction est purifié par HPLC (Gilson) sur une colonne de phase inverse Z5C8 25F (Zorbax) sur laquelle on développe un gradient linéaire de 10 à 95 % solvant B en 60 minutes (A: H₂O 0.1 % TFA; B: CH₃CN 0.1 % TFA) avec un débit de 1 ml/min. Les longueurs d'ondes de détection sont fixées à 219 nm (peptide) et 530 nm (fluorophore). Le produit élué (NKA BO I) au temps 34 min. (Figure 7) est collecté, concentré par évaporation et resuspendu dans de la DMF.
   b) Groupes coumarine et éosine :
      Les conditions réactionnelles employées pour la marquage de la neurokinine A par les dérivés de coumarine (7-diéthylamino-3-((4'-(iodoacétyl)amino)phényl)-4-méthylcoumarine) et d'éosine (éosine-5-iodoacétamide) sont les mêmes que en a). Les purifications sont effectuées sur colonne de phase inverse Z5C8 25F (Zorbax) sur laquelle on développe un gradient linéaire de 10 à 95 % solvant B en 60 minutes (A: H₂O 0.1 % TFA; B: CH3CN 0.1 % TFA) avec un débit de 1 ml/min. le dérivé coumarine de la NKA (NKA-Coum) est élué au temps 25 min., le dérivé éosine de la NKA (NKA-Eos) est élué au temps 27 min.
   c) Groupe sulforhodamine 101 :
      Le réactif utilisé est le chlorure d'acide de la sulforhodamine 101 (Aldrich, ou Texas Red commercialisé par Molecular Probes). Le protocole est le même que pour le greffage du BODIPY 530/550 IA. La purification est effectuée par HPLC sur une colonne de phase inverse Z5C8 25F (Zorbax) sur laquelle on développe un gradient linéaire de 10 à 95 % solvant B en 60 minutes (A: H₂O 0.1% HFBA; B: CH₃CN 0.1% HFBA) avec un débit de 1 ml/min. Les longueurs d'ondes de détection sont fixées à 219 nm (peptide) et 590 nm (fluorophore). le produit élué (NKA TR) au temps 40-41 min. est collecté, concentré par évaporation et resuspendu dans de la DMF.

### EXEMPLE 5 : DETECTION DE L'INTERACTION ENTRE LE RECEPTEUR NK2R FLUORESCENT ET SES LIGANDS FLUORESCENTS.

I) Par déplacement d'un radioligand (expérience de compétition) :
   Les courbes de déplacement de la liaison de 3HSR 48968 par un ligand non radioactif sont effectuées dans du tampon Hepes 10 mM, NaCl 137,5 mM, MgCl₂ 1,25 mM, CaCl₂ 1,25 mM, KCI 6 mM, glucose 5,6 mM, NaH₂PO₄ 0,4 mM, BSA 0,1% (w/v), pH 7,4 à 4°C pendant 1 h 30 en présence ou en absence de NKA 1 *µ*M final. Chaque échantillon de 500 µl contient 25 000 cellules, 1 nM de 3HSR 48968 et un ligand non radioactif à différentes concentrations (de 1 nM à 10 *µ*M). L'incubation est effectuée pendant 1 heure à 4°C. La filtration est effectuée en fin d'incubation comme décrit en 3b).
   La figure 8 montre les courbes de déplacement du radioligand 3HSR 48968 par la neurokinine A (NKA) et la NKA BO I sur respectivement des cellules HEK 293 exprimant le récepteur NK2R sauvage (construction pCEP4-NK2R, figure 8A) ou la fusion de la EGFP en N-terminal du récepteur NK2R (construction pCEP4-NK2R-RF1, figure 8b). Les points représentent la radioactivité liée (en dpm) en présence de concentrations variables de NKA ou de NKA BO I. Les valeurs d'affinité dérivées de ces courbes indiquent que la NKA (tout comme la NKA BO I) se lie avec la même affinité au récepteur sauvage et au récepteur rendu fluorescent. Les valeurs d'affinité sont pour la NKA: KI= 3 nM sur le récepteur sauvage et 2,1 nM sur le récepteur fluorescent et pour la NKA BO I: KI= 16 nM sur le récepteur sauvage et 16 nM sur le récepteur fluorescent. Les même mesures effectuées avec l'antagoniste MEN 10,376 conduisent aux valeurs d'affinité: KI= 53 nM sur le sauvage et KI = 61nM sur le récepteur fluorescent.
II) Par transfert d'énergie :
   a) interactions entre la NKA BO I et le récepteur NK2R-RF1 à l'équilibre:
      Les cellules HEK293 exprimant le récepteur NK2R-RF1 sont placées en suspension à 1 000 000 cellules/ml dans le tampon physiologique Hepes (Hepes 10 mM, NaCl 137,5 mM, MgCl₂ 1,25 mM, CaCl₂ 1,25 mM, KCl 6 mM, glucose 5,6 mM, NaH₂PO₄ 0,4 mM, BSA 0,1% (w/v), pH 7,4). Les cellules sont ensuite placées dans une cuve de fluorescence à une concentration pouvant varier de 100 000 à 1 000 000 cellules/ml. Un spectre d'émission de ces cellules est enregistré entre 490 et 600 nm (excitation 460 nm) puis la NKA BO I est ajoutée à une concentration finale de 100 nM. On enregistre à nouveau le spectre d'émission de la solution. On ajoute ensuite un excès (1-10 *µ*M) de ligand non marqué spécifique du récepteur NK2R (NKA non marquée ou de SR 48968 (Sanofi recherche) ou du peptide cyclique, cyclo(-Gln-Trp-Phe-Gly-Leu-Met), et on enregistre à nouveau le spectre d'émission de la solution. La Figure 9 montre les trois spectres superposés sur lesquels on détecte nettement une extinction de la fluorescence de la EGFP (pic à 510 nm) lors de l'addition de NKA BO I et un retour à la fluorescence initiale lors de l'addition subséquente de ligand non marqué. on détecte aussi l'apparition d'un pic à 550-560 nm lors de l'addition du ligand fluorescent et une diminution de son intensité lors de l'addition de ligand non marqué. Ces résultats indiquent l'occurrence d'un transfert d'énergie entre le récepteur fluorescent et son ligand fluorescent. Ce signal est renversable par addition d'un agent pharmacologique susceptible d'inhiber l'interaction entre le ligand et son récepteur. L'addition de NKA non fluorescente sur des cellules exprimant le récepteur fluorescent NK2R-RF1 est sans effet sur le spectre d'émission de fluorescence.
   b) Interactions entre la NKA BO I et le récepteur NK2R-RF1 en temps réel :
      Les mêmes enregistrements peuvent être effectués en temps réel. Pour cela, la longueur d'onde d'excitation est fixée à 460 nm et la longueur d'onde d'émission à 510 nm. L'addition de NKA BO I sur une suspension de cellules entraîne une diminution de l'intensité de fluorescence à 510 nm et l'addition subséquente d'un excès de ligand non marqué rétablit la fluorescence à sa valeur initiale (voir figure 10). La valeur finale de l'intensité de fluorescence peut être supérieure à la valeur initiale lorsque l'absorption du ligand fluorescent contribue au signal. Ceci est observé avec la NKA BO I pour des concentrations supérieures à 200 nM, lorsque la concentration en cellules est de l'ordre de 5x10⁵ à 1x10⁶ cellules/ml. Enfin, l'addition de NKA non fluorescente au temps 0 est sans effet sur l'intensité du signal mesuré à 510 nM.
   c) Détection de l'interaction entre le récepteur NK2R-RF1 et les ligands NKA TR et NKA Eos :
      La reproduction de l'expérience décrite en a) avec la NKA TR (figure 11a) ou la NKA Eos (figure 11b) indique que la détection d'un signal d'interaction récepteur NK2R-RF1 ligand fluorescent n'est pas restreinte au couple EGFP-BODIPY mais peut aussi être étendue aux couples EGFP-sulforhodamine 101 ou EGFP-Eosine.
   d) Expérience de saturation de la liaison de NKA BO I à ses sites récepteurs :
      Etant donné que l'amplitude du signal enregistré selon la description en 8b (figure 12) est proportionnel à la concentration de NKA BO I ajoutée, on peut effectuer une mesure de l'amplitude du signal spécifique en fonction de la concentration de ligand ajouté. La figure 12 montre la variation d'amplitude du signal lorsque la concentration de NKA BO I ajoutée varie de 0 à 256 nM, par addition successive d'aliquots d'une solution stock de 10-5 M sur une suspension de 1 ml de cellules à 10⁶ cellules/ml dans le tampon physiologique Hepes. Le traitement des données expérimentales (figure 12) permet d'extraire une valeur d'affinité de la NKA BO I (KD = 20-30 nM) en excellent accord avec les mesures effectuées par déplacement d'un radioligand ainsi qu'il est décrit dans l'exemple 5, I).
   e) Expérience de compétition :
      Les fragments de membrane contenant le récepteur NK2R-RF1 préparés selon le procédé décrit dans l'exemple 3, section préparation de fragments de membranes, sont dilués dans le tampon Hepes (Hepes 10 mM, NaCl 137,5 mM, MgCl₂, 1,25 mM, CaCl₂ 1,25 mM, KCl 6 mM, glucose 5,6 mM, NaH₂PO₄ 0,4 mM, BSA 0,1% (w/v), pH 7,4) à raison de 20 µL de membranes/ml. Des aliquots des membranes sont incubés en présence de NKA, MEN 10,376, de cyclo(-Gln-Trp-Phe-Gly-Leu-Met) ou de SR 48968 à différentes concentrations. Après 30 minutes d'incubation, les échantillons sont placés dans une cuve de spectrofluorimétrie et le signal engendré par addition de NKA BO I 25 nM est enregistré pendant 120 sec. à 510 nm, la longueur d'onde d'excitation ayant été fixée à 470 nm. L'amplitude du signal évoqué par l'interaction entre le récepteur NK2R-RF1 et son ligand NKA BO I étant proportionnel au nombre de sites de liaison accessibles au ligand fluorescent, on peut établir une courbe de déplacement de la NKA BO I par le ligand non fluorescent.
      On peut aussi procéder par mélange simultané des fragments de membrane contenant le récepteur NK2R-RF1, le ligand NKA BO I et le ligand non marqué. Le mélange est ensuite incubé pendant le temps nécessaire à atteindre l'équilibre de liaison (30-60 min.) et la fluorescence est mesurée à 510 nm en fixant la longueur d'onde d'excitation à 470 nm. Les mêmes expériences peuvent être effectuées avec des cellules entières à la concentration de 250 000 à 1 000 000 de cellules/ml.
      La figure 14 montre que l'amplitude du signal de fluorescence mesuré à 510 nm (excitation 460 nm) décroît lorsque la concentration de molécule non fluorescente présente dans l'essai augmente. La courbe d'inhibition de la liaison de NKA Bo I permet d'estimer l'affinité des molécules neurokinine A et SR48968 non fluorescentes.
      La figure 15 correspond à l'expérience décrite dans la figure 14, effectuée en présence de neurokinine A (NKA) ou de SR48968.

### EXEMPLE 6 : CLONAGE DE L'ADNC NK2R-RF1 DANS LE VECTEUR PPIC9 ET EXPRESSION DANS LA LEVURE PICHIA PASTORIS

a) Clonage :
   La partie de pCEP4-NK2R-RF1 codant pour la protéine de fusion est amplifiée par PCR (Current Protocols in Molecular Biology, op.cit.) en utilisant les amorces et permettant 1) la production d'un fragment de 1868nt codant pour la totalité de la protéine de fusion NF2R-EGFP à l'exception du peptide signal et 2) le clonage en phase dans le vecteur d'expression de levure pPIC9 (invitrogen) avec la séquence codant pour le peptide signal du facteur alpha promoteur du gène AOX1. Les sites de clonage utilisés sont respectivement XHoI pour l'extrémité 5' du produit d'amplification et Not I pour son extrémité 3'
b) Expression :
   Les levures sont transformées avec le plasmide pPIC9-NK2R-RF1 linéarisé (StuI ou SalI) et mises en culture sur milieu MD sans histidine, préparé selon les indications du manuel fourni avec le vecteur pPIC9 (inVitrogen). L'expression de la construction d'ADN introduite dans la cellules est induite par le Méthanol. Pour cela, les colonies sont propagées en milieu liquide (BMGY) pendant 24 h, puis transférées dans du milieu BMMY contenant 0.5 % de méthanol permettant l'induction de l'expression de la construction d'ADN NK2R-RF1. Des aliquots de ces cultures sont prélevés et les clones exprimant la protéine fluorescente sont identifiés par mesure des spectres d'excitation et d'émission de la EGFP.

### EXEMPLE 7 : CONSTRUCTION D'ADN CODANT POUR LE RECEPTEUR MUSCARINIQUE DE L'ACETYLCHOLINE FUSIONNE AVEC LA EGFP ET EXPRESSION DANS LES CELLULES MAMMIFERES.

I) Clonage : le fragment d'ADNc codant pour le récepteur humain muscarinique M1 (Genbank Accession N° X15263) est amplifié par PCR (Current Protocols in Molecular Biology, op.cit.) en utilisant les amorces : et
   Le fragment de 1383 nt obtenu est clivé par les enzymes Not I (en 5') et Xho I (en 3') et ligué avec le vecteur KS ouvert par les mêmes enzymes (fragment de 2888nt) pour conduire à la construction KS-hM1, ou ligué avec le vecteur pCEP4 ouvert par les mêmes enzymes pour conduire à la construction pCEP4-hM1.
   La construction KS-hM1 est utilisée pour la production d'ADN simple brin (Current Protocols in Molecular Biology, op.cit.) et l'obtention de mutants.
II) fusion de la EGFP en N-terminal du récepteur hM1.
   L'oligonucléotide 5'CCTGCTGTCTCAGATCTCATCACCGTCC3' est utilisé, conjointement avec les réactifs du kit de mutagenèse Sculptor (Amersham) pour produire un mutant permettant la fusion en position 13 de la séquence codante du récepteur hM1 grâce à l'introduction d'un site de restriction pour l'enzyme Bg1 II.
   Le mutant obtenu est digéré par les enzymes Bgl II et Xho I et le fragment de 1354 nt engendré, est ligué avec le fragment Not I - Bgl II, 812 nt, de pCEP4-NK2R-RF1, et le vecteur pCEP4 ouvert par les enzymes Not I et Xho I, pour conduire à la construction d'ADN pCEP4-hM1-RF1.
III) expression de la protéine de fusion hM1-RF1.
   Le plasmide pCEP4-hM1-RF1 est introduit dans des cellules HEK 293 par transfection au phosphate de calcium ou dans des cellules Cos 1 par électroporation (Current Protocols in Molecular Biology, op.cit.).
   L'expression de la protéine est détectée comme décrit plus haut.
IV) synthèse et purification du ligand muscarinique ABT-Bodipy.
   L'ABT base (3-[2'-aminobenzhydryloxy]tropane) est mis en solution dans la DMF (10 mM). 20 µl de cette solution (0.2 µMoles) sont mélangés avec 4 µl d'une solution 100 mM de Bodipy -IA et laissés à température ambiante pendant 20 h. Le produit de la réaction est purifié par HPLC (Gilson) sur une colonne de phase inverse Z5C8 25F (Zorbax) sur laquelle on développe un gradient linéaire de 10 à 95 % solvant B en 60 minutes (A: H₂O 0.1 % TFA; B: CH₃CN 0.1 % TFA) avec un débit de 1 ml/min. Les longueurs d'ondes de détection sont fixées à 219 nm (peptide) et 530 nm (fluorophore). Le produit élué (ABT Bo) au temps 34 min. (Figure 7) est collecté, concentré par évaporation et resuspendu dans de la DMF.
V) fusion de la EYFP en N-terminal du récepteur hM1 et détection de l'interaction avec la pirenzepine-bodipy 558/568.

Cette construction est réalisée de manière identique à la construction décrite au point II.

Les interactions de la protéine de fusion avec son ligand sont effectuées à l'aide du ligand pirenzépine-bodipy dans lequel le groupe fluorescent correspond au bodipy 558/568 (4,4-difluoro-5-(2-thiényl)-4-bora-3a,4a-diaza-s-indacene-3-propionic acid).

### EXEMPLE 8 : CONSTRUCTION D'ADN CODANT POUR DES RECEPTEURS NICOTINIQUES FLUORESCENTS

a) Construction d'ADN codant pour la fusion de la EGFP dans la région MIR du récepteur a7-V201-5HT₃.
   Le plasmide pJL223 (Eiselé *et al.* 1993, Nature 366:479-483) contient le gène de la protéine a7-V201-5HT₃ qui forme un récepteur canal activé par l'acétylcholine et la, nicotine lors de son expression dans des ovocytes de Xénope. l'ADNc codant est compris entre les sites Not I et Xho I du vecteur pMT3 (Swick, A.G. *et al.* 1992, Proc. Natl. Acad. Sci. 89:1812-1816).
   L'insert Not I- Xho I de 1424 nt est cloné entre les sites Not I et Xho I du vecteur Bluescript et le plasmide obtenu (KS 223) sert de matrice à la production d'ADN simple brin.
   L'oligonucléotide est utilisé pour introduire, sur KS223, les sites de restriction pour les enzymes Bsrg I, Bgl II et BamH I dans la même phase que celle des sites identiques portés par le plasmide pEGFP-C3. La mutation est introduite au niveau d'une région du récepteur appelée MIR (Major Immunogenic Region, Barkas *et al.* 1987, Science, 235:77-80) entre les acides aminés 63 et 64.
   Les fragments Not I-BsrG I (267 nt) et Bgl II-Xho I (1147 nt) de ce mutant de KS 223 sont ligués avec le fragment BsrG I-Bgl II (721 nt) de pEGFP-C3 et le vecteur pCEP4 ouvert par les enzymes Not I et Xho I, pour conduire à la construction d'ADN pCEP4-223-RF1.
   Les fragments Not I-BsrG I (267 nt) et BamH I-Xho I (1138 nt) de ce mutant de KS 223 sont ligués avec le fragment BsrG I-BamH I (772 nt) de pEGFP-C3 et le vecteur pCEP4 ouvert par les enzymes Not I et Xho I, pour conduire à la construction d'ADN pCEP4-223-RF2.
b) Construction d'ADN codant pour la fusion de la EGFP dans la région cytoplasmique du récepteur α7-V201-SHT₃,
   L'ADNc codant pour la protéine α7-V201-5HT₃ contient dans son domaine cytoplasmique les séquences des sites Avr II et Pst I respectivement en phase et cohésive avec les séquences clivées par les enzymes Nhe I et Pst I du plasmide pEGFP-C2 (ClonTech) et permettent l'obtention d'un protéine de fusion contenant la séquence de le EGFP dans le domaine cytoplasmique de la protéine α7-V201-5HT₃.
   La construction d'ADN codant pour cette protéine de fusion est donc obtenue par ligation des fragments Not I-Avr II (1036 nt) et Pst I - Xho I (286 nt) de KS 223 avec le fragment Nhe I - Pst I (774 nt) de pEGFP-C2 et le vecteur pCEP4 ouvert par les enzymes Not I et Xho I pour conduire à la construction pCEP4-223-RF3.
   Les constructions pCEP4-223-RF1, -RF2, et -RF3 sont ensuite exprimées dans des cellules HEK 293 ainsi que décrit plus haut.

### EXEMPLE 9 : CONSTRUCTION D'ADN CODANT POUR UN RÉCEPTEUR DES CHIMIOKINES FLUORESCENT

Le cDNA codant pour le récepteur CCR5 humain des chimiokines (numéro d'accès Genbank : U54994) est amplifié à l'aide des oligonucléotides 5'GGCCCAAGCTTATGTCAGGATCCGGGGAT3' et 5'CGCCCGCTCGAGTCACAAGCCCACAGATAT puis cloné dans le vecteur Bluescript KS ouvert par l'enzyme de restriction Eco RV.

L'insert est excisé par les enzymes Hind III ou BamH1 (5') et Xho I (3') (fragment 1) pour être cloné en phase avec le cDNA codant pour la EGFP ou la ECFP.

La fusion en phase de la EGFP ou la ECFP avec le peptide signal du plasmide pJL223 (exemple 1) et le gène codant pour le récepteur CCR5, est effectuée de la manière suivante. Le plasmide pJL223 est ouvert par l'enzyme de restriction Age I puis rendu franc par mélange avec la Klenow et des nucléotides. Le fragment s'étendant jusqu'au site Xho I est ensuite éliminé (coupure Xho I) (fragment 2).

Les plasmides pEGFP-C3 et pECFP-C3 sont ouverts par l'enzyme Age I et les extrémités sont rendues franches. Les inserts Age I franc- Hind III et Age I franc- Xho I (fragments 3) sont ligués avec les fragments 1 et 2 pour donner lieu au constructions KS-SP EGFP-Hind III-CCR5, KS-SP EGFP-Xho I-CCR5, KS-SP ECFP-Hind III-CCR5 et KS-SP ECFP-Xho I-CCR5. Les ADNs correspondant aux séquences SP EGFP-Hind III-CCR5, SP EGFP-Xho I-CCR5, SP ECFP-Hind III-CCR5, et SP ECFP-Xho I-CCR5 sont excisés par Not I et Xho I puis clonés dans le vecteur pCEP4 ouvert pas les mêmes enzymes.

La figure 16 montre le spectre d'émission de cellules HEK 293 transfectées et exprimant le récepteur EGFP-Hind III-CCR5, et la figure 17 montre la réponse de libération de calcium intracellulaire de cellules exprimant la construction d'AND EGFP-HindCCR5.

### EXEMPLE 10 : CONSTRUCTION D'ADN CODANT POUR LA CHIMIOKINE HUMAINE RANTES FLUORESCENTE

L'ADNc codant pour la chimiokine RANTES humaine (numéro d'accès Genbank M21121) est synthétisé par PCR récursive à l'aide des oligonucléotides selon les protocoles décrits dans Prodromou & Pearl, Protein Engineering Vol 5, pp 827-829, 1992, puis cloné dans le vecteur Bluescript KS ouvert par Eco RV.

Les ADN codant respectivement pour la chimiokine RANTES (fragment Hind III - Not I ou Bam HI-Not I) et pour la protéine fluorescente jaune (fragments Age I franc - Bam HI ou Age I franc - Hin DIII du plasmide pEYFP-C3) sont clonés en phase avec le peptide signal de l'alpha-mating factor de levure entre les sites Sna BI et Not I du vecteur pPIC 9, pour conduire aux constructions pPIC9-EYFP- HindIII-RANTES et pPIC9-EYFP-Bam HI-RANTES.

La figure 18 montre un immunoblot révélant l'expression de la construction EYFP- HindIII-RANTES dans un surnageant de culture de levure *pichia pastoris* transformée par le plasmide pPIC9-EYFP- HindIII-RANTES, et détectée à l'aide d'une anticorps anti-GFP.

### EXEMPLE 11 : IDENTIFICATION DE NOUVEAUX LIGANDS POUR DES RECEPTEURS ORPHELINS

1) fusion de la EGFP avec l'extrémité carboxyterminale du récepteur NK2R des tachykinines.
   l'ADNc codant pour le récepteur NK2R compris dans le plasmide pKS NK2R (exemple 1) est clivé par l'enzyme Age I. L'extrémité 5' dépassante obtenue est rendu franche à l'aide de nucleotides et de la Klenow polymerase. L'ADN linéarisé est clivé par l'enzyme Not I pour produire un fragment de 1045 nt (fragment a).
   Le plasmide pEGFP-C3 est ouvert à l'aide de l'enzyme Age I puis rendu franc. Il est ensuite clivé par l'enzyme Xho I pour conduire à un fragment b, de 739 nt.
   Les fragments a et b sont ligués avec le vecteur pCEP4 ouvert par les enzymes Not I et Xho I pour conduire au plasmide pCEP4-NK2R-AgeI-EGFP.
2) fusion de la protéine fluorescente EYFP avec l'extrémité aminoterminale de la sous-unité alpha de la protéine Gq de souris.
   L'ADNc codant pour la sous-unité alpha de la protéine Gq de souris (Genbank accession N° M55412) est amplifiée par PCR à l'aide des l'oligonucléotides 5'GCGGCCGCATGGGGGATCCTACTCTGGAGTCCATCATGGCG et 5' CCGCTCGAGTTAATCTAGAAGGACCAGATTGTACTCCTTCAGG afin d'introduire des sites Not I et Bam HI en 5' et des sites Xho I et Xba I en 3' du gène codant pour la sous-unité alpha de la protéine Gq.
   Le produit PCR obtenu est cloné dans le vecteur KS ouvert par Eco RV.
   Le cDNA codant pour la sous-unité alpha Gq est excisé par les enzymes BamHI et Xba I et ligué dans le plasmide pEYFP-C3 ouvert par les enzymes Bgl II et Xba I pour conduire au plasmide pEYFP-Bgl II-Gq .
3) coexpression du récepteur NK2R-Age I-EGFP et de la protéine EYFP-Bgl II-Gq .
   Des cellules HEK 293 sont transfectées à l'aide des plasmides pEYFP-Bgl II-Gq ou pCEP4-NK2R-AgeI-EGFP epEYFP-Bgl II-Gq Les cellules exprimant le produit des gènes du plasmide pCEP4 sont sélectionnées à l'aide de l'antibiotique hygromycine B, celles qui expriment les produits du gène du plasmide pEGFP modifié sont sélectionnées à l'aide de néomycine.
4) tests de fonctionnalité de la protéine Gq fluorescente.
   Ils sont effectués par le biais d'études fonctionnelles de libération de calcium. La surexpression de protéines G entraîne une augmentation de l'amplitude des réponses calciques.
5) les cellules exprimant la protéine Gq de manière stable sont transfectées à l'aide d'un plasmide d'expression codant pour un récepteur orphelin. L'effet de molécules est mesuré par fluorescence à 405 nm en excitant la suspension de cellules à 355 nm.

## Revendications

1. Utilisation d'une protéine fluorescente choisie parmi les protéines fluorescentes issues ou dérivées de protéines autofluorescentes de cnidaires, dont le coefficient d'extinction moléculaire est supérieur à 14000 M⁻¹cm⁻¹ et le rendement quantique de fluorescence est supérieur à 0,38,
pour la détection et la quantification d'interactions non covalentes sur des cellules ou des fragments de cellules entre un récepteur membranaire marqué par voie génétique par la protéine fluorescente susmentionnée et l'un de ses ligands marqué par un marqueur constitué :
- soit par une molécule susceptible d'absorber la lumière émise par la protéine fluorescente,
- soit par une substance fluorescente,
cette détection et quantification ayant lieu par transfert d'énergie de fluorescence :
. entre la protéine fluorescente susmentionnée et la susdite substance fluorescente, la substance fluorescente étant telle que soit elle est excitable à la longueur d'onde d'émission de la protéine fluorescente susmentionnée, soit elle émet à la longueur d'onde d'excitation de la protéine fluorescente susmentionnée ou
. entre la protéine fluorescente susmentionnée et la susdite molécule susceptible d'absorber la lumière émise par la protéine fluorescente.

2. Utilisation selon la revendication 1, **caractérisée en ce que** la protéine fluorescente est choisie parmi la protéine fluorescente verte GFP ou EGFP, la protéine fluorescente cyan CFP ou ECFP, la protéine fluorescente jaune, YFP ou EYFP, ou GFPUV.

3. Utilisation d'une protéine fluorescente (n° 1) selon l'une des revendications 1 ou 2, dans laquelle le ligand est marqué
* soit par une substance fluorescente, le marquage étant :
- soit effectué par voie chimique, la substance fluorescente étant alors un composé chimique,
- soit effectué par voie recombinante, la substance fluorescente étant alors un peptide ou une protéine fluorescente (n° 2), choisie parmi :
- la protéine fluorescente verte GFP, ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence,
* soit par une substance non fluorescente appartenant au groupe des acides violets, acides rouges, les alizarines, l'aluminon, l'azocarmine B, la fuschine basique, le Bordeaux R, la Carmine.

4. Utilisation d'une protéine fluorescente selon l'une des revendications 1 à 3, dans laquelle le récepteur membranaire et le ligand sont marqués par voie génétique, la protéine fluorescente et la substance fluorescente étant respectivement choisies parmi les couples de composés suivants :
GFPUV-EYFP
EYFP - GFPUV
ECFP - EYFP
EYFP - ECFP
ECFP - EGFP
EGFP - ECFP
EGFP - EYFP
EYFP - EGFP

5. Utilisation selon la revendication 4, dans laquelle le récepteur membranaire est marqué par la protéine EYFP ou EGFP et le ligand est marqué par la protéine ECFP, ou le récepteur membranaire est marqué par la protéine ECFP et le ligand est marqué par la protéine EYFP ou EGFP.

6. Utilisation selon la revendication 1 ou 2 d'une protéine fluorescente choisie parmi :
- la protéine fluorescente verte GFP, ou
- des variants dérivés de la GFP, par addition, délétion ou substitution d'un ou plusieurs acides aminés, sous réserve que ces variants conservent la propriété de fluorescence,
- ou des fragments de la GFP, ou de fragments des susdits variants, sous réserve que ces fragments conservent la propriété de fluorescence,
pour la détection et la quantification d'interactions non covalentes entre un récepteur membranaire marqué par voie génétique par la GFP ou l'un des variants ci-dessus définis ou l'un des fragments ci-dessus définis et l'un de ses ligands marqué par une substance fluorescente, cette détection et quantification ayant lieu par transfert d'énergie de fluorescence entre la GFP ou l'un des variants définis ci-dessus, ou l'un des fragments définis ci-dessus et la susdite substance fluorescente, la substance fluorescente étant telle que soit elle est excitable à la longueur d'onde d'émission de la GFP ou de l'un des susdits variants, ou de l'un des susdits fragments, soit elle émet à la longueur d'onde d'excitation de la GFP, ou de l'un des susdits variants, ou de l'un des susdits fragments.

7. Utilisation selon l'une des revendications 1 à 6, dans laquelle la protéine fluorescente est :
• soit EGFP et dans laquelle :
- soit la EGFP est donneur d'énergie de fluorescence et le marqueur absorbant la lumière émise par la EGFP est une substance fluorescente ou non, et le marqueur étant choisi parmi des substances, dont le spectre d'excitation chevauche le spectre d'émission de la EGFP,
et dans le cas où le marqueur est une substance fluorescente, il est choisi parmi : le 4,4-difluoro-4-bora-3a,4a-diaza-s-indacene (Bodipy), l'éosine, l'érythrosine, la tétraméthylrhodamine, la sulforhodamine 101 commercialisée par Molecular probe sous la dénomination Texas Red, et leurs dérivés permettant d'une part le greffage et, d'autre part, dont le spectre d'excitation recouvre le spectre d'émission de EGFP,
et dans le cas où le marqueur n'est pas une substance fluorescente, il est choisi parmi le groupe des acides violets, acides rouges, les alizarines, l'aluminon, l'azocarmine B, la fuschine basique, le Bordeaux R, la Carmine,
- soit la EGFP est accepteur d'énergie de fluorescence et la substance fluorescente est donneur d'énergie de fluorescence et est choisie parmi des substances, dont le spectre d'émission chevauche le spectre d'excitation de la EGFP, lesdites substances étant choisies parmi : les coumarines, la fluorescamine, le 6-(N-méthylanilino)naphtalène, (mansyl) et leurs dérivés permettent d'une part le greffage et, d'autre part, dont le spectre d'émission recouvre le spectre d'excitation de EGFP,
• soit ECFP et dans laquelle :
- soit la ECFP est donneur d'énergie de fluorescence et la substance fluorescente est accepteur d'énergie et est choisi parmi la fluoresceïne et le 7-nitrobenz-2-oxa-1,3-diazole,
- soit la ECFP est accepteur d'énergie de fluorescence et la substance fluorescente est donneur d'énergie et est choisie parmi le pyrène ou la coumarine ou leurs dérivés permettant d'une part le greffage, et, d'autre part, dont le spectre d'émission chevauche le spectre d'excitation de la ECFP.

8. Utilisation selon l'une des revendications 1 à 7, dans laquelle le récepteur membranaire est choisi parmi :
- les récepteurs membranaires couplés à la protéine G,
- les récepteurs des facteurs de croissance, et
- les récepteurs canaux.

9. Utilisation selon l'une des revendications 1 à 8, dans laquelle le récepteur membranaire est choisi parmi les récepteurs membranaires couplés à la protéine G.

10. Procédé de détection et de quantification d'interactions non covalentes entre un récepteur membranaire et l'un de ses ligands, **caractérisé en ce que** :
- on prépare des cellules soit des fragments de cellules exprimant une séquence d'ADN comprenant le gène codant pour une protéine fluorescente fusionnée avec le gène du récepteur membranaire, la fusion entre le gène de la protéine fluorescente et le gène du susdit récepteur membranaire étant telle que les propriétés du récepteur membranaire ne sont pas modifiées par la présence de la protéine fluorescente, à savoir :
* l'interaction entre le récepteur membranaire et le ligand n'est pas modifiée,
* la fonction de transduction de la réponse n'est pas modifiée, la protéine fluorescente étant choisie parmi les protéines fluorescentes issues ou dérivées de protéines autofluorescentes de cnidaires, dont le coefficient d'extinction moléculaire est supérieur à 14000 M⁻¹cm⁻¹ et le rendement quantique de fluorescence est supérieur à 0,38,
- on met en présence les susdites cellules ou les susdits fragments de cellules avec un ligand du susdit récepteur membranaire marqué par un marqueur constitué :
- soit par une molécule susceptible d'absorber la lumière émise par la protéine fluorescente,
- soit par une substance fluorescente
et soit la protéine fluorescente étant donneur d'énergie de fluorescence et le marqueur étant accepteur d'énergie de fluorescence, ou soit la protéine fluorescente étant accepteur d'énergie de fluorescence et le marqueur étant une substance fluorescente donneur d'énergie de fluorescence, et
- on irradie à une longueur d'onde permettant soit d'exciter la protéine fluorescente, soit d'exciter la substance fluorescente,
- les susdites étapes de mise en présence et d'irradiation pouvant être effectuées soit simultanément, soit l'une après l'autre, ou
- on met en présence les susdites cellules ou les susdits fragments de cellules avec un ligand de la susdite protéine marqué par un marqueur, les cellules ou le ligand ayant été irradiés préalablement à leur mise en présence,
- on détecte soit une diminution d'amplitude de l'émission du donneur et/ou un signal d'émission caractéristique de l'émission de l'accepteur.

11. Procédé selon la revendication 10, **caractérisé en ce que** la protéine fluorescente est choisie parmi la protéine fluorescente verte GFP ou EGFP, la protéine fluorescente cyan CFP ou ECFP, la protéine fluorescente jaune YFP ou EYFP, ou GFPUV.

12. Procédé de détection et de quantification d'interactions non covalentes entre un récepteur membranaire et l'un de ses ligands, **caractérisé en ce que** :
- on prépare une protéine fluorescente fusionnée avec un récepteur membranaire dont on veut déterminer l'interaction récepteur-ligand, la fusion entre la protéine fluorescente et le susdit récepteur membranaire étant telle que les propriétés du récepteur ne sont pas modifiées par la présence de la protéine fluorescente, à savoir :
* l'interaction entre le récepteur membranaire et le ligand n'est pas modifiée,
* la fonction de transduction de la réponse n'est pas modifiée,
la protéine fluorescente étant telle que définie dans la revendication 10 ou 11, et,
- on met en présence la susdite protéine fluorescente fusionnée avec le récepteur membranaire avec un ligand du susdit récepteur, ce ligand étant marqué par un marqueur constitué:
- soit par une molécule susceptible d'absorber la lumière émise par la protéine fluorescente,
- soit par une substance fluorescente,
et soit la protéine fluorescente étant donneur d'énergie de fluorescence et le marqueur étant accepteur d'énergie de fluorescence, ou soit la protéine fluorescente étant accepteur d'énergie de fluorescence et le marqueur est une substance fluorescente donneur d'énergie de fluorescence, et
- on irradie à une longueur d'onde permettant soit d'exciter la protéine fluorescente, soit d'exciter la substance fluorescente,
- les susdites étapes de mise en présence et d'irradiation pouvant être effectuées soit simultanément, soit l'une après l'autre, ou
- on met en présence la susdite protéine fluorescente fusionnée avec le récepteur membranaire avec un ligand du susdit récepteur, ce ligand étant marqué par un marqueur constitué :
- soit par une molécule susceptible d'absorber la lumière émise par la protéine fluorescente,
- soit par une substance fluorescente,
la protéine fluorescente fusionnée avec le récepteur membranaire ou le ligand ayant été irradiés préalablement à leur mise en présence,
- on détecte soit une diminution d'amplitude de l'émission du donneur et/ou un signal d'émission caractéristique d'émission de l'accepteur.

13. Procédé selon l'une des revendications 10 à 12, dans lequel on introduit une étape supplémentaire :
- avant ou après, ou simultanément à l'étape de mise en présence de la protéine fluorescente fusionnée avec le récepteur membranaire et d'un ligand du susdit récepteur membranaire, ce ligand étant marqué par un marqueur, ou
- avant, ou après, ou simultanément à l'étape de mise en présence de cellule ou de fragments de cellules et d'un ligand du susdit récepteur membranaire, marqué par un marqueur,
cette étape supplémentaire consistant :
- soit à mettre la susdite protéine fluorescente fusionnée avec le récepteur membranaire en présence du susdit ligand non marqué et simultanément du susdit ligand marqué,
- soit à mettre en présence les susdites cellules ou les susdits fragments de cellules en présence simultanée du susdit ligand non marqué et du susdit ligand marqué,
- on détecte soit une diminution d'amplitude de l'émission du donneur et/ou un signal d'émission caractéristique de l'émission de l'accepteur, respectivement dans le cas de l'utilisation du ligand marqué et dans le cas de l'utilisation simultanée du ligand marqué et du ligand non marqué,
- et on compare soit les diminutions d'amplitude de l'émission du donneur respectivement obtenues et/ou les signaux d'émission caractéristiques de l'émission de l'accepteur respectivement obtenus.

14. Procédé selon la revendication 13, dans lequel la protéine fluorescente est EGFP et dans lequel :
- soit la EGFP est donneur d'énergie de fluorescence et le marqueur est accepteur d'énergie de fluorescence et est choisie parmi des substances, dont le spectre d'excitation chevauche le spectre d'émission de la EGFP,
et dans le cas où le marqueur est une substance fluorescente, il est choisi parmi : le 4,4-difluoro-4-bora-3a,4a-diaza-s-indacene (Bodipy), l'éosine, l'érythrosine, la tétraméthylrhodamine, la sulforhodamine 101 commercialisée par Molecular probe sous la dénomination Texas Red, et leurs dérivés permettent d'une part le greffage et, d'autre part, dont le spectre d'excitation recouvre le spectre d'émission de EGFP,
et dans le cas où le marqueur n'est pas une substance fluorescente, il est choisi parmi le groupe des acides violets, acides rouges, les alizarines, l'aluminon, l'azocarmine B, la fuschine basique, le Bordeaux R, la Carmine,
- soit la EGFP est accepteur d'énergie de fluorescence et la substance fluorescente est donneur d'énergie de fluorescence et est choisie parmi des substances, dont le spectre d'émission chevauche le spectre d'excitation de la EGFP, lesdites substances étant choisies parmi : les coumarines, la fluorescamine, le 6-(N-méthylanilino)naphtalène, (mansyl) et leurs dérivés permettent d'une part le greffage et, d'autre part, dont le spectre d'émission recouvre le spectre d'excitation de EGFP.

15. Procédé selon l'une des revendications 10 à 12, dans lequel le récepteur membranaire dont on veut déterminer l'interaction récepteur-ligand est choisi parmi :
- les récepteurs membranaires couplés à la protéine G,
- les récepteurs des facteurs de croissance et
- les récepteurs canaux.

16. Procédé selon la revendication 15, **caractérisé en ce que** les récepteurs des facteurs de croissance sont choisis parmi ceux qui sont structurellement reliés au récepteur de l'insuline ou au récepteur de l'interféron γ.

17. Procédé selon l'une des revendications 10 à 16, dans lequel la protéine fluorescente est la EGFP et le marqueur est le Bodipy et dans lequel on détecte soit la diminution d'amplitude d'émission de la EGFP, soit le signal d'émission du Bodipy résultant du transfert d'énergie, la longueur d'onde d'irradiation correspondant à la longueur d'onde d'excitation de la EGFP.

18. Procédé selon l'une des revendications 10 à 17, dans lequel la protéine fluorescente est la EGFP et le marqueur est une coumarine et dans lequel on détecte soit la diminution d'amplitude de la coumarine, soit le signal d'émission de la EGFP résultant du transfert d'énergie, la longueur d'onde d'irradiation correspondant à la longueur d'onde d'excitation de la coumarine.

19. Procédé selon l'une des revendications 10 à 18, dans lequel la protéine fluorescente est fusionnée du côté N-terminal et le récepteur membranaire est fusionné du côté C-terminal.

20. Procédé selon l'une des revendications 10 à 19, dans lequel la protéine fluorescente est fusionnée du côté C-terminal et le récepteur membranaire est fusionné du côté N-terminal.

21. Procédé selon l'une des revendications 10 à 20, dans lequel la protéine fluorescente est insérée dans le récepteur membranaire à un endroit ne correspondant pas à un site de liaison récepteur membranaire-ligand, dans le cas des récepteurs couplés à la protéine G, cette insertion ayant lieu dans la première ou la troisième boucle intracellulaire du récepteur, sous réserve que l'insertion ne détruise ni les propriétés du récepteur, ni la fluorescence de la protéine fluorescente.

22. Procédé selon l'une des revendications 10 à 21, dans lequel les cellules sont des cellules de mammifères, choisies parmi les cellules HEK 293 adhérentes ou en suspension, cellules CHO, cellules COS, lignées lymphocytaires, fibroblastes, ou des cellules de levure, choisies parmi les levures *pichia, saccharomyces, Hansenula,* ou des cellules d'insectes infectées par un virus, les dites cellules étant choisies parmi les cellules TNI ou sf9, ou des champignons, choisis parmi les souches de *Aspergillus, Neurospora, Fusarium, Trichoderma.*

23. Procédé selon l'une quelconque des revendications 10 à 22, dans lequel un signal est détectable, dans un appareil de fluorimétrie conventionnel ou dans un appareil de mélange rapide équipé d'un système de détection de fluorescence, après mélange du donneur et de l'accepteur et peut être aboli par l'addition d'une substance non fluorescente de même spécificité pharmacologique, et dans lequel le rapport signal/bruit est supérieur à environ 2.

24. Utilisation selon l'une des revendications 1 à 3, pour la détection et la quantification d'interactions non covalentes entre un récepteur membranaire constitué par un récepteur couplé aux protéines G et une protéine G, en vue d'identifier les molécules biologiquement actives vis-à-vis du récepteur, susceptibles de former une interaction non covalente réversible avec ledit récepteur, ledit récepteur étant marqué par voie génétique par la protéine fluorescente telle que définie dans l'une des revendications 1 à 3 et la protéine G étant marquée par un marqueur tel que défini dans l'une des revendications 1 à 3.

25. Utilisation selon la revendication 24, dans laquelle le récepteur est choisi parmi :
- les récepteurs couplés aux protéines G,
- les séquences codant pour des récepteurs couplés aux protéines G putatifs dont les molécules biologiquement actives vis-à-vis d'eux-mêmes sont à identifier.

26. Procédé selon l'une quelconque des revendications 10 à 23, **caractérisé en ce que** le récepteur membranaire est un récepteur couplé aux protéines G et **en ce que** le ligand est une protéine G, en vue d'identifier les molécules biologiquement actives vis-à-vis du récepteur, susceptibles de former une interaction non covalente réversible avec ledit récepteur, et **caractérisé en ce que** l'interaction entre le récepteur et la molécule biologiquement active n'est pas modifiée.

27. Procédé d'identification et éventuellement de quantification d'interactions entre un récepteur membranaire et une molécule non fluorescente biologiquement active vis-à-vis dudit récepteur, susceptibles de former une interaction non covalente réversible avec ledit récepteur, par mise en oeuvre du procédé selon la revendication 26, dans lequel une molécule non fluorescente biologiquement active est ajoutée à des cellules, ou fragments de cellules, exprimant l'ADN codant pour le récepteur marqué par la protéine fluorescente et pour la protéine G marquée par le marqueur, **caractérisé en ce que** :
- une molécule non fluorescente biologiquement active et agoniste déclenche une transduction de signal détectée par variation de transfert d'énergie entre le récepteur marqué par la protéine fluorescente et la protéine G marquée par le marqueur ;
- une molécule non fluorescente biologiquement active antagoniste inhibe la transduction de signal provoquée par un agoniste et détectée par variation de transfert d'énergie de fluorescence entre le récepteur marqué par la protéine fluorescente et la protéine G marquée par le marqueur.

28. Cellules ou fragment de cellules contenant une séquence d'ADN comprenant le gène codant pour une substance fluorescente fusionnée avec le gène d'un ligand constitué par la protéine G, la substance fluorescente étant choisie parmi les protéines fluorescentes issues ou dérivées de protéines autofluorescentes de cnidaires, dont le coefficient d'extinction moléculaire est supérieur à 14000 M⁻¹cm⁻¹ et le rendement quantique de fluorescence est supérieur à 0,38,
- et comprenant le gène codant pour une protéine fluorescente fusionnée avec le gène d'un récepteur membranaire, la protéine fluorescente étant choisie parmi les protéines fluorescentes issues ou dérivées de protéines autofluorescentes de cnidaires,
- la fusion entre le gène de la substance fluorescente et le gène de la susdite protéine G, et la fusion entre le gène de la protéine fluorescente et le gène du récepteur étant telle que
* les propriétés du récepteur ne sont pas modifiées par la présence de la protéine fluorescente, à savoir
* la fonction de transduction de la réponse n'est pas modifiée, sous réserve que :
* lorsque le récepteur membranaire est le récepteur des glucocorticoïdes de rat fusionné en N-terminal avec successivement une séquence de purification comportant 6 histidines, un épitope hémaglutinine et une protéine fluorescente et est exprimée dans la lignée cellulaire 1471.1, la protéine fluorescente est différente de la GFP (768 paires de bases du plasmide TU65 avec la mutation S65T),
* lorsque le récepteur membranaire est le récepteur humain des glucocorticoïdes tronqué de ses 131 premiers amino acides, fusionné en C-terminal d'une protéine fluorescente dans les sites Sal I et BamH I et est exprimée dans les cellules Cos-1, ladite protéine fluorescente est différente de la GFP de séquence Met Ser Lys Gly Glu Glu Leu Phe Thr Gly Val Val Pro Ile Leu Val Glu Leu Asp Gly Asp Val Asn Gly Gln Lys Phe Ser Val Ser Gly Glu Gly Glu Gly Asp Ala Thr Tyr Gly Lys Leu Thr Leu Lys Phe Ile Cys Thr Thr Gly Lys Leu Pro Val Pro Trp Pro Thr Leu Val Thr Thr Phe Ser Tyr Gly Val Gln Cys Phe Ser Arg Tyr Pro Asp His Met Lys Gln His Asp Phe Phe Lys Ser Ala Met Pro Glu Gly Tyr Val Gln Glu Arg Thr Ile Phe Phe Lys Asp Asp Gly Asn Tyr Lys Thr Arg Ala Glu Val Lys Phe Glu Gly Asp Thr Leu Val Asn Arg Ile Glu Leu Lys Gly Ile Asp Phe Lys Glu Asp Gly Asn Ile Leu Gly His Lys Met Glu Tyr Asn Tyr Asn Ser His Asn Val Tyr Ile Met Ala Asp Lys Pro Lys Asn Gly Ile Lys Val Asn Phe Lys Ile Arg His Asn Ile Lys Asp Gly Ser Val Gln Leu Ala Asp His Tyr Gln Gln Asn Thr Pro Ile Gly Asp Gly Pro Val Leu Leu Pro Asp Asn His Tyr Leu Ser Thr Gln Ser Ala Leu Ser Lys Asp Pro Asn Glu Lys Arg Asp His Met Ile Leu Leu Glu Phe Val Thr Ala Ala Gly Ile Thr His Gly Met Asp Glu Leu Tyr Lys,
* lorsque le récepteur membranaire est la sous-unité NMDA R1 de rat-exprimé dans les cellules HEK 293 fusionnée en C-terminal avec une protéine fluorescente, la protéine fluorescente est différente de celle constituée par les acides aminés 2-238 de la GFP sauvage,
* lorsque le récepteur membranaire est un récepteur ou un fragment de récepteur de messagers secondaires intracellulaires, la protéine fluorescente est différente de la GFP et de ses dérivés.

29. Trousse ou nécessaire pour la détection et la quantification d'interactions non covalentes entre un récepteur membranaire marqué par une protéine fluorescente telle que définie dans l'une des revendications 1 à 3, et l'un de ses ligands marqué par un marqueur constitué :
- soit par une molécule susceptible d'absorber la lumière émise par la protéine fluorescente,
- soit par une substance fluorescente,
ladite trousse comprenant :
- le récepteur membranaire fusionné avec une protéine fluorescente telle que définie ci-dessus ou une lignée cellulaire stable susceptible d'exprimer le récepteur membranaire fusionné avec une protéine fluorescente telle que définie ci-dessus ou un plasmide contenant la séquence nucléique codant pour ledit récepteur membranaire fusionné avec une protéine fluorescente telle que définie ci-dessus,
- le ligand marqué par le susdit marqueur,
- les tampons et milieux nécessaires au transfert d'énergie entre la susdite protéine et le susdit ligand.

30. Trousse selon la revendication 29, **caractérisée en ce que** le récepteur membranaire est marqué par une protéine fluorescente (n° 1) et l'un de ses ligands est marqué par une substance fluorescente correspondant à une protéine fluorescente (n° 2), la protéine fluorescente (n° 1) étant choisie parmi la protéine fluorescente EYFP ou EGFP et le ligand étant marqué par la protéine fluorescente (n° 2) ECFP ou la protéine fluorescente (n° 1) étant ECFP et le ligand étant marqué par la protéine fluorescente (n° 2) EYFP ou EGFP, ladite trousse comprenant :
- soit un plasmide contenant un acide nucléique codant pour le récepteur membranaire fusionné avec une protéine fluorescente (n° 1), et
* un plasmide contenant un acide nucléique codant pour le ligand fusionné avec une protéine fluorescente (n° 2), ou
* un ligand fusionné avec une protéine fluorescente (n° 2), obtenu par voie recombinante et purifié,
- soit une lignée cellulaire stable susceptible d'exprimer le récepteur membranaire fusionné avec une protéine fluorescente (n° 1), et
* une lignée cellulaire stable susceptible d'exprimer le ligand fusionné avec une protéine fluorescente (n° 2), ou
* un ligand fusionné avec une protéine fluorescente (n° 2), obtenu par voie recombinante et purifié.

31. Trousse ou nécessaire selon la revendication 30, **caractérisé en ce que** le récepteur membranaire est constitué par un récepteur couplé à la protéine G et **en ce que** le ligand correspond à la protéine G.

## Patentansprüche

1. Verwendung eines fluoreszierenden Proteins, ausgewälht aus den fluoreszierenden Proteinen, die von autofluorezenten Proteinen von Nesseltieren abgeleitet sind oder stammen, deren molarer Extinktionskoeffizient höher als 14.000 M⁻¹cm⁻¹ und deren Fluoreszenzquantenausbeute größer als 0,38 ist,
für das Auffinden und die Quantifizierung von nichtkovalenten Wechselwirkungen auf Zellen oder Fragmenten von Zellen zwischen einem Membranrezeptor, der auf genetischen Weg durch das genannte fluoreszierende Protein markiert ist, und einem seiner Liganden, der durch einen Marker markiert ist, der gebildet wird:
- entweder durch ein Molekül, das fähig ist, das durch das fluoreszierende Protein emittierte Licht zu absorbieren,
- oder durch eine fluoreszierende Substanz,
wobei das Auffinden und Quantifizieren durch Übertragung von Fluoreszenzenergie stattfindet:
. zwishen dem genannten fluoreszierenden Protein und der genannten fluoreszierenden Substanz, wobei die fluoreszierende Substanz so ist, dass sie entweder auf der Emisssionswellenlänge des gennanten fluoreszierenden Proteins anregbar ist, oder das sie bei der Anregungswellenlänge des genannten fluoreszierenden Proteins emittiert, oder
. zwishen dem genannten fluoreszierenden Protein und dem genannten Molekül, das fähig ist, das durch das fluoreszierende Protein emittierte Licht zu absorbieren.

2. Verwendung gemäß Anspruch 1, **dadurch gekennzeichnet, dass** das fluoreszierende Protein ausgewählt wird aus dem grün fluoreszierenden Protein GFP oder EGFP, dem cyan fluoreszierenden Protein CFP oder ECFP, dem gelb fluoreszierenden Protein YFP oder EYFP oder GFPUV.

3. Verwendung eines fluoreszierenden Proteins (Nr. 1) gemäß einem der Ansprüche 1 oder 2, bei welcher der Ligand markiert ist
* entweder durch eine fluoreszierende Substanz, wobei die Markierung bewirkt ist:
- entweder auf chemischem Weg, wobei die Substanz also eine chemische Verbindung ist,
- oder auf rekombinantem Weg, wobei die fluoreszierende Substanz also ist: ein Peptid oder ein fluoreszierendes Protein (Nr. 2) ausgewählt aus:
dem grün fluoreszierenden Protein GFP oder
- den durch Hinzufügen, Deletion oder Substitution einer oder mehrerer Aminosäuren von dem GFP stammenden Varianten, unter der Bedingung, dass diese Varianten die fluoreszierende Eigenschaft bewahren,
- oder Fragmenten des GFP oder Fragmenten der genannten Varianten unter der Bedingung, dass diese Fragmente die fluoreszierende Eigenschaft bewahren,
* oder durch eine nicht fluoreszierende Substanz, die zur Gruppe der violetten Säuren, roten Säuren, Alizarine, des Aluminon, des Azocarmin B, des basischen Fuscin, des Bordeaux R, des Carmins gehört.

4. Verwendung eines fluoreszierenden Proteins gemäß einem der Anprüche 1 bis 3, bei welcher der Membranrezeptor und der Ligand auf genetischem Weg markiert sind, wobei das fluoreszierende Protein und die fluoreszierende Substanz jeweils aus den folgenden Verbindungspaaren ausgewählt sind:
GFPUV - EYFP
EYFP - GFPUV
ECFP - EYFP
EYFP - ECFP
ECFP - EGFP
EGFP - ECFP
EGFP - EYFP
EYFP - EGFP

5. Verwendung gemäß Anspruch 4, bei welcher der Membranrezeptor durch das Protein EYFP oder EGFP markiert ist, und der Ligand durch das Protein ECFP markiert ist, oder der Membranrezeptor durch das Protein ECFP markiert ist, und der Ligand durch das Protein EYFP oder EGFP markiert ist.

6. Verwendung gemäß Anspruch 1 oder 2, eines fluoreszierenden Proteins ausgewählt aus:
- dem grün fluoreszierenden Protein GFP oder
- den durch Hinzufügung, Deletion oder Substitution von einer oder mehreren Aminosäuren von dem GFP stammenden Varianten unter der Bedingung, dass diese Varianten die fluoreszierende Eigenschaft bewahren,
- oder Fragmenten des GFP oder Fragmenten der genannten Varianten, unter der Bedingung, dass diese Fragmente die fluoreszierende Eigenschaft bewahren,
für das Auffinden und die Quantifizierung von nichtkovalenten Wechselwirkungen zwischen einem Membranrezeptor, der auf genetischem Weg durch das GFP oder eine der oben definierten Varianten oder eines der oben definierten Fragmente markiert ist und einem seiner Liganden, der durch eine fluoreszierende Substanz markiert ist, wobei dieses Auffinden und diese Quantifizierung durch Übertragung von Fluoreszenzenergie zwischen dem GFP oder einer der oben definierten Varianten oder eines der oben definierten Fragmenten und der oben genannten fluoreszierenden Substanzen stattfindet, wobei die fluoreszierende Substanz so ist, dass sie bei der Emissionswellenlänge des GFP oder einer der genannten Varianten oder eines der genannten Fragmente anregbar ist, oder das sie bei der Anregungswellenlänge des GFP oder einer der genannten Varianten, oder eines der genannten Fragmente emittiert.

7. Verwendung gemäß einem der Ansprüche 1 bis 6, bei der das fluoreszierende Protein ist:
• entweder EGFP und bei welcher:
- entweder das EGFP der Geber für Fluoreszenzenergie ist und der Marker, der das durch das EGFP emittierte Licht absorbiert, eine fluoreszierende Substanz ist oder nicht, und der Marker ausgewählt ist aus den Substanzen, deren Anregungsspektrum mit dem Emissionsspektrum des EGFP überlappt,
und in dem Fall, wo der Marker eine fluoreszierende Substanz ist, ist er ausgewählt aus: dem 4,4-Difluor-4-bora-3a,4a-diaza-s-indacen (Bodipy), dem Eosin, dem Erythrosin, dem Tetramethylrhodamin, dem Sulforhodamin 101 vertrieben durch Molekular Probe unter der Bezeichnung Texas Red, und ihren Derivaten, was einerseits das Aufpfropfen erlaubt und andererseits deren Anregungsspektrum das Emissionsspektrum des EGFP überdeckt,
und in dem Fall wo der Marker keine fluoreszierende Substanz ist, ist er ausgewählt aus der Gruppe der violetten Säuren, roten Säuren, Alizarinen, des Aluminon, des Azocarmin B, des basischen Fuscin, des Bordeaux R, des Carmin,
- oder das EGFP ist der Empfänger der Fluoreszenzenergie und die fluoreszierende Substanz ist der Geber der Fluoreszenzenergie und ist ausgewählt aus den Substanzen, deren Emissionsspektrum das Anregungsspektrum des EGFP überlappt, wobei diese Substanzen ausgewählt sind aus: den Cumarinen, dem Fluorescamin, dem 6-(N-Methylanilin)naphtalen, (Mansyl) und ihren Derivaten, was einerseits das Aufpfropfen erlaubt und anderseits deren Emissionsspektrum das Anregungsspektrum des EGFP überdeckt,
• oder ECFP und bei welcher:
- entweder das ECFP Geber der Fluoreszenzenergie ist und die fluoreszierende Substanz Empfänger der Energie ist, und ausgewählt ist aus dem Fluoreszein und dem 7-Nitrobenz-2-oxa-1,3-diazol,
- oder das ECFP ist Empfänger der Fluoreszenzenergie und die fluoreszierende Substanz ist Geber der Energie und ist ausgewählt aus dem Pyren oder dem Cumarin oder ihren Derivaten, was einerseits das Aufpfropfen erlaubt und andererseits deren Emissionsspektrum das Anregungsspektrum des ECFP überlappt.

8. Verwendung gemäß einem der Ansprüche 1 bis 7, bei welcher der Membranrezeptor ausgewählt ist aus:
- den Membranrezeptoren, die an das Protein G gekoppelt sind,
- den Rezeptoren der Wachstumsfaktoren und
- den Kanalrezeptoren

9. Verwendung gemäß einem der Ansprüche 1 bis 8, bei welcher der Membranrezeptor ausgewählt ist aus den Membranrezeptoren, die an das Protein G gekoppelt sind.

10. Verfahren zum Auffinden und zur Quantifizierung von nichtkovalenten Wechselwirkungen zwischen einem Membranrezeptor und einem seiner Liganden, **dadurch gekennzeichnet:**
- **dass** man Zellen oder Fragmente von Zellen herstellt, die eine DNA-Sequenz exprimieren, welche das Gen enthält, das für ein fluoreszierendes Protein kodiert, das mit dem Gen des Membranrezeptors fusioniert ist, wobei die Fusion zwischen dem Gen des fluoreszierenden Proteins und dem Gen des genannten Membranrezeptors derart ist, dass die Eigenschaften des Membranrezeptors nicht durch die Anwesenheit des fluoreszierenden Proteins geändert werden, nämlich:
* die Wechselwirkung zwischen dem Membranrezeptor und dem Liganden wird nicht geändert.
* die Funktion der Transduktion der Antwort wird nicht geändert, wobei das fluoreszierende Protein ausgewählt ist aus fluoreszierenden Proteinen, die von autofluoreszierenden Proteinen von Nesseltieren abgeleitet sind oder stammen, deren molarer Extinktionskoeffizient höher als 14.000 M⁻¹ cm⁻¹ und deren Fluoreszenzquantenausbeute größer als 0,38 ist,
- **dass** man die genannten Zellen oder die genannten Fragmente von Zellen in die Gegenwart eines Liganden des genannten Membranrezeptors bringt, der durch einen
- Marker markiert ist, der gebildet wird:
- entweder durch eine Molekül, das fähig ist, das durch das fluoreszierende Protein emittierte Licht zu absorbieren,
- oder durch eine fluoreszierende Substanz,
wobei entweder das fluoreszierende Protein der Geber der Fluoreszenzenergie ist, und der Marker ist der Empfänger der Fluoreszenzenergie, oder das fluoreszierende Protein ist der Empfänger der Fluoreszenzenergie und der Marker ist eine fluoreszierende Substanz als Geber der Fluoreszenzenergie, und
- **dass** man mit einer Wellenlänge, die es erlaubt das fluoreszierende Protein anzuregen oder die fluoreszierende Substanz anzuregen, bestrahlt,
- wobei die genannten Schritte des in Gegenwart Bringens und der Bestrahlung gleichzeitig oder einer nach dem anderen durchgeführt werden können, oder
- **dass** die genannten Zellen oder die genannten Fragmente von Zellen in die Gegenwart eines Liganden des genannten Proteins bringt, welcher durch einen Marker markiert ist, wobei die Zellen oder der Ligand vor ihrem in Gegenwart Bringen bestrahlt wurden,
- **dass** man entweder eine Verringerung der Emissionsamplitude des Gebers und/oder ein Emissionssignal, das für die Emission des Empfängers charakteristisch ist, detektiert.

11. Verfahren gemäß Anspruch 10, **dadurch gekennzeichnet, dass** das fluoreszierende Protein ausgewählt ist, aus dem grün fluoreszierenden Protein GFP oder EGFP, dem cyan fluoreszierenden Protein CFP oder ECFP, dem gelb fluoreszierenden Protein YFP oder EYFP oder GFPUV.

12. Verfahren zur Auffindung und Quantifizierung von nichtkovalenten Wechselwirkungen zwischen einem Membranrezeptor und einem seiner Liganden, **dadurch gekennzeichnet**
- **dass** man ein fluoreszierendes Protein herstellt, das mit einem Membranrezeptor fusioniert ist, von dem man die Wechselwirkung Rezeptor-Ligand bestimmen will, wobei die Fusion zwischen dem fluoreszierenden Protein und dem genannten Membranrezeptor so ist, dass die Eigenschaften des Rezeptors nicht durch die Anwesenheit des fluoreszierenden Proteins geändert werden, nämlich:
* die Wechselwirkung zwischen dem Membranrezeptor und dem Liganden wird nicht geändert,
* die Funktion der Transduktion der Antwort wird nicht geändert,
wobei das fluoreszierende Protein wie in den Ansprüchen 10 oder 11 definiert ist, und
- **dass** man das genannte fluoreszierende Protein, das mit dem Membranrezeptor fusioniert ist, in Gegenwart eines Liganden des genannten Rezeptors bringt, wobei dieser Ligand durch einen Marker markiert ist, der gebildet wird aus:
- entweder einem Molekül, das fähig ist, das von dem fluoreszierenden Protein emittierte Licht zu absorbieren,
- oder einer fluoreszierenden Substanz,
und wobei entweder das fluoreszierende Protein ein Geber der Fluoreszenzenergie und der Marker ein Empfänger der Fluoreszenzenergie ist, oder das fluoreszierende Protein ein Empfänger der Fluoreszenzenergie und der Marker eine fluoreszierende Substanz als Geber der Fluoreszenzenergie ist, und
- **dass** man mit einer Wellenlänge, die es erlaubt, das fluoreszierende Protein anzuregen oder die fluoreszierende Substanz anzuregen, bestrahlt,
- wobei die genannten Schritte des in Gegenwart Bringens und der Bestrahlung gleichzeitig, oder einer nach dem anderen durchgeführt werden können oder
- **dass** man das genannte fluoreszierende Protein, das mit dem Membranrezeptor fusioniert ist, in Gegenwart eines Liganden des genannten Rezeptors bringt, wobei dieser Ligand durch einen Marker markiert ist, der gebildet wird aus:
- entweder einem Molekül, das fähig ist, das durch das fluoreszierende Protein emittierte Licht zu absorbieren,
- oder einer fluoreszierenden Substanz,
wobei das fluoreszierende Protein, das mit dem Membranrezeptor fusioniert ist oder der Ligand vor ihrem in Gegenwart Bringen, bestrahlt wurden,
- **dass** man entweder eine Verringerung der Emissionsamplitude des Gebers und/oder ein Emissionssignal, das für die Emission des Empfängers charakteristisch ist, detektiert.

13. Verfahren gemäß einem der Ansprüche 10 bis 12, bei welchem man einen zusätzlichen Schritt einführt:
- vor oder nach oder gleichzeitig mit dem Schritt des in Gegenwart Bringens des fluoreszierenden Proteins, das mit dem Membranrezeptor fusioniert ist, mit einem Liganden des genannten Membranrezeptors, wobei dieser Ligand durch einen Marker markiert ist oder
- vor oder nach oder gleichzeitig mit dem Schritt des in Gegenwarts Bringens der Zellen oder der Fragmente von Zellen mit einem Liganden des genannten Membranrezeptors, der durch einen Marker markiert ist,
wobei dieser zusätzliche Schritt besteht aus:
- entweder einem in Gegenwart Bringen des genannten fluoreszierenden Proteins, das mit dem Membranrezeptor fusioniert ist, mit dem genannten nicht markierten Liganden und gleichzeitig mit dem genannten markierten Liganden,
- oder durch in Gegenwart Bringen der genannten Zellen oder der genannten Fragmente von Zellen gleichzeitig mit dem nicht markierten Liganden und dem markierten Liganden,
- wobei man entweder eine Verringerung der. Emissionsamplitude des Gebers und/oder ein Emissionssignal das charakteristisch für die Emission des Empfängers ist, jeweils für den Fall der Verwendung eines markierten Liganden, und für den Fall der gleichzeitigen Verwendung eines markierten Liganden und eines nicht markierten Liganden, detektiert,
- und man entweder die jeweils erhaltenen Verringerungen der Emissionsamplitude des Gebers und/oder die jeweils erhaltenen Emissionssignale, die für die Emission des Empfängers charakteristisch sind, vergleicht.

14. Verfahren gemäß Anspruch 13, bei welchem das fluoreszierende Protein EGFP ist und bei welchem:
- entweder das EGFP Geber der Fluoreszenzenergie ist, und der Marker Empfänger der Fluoreszenzenergie ist, und ausgewählt ist aus den Substanzen, deren Anregungsspektrum mit dem Emissionsspektrum des EGFP überlappt,
und in dem Fall wo der Marker eine fluoreszierende Substanz ist, ist er ausgewählt aus: dem 4,4-Difluor-4-bora-3a,4a-diaza-s-indacen (Bodipy), dem Eosin, dem Erythrosin, dem Tetramethylrhodamin, dem Sulforhodamine 101, vertrieben durch Molekular Probe unter der Bezeichnung Texas Red, und ihren Derivate, wodurch einerseits ein Aufpfropfen erlaubt wird und anderseits deren Anregungsspektrum das Emissionsspektrum des EGFP überdeckt,
und in dem Fall wo der Marker keine fluoreszierende Substanz ist, ist er aus der Gruppe der violetten Säuren, roten Säuren, der Alizarine, des Aluminon, des Azocarmin B, des basischen Fuscin, dem Bordeaux R und dem Carmin, ausgewählt,
- oder das EGFP ist Empfänger der Fluoreszenzenergie und die Fluoreszenzsubstanz ist Geber der Fluoreszenzenergie und ist ausgewählt aus den Substanzen, deren Emissionsspektrum das Anregungsspektrum des EGFP überlappt, wobei die Substanzen ausgewählt sind aus: den Coumarinen, dem Fluorescamin, dem 6-(N-Methylanilin)naphtalen, (Mansyl) und ihren Derivaten, wodurch einerseits das Aufpfropfen erlaubt wird und anderseits deren Emissionsspektrum das Anregungsspektrum des EGFP überdeckt.

15. Verfahren gemäß einem der Ansprüche 10 bis 12, bei dem der Membranrezeptor, dessen Rezeptor-Ligand-Wechselwirkung bestimmt werden soll, ausgewählt ist aus:
- den Membranrezeptoren, die an das Protein G gekoppelt sind,
- den Rezeptoren der Wachstumsfaktoren und
- den Kanalrezeptoren.

16. Verfahren gemäß Anspruch 15, **dadurch gekennzeichnet, dass** die Rezeptoren der Wachstumsfaktoren ausgewählt sind aus jenen, die strukturell mit dem Rezeptor des Insulins oder dem Rezeptor des Interferons γ verbunden sind.

17. Verfahren gemäß einem der Ansprüche 10 bis 16, bei dem das fluoreszierende Protein EGFP ist und der Marker Bodipy ist, und bei welchem man entweder die Verringerung der Emissionsamplitude des EGFP oder das Emissionssignal des Bodipy, das aus der Energieübertragung resultiert, detektiert, wobei die Bestrahlungswellenlänge der Anregungswellenlänge des EGFP entspricht.

18. Verfahren gemäß einem der Ansprüche 10 bis 17, bei welchem das fluoreszierende Protein das EGFP ist und der Marker ein Coumarin ist, und bei welchem man entweder die Verringerung der Amplitude des Coumarin oder das Emissionssignal des EGFP, das aus der Energieübertragung resultiert, detektiert, wobei die Wellenlänge der Bestrahlung der Wellenlänge der Anregung des Coumarin entspricht.

19. Verfahren gemäß einem der Ansprüche 10 bis 18, bei welchem das fluoreszierende Protein mit der N-terminalen Seite fusioniert ist und der Membranrezeptor mit der C-terminalen Seite fusioniert ist.

20. Verfahren gemäß einem der Ansprüche 10 bis 19, bei welchem das fluoreszierende Protein mit der C-terminalen Seite fusioniert ist und der Membranrezeptor mit der N-terminalen Seite fusioniert ist.

21. Verfahren gemäß einem der Ansprüche 10 bis 20, bei welchem das fluoreszierende Protein an einer Stelle im Membranrezeptor eingefügt ist, die nicht einer Bindungsstelle Membranrezeptor-Ligand entspricht, im Falle von Rezeptoren die an das Protein G gekoppelt sind, wobei diese Einfügung im ersten oder im dritten intrazellulären Loop des Rezeptors stattfindet, mit der Einschränkung, dass die Einfügung weder die Eigenschaften des Rezeptors noch die Fluoreszenz des fluoreszierenden Proteins zerstört.

22. Verfahren gemäß einem der Ansprüche 10 bis 21, bei welchem die Zellen Zellen von Säugetieren sind, ausgewählt aus adherenten oder in Suspension befindlichen HEK 293-Zellen, CHO-Zellen, COS-Zellen, Lymphozytenstämme, Fibroblasten oder Hefe-Zellen, ausgewählt unter den Hefen *Pichia, Saccharomyces, Hansenula* oder durch ein Virus infizierte Insektenzellen, wobei die Zellen ausgewählt sind aus TNI- oder sf9-Zellen, oder von Pilzen ausgewählt unter den Stämmen von *Aspergillus, Neurospora, Fusarium, Trichoderma.*

23. Verfahren gemäß irgendeinem der Ansprüche 10 bis 22, bei welchem in einem herkömmlichen Fluorimetriegerät oder in einem Gerät zur raschen Mischung mit einem Fluoreszenzdetektionssystem, nach dem Mischen des Gebers mit dem Empfänger ein Signal detektierbar ist und durch Zugabe einer nicht fluoreszierenden Substanz von gleicher pharmakologischer Spezifität aufgehoben werden kann, und bei dem das Verhältnis Signal/Rauschen höher als ungefähr 2 ist.

24. Verwendung gemäß einem der Ansprüche 1 bis 3, für die Auffindung und die Quantifizierung von nichtkovalenten Wechselwirkungen zwischen einem Membranrezeptor, der durch einen an G-Proteine gekoppelten Rezeptor gebildet wird, und einem G-Protein um die gegenüber dem Rezeptor biologisch, aktiven Moleküle zu identifizieren, die fähig sind eine nichtkovalente reversible Wechselwirkung mit dem Rezeptor zu bilden, wobei dieser Rezeptor auf genetischem Weg mit dem fluoreszierenden Protein markiert ist, wie es in einem der Ansprüche 1 bis 3 definiert ist, und das G-Protein durch einen Marker markiert ist, wie es in einem der Ansprüche 1 bis 3 definiert ist.

25. Verwendung gemäß Anspruch 24, bei der der Rezeptor ausgewählt wird aus:
- den an G-Proteine gekoppelten Rezeptoren,
- den Sequenzen, die für Rezeptoren kodieren, die an vermeintliche G-Proteine gekoppelt sind, deren biologisch gegenüber denselben aktive Moleküle identifiziert werden.

26. Verfahren gemäß irgendeinem der Ansprüche 10 bis 23, **dadurch gekennzeichnet, dass** der Membranrezeptor ein Rezeptor ist, der an G-Proteine gekoppelt ist, und dass der Ligand ein G-Protein ist, um die biologisch gegenüber dem Rezeptor aktiven Moleküle zu identifizieren, die fähig sind eine reversible, nichtkovalente Wechselwirkung mit dem Rezeptor zu bilden, und **dadurch gekennzeichnet, dass** die Wechselwirkung zwischen dem Rezeptor und dem biologisch aktiven Molekül nicht verändert wird.

27. Verfahren zur Identifizierung und eventuell Quantifizierung von Wechselwirkungen, zwischen einem Membranrezeptor und einem nicht fluoreszierenden Molekül, das gegenüber dem Rezeptor biologisch aktiv ist, welche fähig sind eine reversible, nichtkovalente Wechselwirkung mit dem Rezeptor zu bilden, durch Ausführung des Verfahrens gemäß Anspruch 26, bei dem ein biologisch aktives, nicht fluoreszierendes Molekül zu den Zellen oder den Fragmenten von Zellen zugegeben wird, wobei die DNA exprimiert wird, die für den durch das fluoreszierende Protein markierten Rezeptor und für das mit dem Marker markierte G-Protein kodiert, **dadurch gekennzeichnet dass**,
- ein biologisch aktives und agonistisches, nicht fluoreszierendes Molekül eine Transduktion des detektierten Signals durch Variation der Energieübertragung zwischen dem durch das fluoreszierende Protein markierten Rezeptor und dem durch den Marker markierten G-Protein auslöst;
- und ein, antagonistisches biologisch aktives, nicht fluoreszierendes Molekül die Transduktion eines Signals, das durch einen Agonisten hervorgerufen wurde und durch die Variation der Fluoreszenzenergieübertragung zwischen dem mit dem fluoreszierenden Protein markierten Rezeptor und dem mit dem Marker markierten G-Protein detektiert wurde, hemmt.

28. Zellen oder Fragmenten von Zellen, die eine DNA-Sequenz enthalten, die das Gen aufweist, das für eine fluoreszierende Substanz kodiert, die mit dem Gen eines Liganden fusioniert ist, der durch das G-Protein gebildet wird, wobei die fluoreszierende Substanz ausgewählt wird aus den fluoreszierenden Proteinen, die von autofluoreszierenden Proteinen von Nesseltieren abgeleitet sind oder stammen, deren molekularer Extinktionskoeffizient höher als 14.000 M⁻¹cm⁻¹ und deren Fluoreszenzquantenausbeute größer als 0,38 liegt,
- und enthaltend das Gen, das für ein fluoreszierendes Protein kodiert, welches mit dem Gen eines Membranrezeptors fusioniert ist, wobei das fluoreszierende Protein ausgewählt ist, aus den fluoreszierenden Proteinen, die von autofluoreszierenden Proteinen von Nesseltieren abgeleitet sind oder stammen,
- wobei die Fusion zwischen dem Gen der fluoreszierenden Substanz und dem Gen des vorgenannten G-Proteins, und die Fusion zwischen dem Gen des fluoreszierenden Proteins und dem Gen des Rezeptors derart ist, dass
* die Eigenschaften des Rezeptors durch die Anwesenheit des fluoreszierenden Proteins nicht geändert werden, nämlich
* die Funktion der Transduktion der Antwort wird nicht geändert, unter der Bedingung dass:
* wenn der Membranrezeptor der Rezeptor von Glucocorticoiden von Ratten ist, fusioniert am N-Terminal mit aufeinanderfolgend einer Reinigungssequenz mit 6 Histidinen, einem Hemaglutinin-Epitop und einem fluoreszierenden Protein und in der Zellenlinie 1471.1 exprimiert wird, das fluoreszierende Protein sich von dem GFP unterscheidet (768 Basenpaare des Plasmids TU65 mit der Mutation S65T),
* wenn der Membranrezeptor der humane Rezeptor der Glucocorticoide ist, der an seinen 131 ersten Aminosäuren trunkiert ist, fusioniert am C-Terminal mit einem fluoreszierenden Protein an seinen Stellen Sal I und BamH I und in den Cos-1-Zellen exprimiert wird, ist das fluoreszierende Protein verschieden von dem GFP der Sequenz Met Ser Lys Gly Glu Glu Leu Phe Thr Gly Val Val Pro Ile Leu Val Glu Leu Asp Gly Asp Val Asn Gly Gln Lys Phe Ser Val Ser Gly Glu Gly Glu Gly Asp Ala Thr Tyr Gly Lys Leu Thr Leu Lys Phe Ile Cys Thr Thr Gly Lys Leu Pro Val Pro Trp Pro Thr Leu Val Thr Thr Phe Ser Tyr Gly Val Gln Cys Phe Ser Arg Tyr Pro Asp His Met Lys Gln His Asp Phe Phe Lys Ser Ala Met Pro Glu Gly Tyr Val Gln Glu Arg Thr Ile Phe Phe Lys Asp Asp Gly Asn Tyr Lys Thr Arg Ala Glu Val Lys Phe Glu Gly Asp Thr Leu Val Asn Arg Ile Glu Leu Lys Gly Ile Asp Phe Lys Glu Asp Gly Asn Ile Leu Gly His Lys Met Glu Tyr Asn Tyr Asn Ser His Asn Val Tyr Ile Met Ala Asp Lys Pro Lys Asn Gly Ile Lys Val Asn Phe Lys Ile Arg His Asn Ile Lys Asp Gly Ser Val Gln Leu Ala Asp His Tyr Gln Gln Asn Thr Pro Ile Gly Asp Gly Pro Val Leu Leu Pro Asp Asn His Tyr Leu Ser Thr Gln Ser Ala Leu Ser Lys Asp Pro Asn Glu Lys Arg Asp His Met Ile Leu Leu Glu Phe Val Thr Ala Ala Gly Ile Thr His Gly Met Asp Glu Leu Tyr Lys,
* wenn der Membranrezeptor die Untereinheit NMDA R1 von Ratten exprimiert in den HEK 293 Zellen ist, fusioniert am C-Terminus mit einem fluoreszierenden Protein, ist das fluoreszierende Protein von jenem verschieden, das durch die Aminosäuren 2-238 des Wildtype GFP gebildet wird,
* wenn der Membranrezeptor ein Rezeptor oder einen Fragment eines Rezeptors von intrazellulären Sekundärmessengers ist, ist das fluoreszierende Protein verschieden von dem GFP und seinen Derivaten.

29. Kit für das Auffinden und die Quantifizierung von nichtkovalenten Wechselwirkungen zwischen einem Membranrezeptor, der durch ein fluoreszierendes Protein markiert ist, wie es in den Ansprüchen 1 bis 3 definiert ist, und einem seiner Liganden, der durch einen Marker markiert ist, der gebildet ist aus:
- entweder einem Molekül, das fähig ist, das durch das fluoreszierende Protein emittierte Licht zu absorbieren,
- oder durch eine fluoreszierende Substanz,
wobei der Kit enthält:
- den Membranrezeptor fusioniert mit einem fluoreszierenden Protein, wie es oben definiert ist, oder eine stabile Zelllinie, die fähig ist den Membranrezeptor, der mit einem fluoreszierenden Protein fusioniert ist, wie es oben definiert ist, zu exprimieren oder ein Plasmid, das die Nukleotidsequenz enthält, die für den Membranrezeptor, der mit einem fluoreszierenden Protein, wie es oben definiert ist, fusioniert ist, kodiert,
- den durch den genannten Marker markierten Liganden,
- die für den Energieübergang zwischen dem genannten Protein und dem genannten Liganden, notwendigen Puffer und Milieus.

30. Kit gemäß Anspruch 29, **dadurch gekennzeichnet, dass** der Membranrezeptor durch ein fluoreszierendes Protein (Nr.1) markiert ist und der eine seiner Liganden durch eine fluoreszierende Substanz markiert ist, die einem zweiten fluoreszierenden Protein (Nr. 2) entspricht, wobei das erste fluoreszierende Protein (Nr. 1) ausgewählt ist, aus dem fluoreszierenden Protein EYFP oder EGFP und der Ligand durch das fluoreszierende Protein (Nr. 2) ECFP markiert ist, oder wobei das fluoreszierende Protein (Nr.1) ECFP ist und der Ligand durch das fluoreszierende Protein (Nr. 2) EYFP und EGFP markiert ist, wobei der Kit enthält:
- entweder ein Plasmid, das eine Nukleinsäure enthält, die für den Membranrezeptor fusioniert mit einem fluoreszierenden Protein (Nr.1) kodiert, und
_{*} ein Plasmid, das eine Nukleinsäure enthält, die für den Liganden fusioniert mit einem fluoreszierenden Protein (Nr. 2) kodiert, oder
* einen Liganden, der mit einem fluoreszierenden Protein (Nr. 2) fusioniert, der auf rekombinanten Weg erhalten wurde, und gereinigt wurde,
- oder eine stabile Zelllinie, die fähig ist den Membranrezeptor, der mit einem fluoreszierenden Protein (Nr. 1) fusioniert ist, zu exprimieren und
* eine stabile Zelllinie die fähig ist den Liganden, der mit einem fluoreszierenden Protein (Nr. 2) fusioniert ist zu exprimieren oder
* einen Liganden, der mit einem fluoreszierenden Protein (Nr. 2) fusioniert ist, erhalten auf rekombinanten Weg und gereinigt.

31. Kit gemäß Anspruch 30, **dadurch gekennzeichnet, dass** der Membranrezeptor durch einen Rezeptor gebildet wird, der an das G-Protein gekoppelt ist, und das der Ligand dem G-Protein entspricht.

## Claims

1. Use of a fluorescent protein chosen from fluorescent proteins obtained or derived from autofluorescent proteins of cnidarians, the molecular extinction coefficient of which is greater than about 14,000 M⁻¹cm⁻¹ and the quantic fluorescent yield of which is greater than about 0.38,
for the detection and quantification of non-covalent interactions on cells or cell fragments between a membrane-bound receptor labeled genetically with the fluorescent protein as defined above and one of its ligands labeled with a label consisting:
- either of a molecule which is capable of absorbing the light emitted by the fluorescent protein,
- or of a fluorescent substance,
this detection and quantification taking place by fluorescence energy transfer:
. between the fluorescent protein as mentioned above, and the above-mentioned fluorescent substance, the fluorescent substance being such that either it is excitable at the emission wavelength of the above-mentioned fluorescent protein, or it emits at the excitation wavelength of the above-mentioned fluorescent protein, or
. between the above-mentioned fluorescent protein, and the above-mentioned molecule which is capable of absorbing the light emitted by the fluorescent protein.

2. Use according to claim 1, **characterized in that** the fluorescent protein is chosen from green fluorescent protein GFP or EGFP, cyan fluorescent protein CFP or ECFP, yellow fluorescent protein YFP or EYFP, or GFPUV.

3. Use of a fluorescent protein (No 1) according to claim 1 or 2, in which the ligand is labeled
* either with a fluorescent substance, the labeling being carried out:
- either via a chemical route, the fluorescent substance then being a chemical compound,
- or via a recombinant route, the fluorescent substance then being a fluorescent peptide or protein (No 2) which can be chosen in particular from:
- green fluorescent protein GFP, or
- variants derived from GFP by addition, deletion or substitution of one or more amino acids, with the proviso that these variants conserve the fluorescence property,
- or fragments of GFP, or fragments of the above-mentioned variants, with the proviso that these fragments conserve the fluorescence property,
* or with a non-fluorescent substance belonging to the Acid Violet group, the Acid Red group, alizarins, aluminon, azocarmine B, basic fuschin, Bordeaux R, and Carmine.

4. Use of a fluorescent protein according to one of claims 1 to 3, in which the membrane-bound receptor and the ligand are labeled genetically, the fluorescent protein and the fluorescent substance being chosen, respectively, from the following compound pairs:
GFPUV - EYFP
EYFP - GFPUV
ECFP - EYFP
EYFP - ECFP
ECFP - EGFP
EGFP - ECFP
EGFP - EYFP
EYFP - EGFP

5. Use according to claim 4, wherein the membrane-bound receptor is labeled with the EYFP or EGFP protein and the ligand is labeled with the ECFP protein, or the membrane-bound receptor is labeled with the ECFP protein and the ligand is labeled with the EYFP or EGFP protein.

6. Use according to claim 1 or 2, of a fluorescent protein chosen from:
- green fluorescent protein GFP, or
- variants derived from GFP by addition, deletion or substitution of one or more amino acids, with the proviso that these variants conserve the fluorescence property,
- or fragments of GFP, or fragments of the above-mentioned variants, with the proviso that these fragments conserve the fluorescence property,
for the detection and quantification of non-covalent interactions between a membrane-bound receptor labeled genetically with GFP or one of the variants defined above or one of the fragments defined above and one of its ligands labeled with a fluorescent substance, this detection and quantification taking place by fluorescence energy transfer between GFP or one of the variants defined above, or one of the fragments defined above, and the said fluorescent substance, the fluorescent substance being such that either it is excitable at the emission wavelength of GFP or of one of the above-mentioned variants, or of one of the above-mentioned fragments, or it emits at the excitation wavelength of GFP, or of one of the above-mentioned variants, or of one of the above-mentioned fragments.

7. Use according to one of claims 1 to 6, in which the fluorescent protein is:
• either EGFP and wherein:
- either the EGFP is a fluorescence energy donor and the label absorbing the light emitted by the EGFP is a fluorescent or non-fluorescent substance, and the marker being chosen from substances whose excitation spectrum overlaps the emission spectrum of EGFP,
and when the label is a fluorescent substance, it is chosen from: 4,4-difluoro-4-bora-3a,4a-diaza-s-indacene (Bodipy), eosin, erythrosin, tetramethylrhodamine, sulphorhodamine 101 sold by Molecular Probe under the name Texas Red, and derivatives thereof which, on the one hand, allow grafting, and, on the other hand, have an excitation spectrum which overlaps the emission spectrum of EGFP,
and, when the label is not a fluorescent substance, it is chosen from the Acid Violet group, the Acid Red group, alizarins, aluminon, azocarmine B, basic fuschin, Bordeaux R, and Carmine,
- or the EGFP is a fluorescence energy acceptor and the fluorescent substance is a fluorescence energy donor and is chosen from substances whose emission spectrum overlaps the excitation spectrum of EGFP, said substances being chosen from: coumarins, fluorescamine, 6-(N-methylanilino)naphthalene, (mansyl) and derivatives thereof which, on the one hand, allow grafting, and, on the other hand, have an excitation spectrum which overlaps the emission spectrum of EGFP,
• or ECFP and wherein:
- either ECFP is a fluorescence energy donor and the fluorescent substance is an energy acceptor and is chosen from fluorescein and 7-nitro-2-benzoxa-1,3-diazole,
- or ECFP is a fluorescence energy acceptor and the fluorescent substance is an energy donor and is chosen from pyrene and coumarin or derivatives thereof which, on the one hand, allow grafting, and, on the other hand, have an excitation spectrum which overlaps the emission spectrum of ECFP.

8. Use according to one of Claims 1 to 7, in which the membrane-bound receptor is chosen from:
- membrane-bound receptors coupled to protein G,
- growth factor receptors,
- ion channel receptors.

9. Use according to one of claims 1 to 8, in which the membrane-bound receptor is chosen from membrane-bound receptors coupled to the G protein.

10. Process for detecting and quantifying non-covalent interactions between a membrane-bound receptor, and one of its ligands, **characterized in that**:
- cells or cell fragments are prepared containing a DNA sequence comprising the gene coding for a fluorescent protein fused with the gene for the membrane-bound receptor, the fusion between the gene for the fluorescent protein and the gene for the above-mentioned membrane-bound receptor being such that the properties of the membrane-bound receptor, are not modified by the presence of the fluorescent protein, namely:
* the interaction between the membrane-bound receptor, and the ligand is not modified,
* the response transduction function is not modified, the fluorescent protein being chosen from the fluorescent proteins obtained or derived from autofluorescent proteins of cnidarians, the molar extinction coefficient of which is greater than about 14,000 M-1cm-1 and the quantic fluorescence yield is greater than about 0.38,
- the above-mentioned cells or the above-mentioned cell fragments are placed in contact with a ligand for the above-mentioned membrane-bound receptor labeled with a label consisting:
- either of a molecule capable of absorbing the light emitted by the fluorescent protein,
- or of a fluorescent substance,
and either the fluorescent protein being the fluorescence energy donor and the label being the fluorescence energy acceptor, or the fluorescent protein being the fluorescence energy acceptor and the label being a fluorescent substance which is a fluorescence energy donor, and
- irradiation is carried out at a wavelength which makes it possible either to excite the fluorescent protein or to excite the fluorescent substance,
- it being possible for the above-mentioned steps of placing in contact and irradiation to be carried out either simultaneously or one after the other, or
- the above-mentioned cells or the above-mentioned cell fragments are placed in contact with a ligand for the above-mentioned membrane-bound receptor, labeled with a label, the cells or the ligand having been irradiated before being placed in contact,
- either a reduction in the amplitude of the donor's emission and/or emission signal characteristic of the acceptor's emission is detected.

11. Process according to claim 10, **characterized in that** the fluorescent protein is chosen from green fluorescent protein GFP or EGFP, cyan fluorescent protein CFP or ECFP, yellow fluorescent protein YFP or EYFP, or GFPUV.

12. Process for detecting and quantifying non-covalent interactions between a membrane-bound receptor, and one of its ligands, **characterized in that**:
- a fluorescent protein fused with a membrane-bound receptor, the receptor-ligand interaction of which it is desired to determine, is prepared, the fusion between the fluorescent protein and the above-mentioned membrane-bound receptor being such that the properties of the membrane-bound receptor are not modified by the presence of the fluorescent protein, namely:
* the interaction between the membrane-bound receptor, and the ligand is not modified,
* the response transduction function is not modified,
the fluorescent protein being as defined in claim 10 or 11, and
- the above-mentioned fluorescent protein fused with the membrane-bound receptor is placed in contact with a ligand for the above-mentioned membrane-bound receptor, this ligand being labeled with a label consisting:
- either of a molecule which is capable of absorbing the light emitted by the fluorescent protein,
- or of a fluorescent substance,
and either the fluorescent protein being a fluorescence energy donor and the label being a fluorescence energy acceptor, or the fluorescent protein being a fluorescence energy acceptor and the label being a fluorescent substance which is a fluorescence energy donor, and
- irradiation is carried out at a wavelength which makes it possible either to excite the fluorescent protein or to excite the fluorescent substance,
- it being possible for the above-mentioned steps of placing in contact and irradiation to be carried out either simultaneously or one after the other, or
- the above-mentioned fluorescent protein fused with the membrane-bound receptor is placed in contact with a ligand for the above-mentioned membrane-bound receptor, this ligand being labeled with a label consisting:
- either of a molecule which is capable of absorbing the light emitted by the fluorescent protein,
- or of a fluorescent substance,
the fluorescent protein fused with the membrane-bound receptor or the ligand having been irradiated before being placed in contact,
- either a reduction in the amplitude of the donor's emission and/or an emission signal characteristic of the acceptor's emission is detected.

13. Process according to any of claims 10 to 12, in which an additional step is introduced:
- before, after or simultaneously with the step for placing in contact of the fluorescent protein fused with the membrane-bound receptor and of a ligand for the above-mentioned membrane-bound receptor, this ligand being labeled with a label, or
- before, after or simultaneously with the step for placing in contact of cells or cell fragments and of a ligand for the above-mentioned membrane-bound receptor, labeled with a label,
this additional step consisting:
- either in placing the above-mentioned fluorescent protein fused with the membrane-bound receptor in contact with the above-mentioned non-labeled ligand and simultaneously with the above-mentioned labeled ligand,
- or in placing the above-mentioned cells or the above-mentioned cell fragments in simultaneous contact with the above-mentioned non-labeled ligand and the above-mentioned labeled ligand,
- either a decrease in the amplitude of the donor's emission and/or an emission signal characteristic of the acceptor's emission is detected, respectively, in the case of using the labeled ligand and in the case of simultaneously using the labeled ligand and the non-labeled ligand,
- and either the reductions in the amplitude of the donor's emission respectively obtained and/or the emission signals characteristic of the acceptor's emission respectively obtained are compared.

14. Process according to claim 13, in which the fluorescent protein is EGFP and in which:
- either the EGFP is a fluorescence energy donor and the label is a fluorescence energy acceptor and is chosen from substances whose excitation spectrum overlaps the emission spectrum of EGFP,
and, when the label is a fluorescent substance, it is chosen from: 4,4-difluoro-4-bora-3a,4a-diaza-s-indacene (Bodipy), eosin, erythrosin, tetramethylrhodamine, sulphorhodamine 101 sold by Molecular Probe under the name Texas Red, and derivatives thereof which, on the one hand, allow grafting, and, on the other hand, have an excitation spectrum which overlaps the emission spectrum of EGFP,
and, when the label is not a fluorescent substance, it is chosen from the Acid Violet group, the Acid Red group, alizarins, aluminon, azocarmine B, basic fuschin, Bordeaux R and Carmine,
- or the EGFP is a fluorescence energy acceptor and the fluorescent substance is a fluorescence energy donor and is chosen from substances whose emission spectrum overlaps the excitation spectrum of EGFP, said substances being chosen from: coumarins, fluorescamine, 6-(N-methylanilino)naphthalene, (mansyl) and derivatives thereof which, on the one hand, allow grafting, and, on the other hand, have an excitation spectrum which overlaps the emission spectrum of EGFP.

15. Process according to any of claims 10 to 12, in which the membrane-bound receptor whose receptor-ligand interaction it is desired to determine is chosen from:
- membrane-bound proteins coupled to the G protein,
- growth factor receptors,
- ion channel-receptors.

16. Process according to claim 15, **characterized in that** the growth factor receptors are chosen from those which are structurally linked to the insulin receptor or to the γ interferon receptor.

17. Process according to one of claims 10 to 16, in which the fluorescent protein is EGFP and the labeled substance is Bodipy and in which either the reduction in the emission amplitude of EGFP or the emission signal of Bodipy resulting from the energy transfer is detected, the irradiation wavelength corresponding to the excitation wavelength of EGFP.

18. Process according to one of Claims 10 to 17, in which the fluorescent protein is EGFP and the labeled substance is a coumarin, and in which either the diminution of amplitude of coumarin or the emission signal of EGFP resulting from the energy transfer is detected, the irradiation wavelength corresponding to the excitation wavelength of coumarin.

19. Process according to one of Claims 10 to 18, in which the fluorescent protein is fused on the N-terminal side and the membrane-bound receptor is fused on the C-terminal side.

20. Process according to one of Claims 10 to 19, in which the fluorescent protein is fused on the C-terminal side and the membrane-bound receptor is fused on the N-terminal side.

21. Process according to one of Claims 10 to 20, in which the fluorescent protein is inserted into the membrane-bound receptor in a place not corresponding to a membrane-bound receptor-ligand binding sites, in the case of receptors coupled to the G protein, this insertion taking place in the first or the third intracellular loop of the receptor, with the proviso that the insertion does not destroy either the properties of the receptor or the fluorescence of the fluorescent protein.

22. Process according to one of Claims 10 to 21, in which the cells are mammalian cells, chosen from: HEK 293 cells which are adherent or in suspension, CHO cells, COS cells, lymphocytic lines, fibroblasts, or yeast cells, chosen from: *pichia, saccharomyces, Hansenula*, or insect cells infected with a virus, said cells being chosen from TNI or sf9 cells, or fungi, chosen from strains of *Aspergillus, Neurospora, Fusarium* or *Trichoderma.*

23. Process according to any one of Claims 10 to 22, in which a signal can be detected, in a conventional fluorimetry device or in a rapid-mixing device equipped with a system for detecting fluorescence, after mixing the donor and the acceptor, and can be abolished by the addition of a non-fluorescent substance of the same pharmacological specificity, and in which the signal/noise ratio is greater than about 2.

24. Use according to one of claims 1 to 3, for detecting and quantifying non-covalent interactions between a membrane-bound receptor consisting of a receptor coupled to the G proteins and a G protein, in order to identify the molecules which are biologically active with respect to the receptor, and which are capable of forming a reversible non-covalent interaction with the said receptor, the said receptor being labeled genetically with a fluorescent protein as defined in one of claims 1 to 3, and the G protein being labeled with a label as defined in one of claims 1 to 3.

25. Use according to claim 24, in which the receptor is chosen from:
- the receptors coupled to the G proteins,
- the sequences coding for receptors coupled to the putative G proteins in which the molecules which are biologically active with respect to these receptors are to be identified.

26. Process according to any of claims 10 to 23, **characterized in that** membrane-bound receptor is a receptor coupled to the G proteins, and **in that** the ligand is a G protein, in order to identify the molecules which are biologically active with respect to the receptor, and which are capable of forming a reversible non-covalent interaction with the said receptor, **characterised in that** the interaction between the receptor and the biologically active molecule is not modified.

27. Process for identifying and possibly quantifying interactions between a membrane-bound receptor and a non-fluorescent molecule which is biologically active with respect to the said receptor, which are capable of forming a reversible non-covalent interaction with the said receptor, by implementing the process of claim 26, in which a biologically active non-fluorescent molecule is added to cells, or cell fragments, which express the DNA coding for the receptor labeled with the fluorescent protein and for the G protein labeled with the label, **characterized in that**:
- an agonist and biologically active non-fluorescent molecule triggers a signal transduction detected by variation in the energy transfer between the receptor labeled with the fluorescent protein and the G protein labeled with the label;
- an antagonistic, biologically active non-fluorescent molecule inhibits the signal transduction brought about by an agonist and detected by variation in the transfer of fluorescence energy between the receptor labeled with the fluorescent protein and the G protein labeled with the label.

28. Cells or cell fragments containing a DNA sequence comprising the gene coding for a fluorescent substance fused with the gene for a ligand constituted by the G protein, the fluorescent substance being chosen from the fluorescent proteins obtained or derived from autofluorescent proteins of cnidarians, the molecular extinction coefficient of which is greater than about 14,000 M⁻¹cm⁻¹ and the quantic fluorescence yield of which is greater than about 0.38,
- and comprising the gene coding for a fluorescent protein fused with the gene for a membrane-bound receptor, the fluorescent protein being chosen from the fluorescent proteins obtained or derived from autofluorescent proteins of cnidarians,
- the fusion between the gene for the fluorescent substance and the gene for the above-mentioned G protein, and the fusion between the gene for the fluorescent protein and the gene for the above-mentioned membrane-bound receptor being such that
* the properties of the membrane-bound receptor are not modified by the presence of the fluorescent protein, that is to say
* the response transduction function is not modified, with the proviso that:
* when the membrane-bound receptor is the rat glucocorticoid receptor fused at the N-terminal with, successively, a purification sequence comprising 6 histidines, a haemaglutinin epitope and a fluorescent protein and is expressed in the cell line 1471.1, the fluorescent protein is other than GFP (768 base pairs of the plasmid TU65 with the mutation S65T),
* when the membrane-bound receptor is the human glucocorticoid receptor truncated of its first 131 amino acids, fused at the C-terminal of a fluorescent protein in the sites Sal I and BamHI and is expressed in the cells Cos-1, the said fluorescent protein is other than that GFP having the following sequence Met Ser Lys Gly Glu Glu Leu Phe Thr Gly Val Val Pro Ile Leu Val Glu Leu Asp Gly Asp Val Asn Gly Gln Lys Phe Ser Val Ser Gly Glu Gly Glu Gly Asp Ala Thr Tyr Gly Lys Leu Thr Leu Lys Phe Ile Cys Thr Thr Gly Lys Leu Pro Val Pro Trp Pro Thr Leu Val Thr Thr Phe Ser Tyr Gly Val Gln Cys Phe Ser Arg Tyr Pro Asp His Met Lys Gln His Asp Phe Phe Lys Ser Ala Met Pro Glu Gly Tyr Val Gln Glu Arg Thr Ile Phe Phe Lys Asp Asp Gly Asn Tyr Lys Thr Arg Ala Glu Val Lys Phe Glu Gly Asp Thr Leu Val Asn Arg Ile Glu Leu Lys Gly Ile Asp Phe Lys Glu Asp Gly Asn Ile Leu Gly His Lys Met Glu Tyr Asn Tyr Asn Ser His Asn Val Tyr Ile Met Ala Asp Lys Pro Lys Asn Gly Ile Lys Val Asn Phe Lys Ile Arg His Asn Ile Lys Asp Gly Ser Val Gln Leu Ala Asp His Tyr Gln Gln Asn Thr Pro Ile Gly Asp Gly Pro Val Leu Leu Pro Asp Asn His Tyr Leu Ser Thr Gln Ser Ala Leu Ser Lys Asp Pro Asn Glu Lys Arg Asp His Met Ile Leu Leu Glu Phe Val Thr Ala Ala Gly Ile Thr His Gly Met Asp Glu Leu Tyr Lys,
* when the membrane-bound receptor is the rat NMDA R1 sub-unit expressed in HEK 293 cells fused at the C-terminal with a fluorescent protein, the fluorescent protein is other than that consisting of the amino acids 2-238 of wild-type GFP,
* when the membrane-bound receptor is a receptor or a fragment of a receptor for intracellular second messengers, the fluorescent protein is other than that GFP and its derivatives.

29. Kit for detecting and quantifying non-covalent interactions between a membrane-bound receptor labeled with a fluorescent protein as defined in one of claims 1 to 3, and one of its ligands labeled with a label consisting:
- either of a molecule which is capable of absorbing the light emitted by the fluorescent protein,
- or of a fluorescent substance,
the said kit comprising:
- the membrane-bound receptor fused with a fluorescent protein as defined above or a stable cell line which is capable of expressing the membrane-bound receptor fused with a fluorescent protein as defined above or a plasmid containing the nucleic acid sequence coding for the said membrane-bound receptor fused with a fluorescent protein as defined above,
- the ligand labeled with the above-mentioned label,
- the buffers and media required for the energy transfer between the above-mentioned protein and the above-mentioned ligand.

30. Kit according to claim 29, **characterized in that** the membrane-bound receptor is labeled with a fluorescent protein (No 1) and one of its ligands is labeled with a fluorescent substance corresponding to a fluorescent protein (No 2), the fluorescent protein (No 1) being chosen from the fluorescent protein EYFP or EGFP and the ligand being labeled with a fluorescent protein (No 2) ECFP, or the fluorescent protein (No 1) being ECFP and the ligand being labeled with the fluorescent protein (No 2) EYFP or EGFP, the said kit comprising:
- either a plasmid containing a nucleic acid sequence coding for the membrane-bound receptor fused with a fluorescent protein (No 1), and
* a plasmid containing a nucleic acid sequence coding for the ligand fused with a fluorescent protein (No 2), or
* a ligand fused with a fluorescent protein (No 2), obtained via a recombinant route and purified,
- or a stable cell line which is capable of expressing the membrane-bound receptor fused with a fluorescent protein (No 1), and
* a stable cell line which is capable of expressing the ligand fused with a fluorescent protein (No 2) or
* a ligand fused with a fluorescent protein (No 2), obtained via a recombinant route and purified.

31. Kit according to claim 30, **characterized in that** the membrane-bound receptor consists of a receptor coupled to the G protein and **in that** the ligand corresponds to the G protein.


### LISTE DE SEQUENCES

(1) INFORMATIONS GENERALES:
   (i) DEPOSANT:
      (A) NOM: CENTRE NATIONAL DE LA RECHERCHE SCIENTIFIQUE
      (B) RUE: 3, RUE MICHEL-ANGE
      (C) VILLE: PARIS
      (E) PAYS: FRANCE
      (F)CODE POSTAL: 75794 CEDEX 16
   (ii) TITRE DE L'INVENTION: UTILISATION D'UNE PROTEINE FLUORESCENTE POUR LA DETECTION D'INTERACTIONS ENTRE UNE PROTEINE CIBLE ET SON LIGAND
   (iii) NOMBRE DE SEQUENCES: 2
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patent In Release #1.0, Version #1.30 (OEB)
   (v) DONNEES DE LA DEMANDE ACTUELLE:
      NUMERO DE LA DEMANDE: PCT/FR98/01136
   (vi) DONNEES DE LA DEMANDE ANTERIEURE:
      (A) NUMERO DE LA DEMANDE: FR 97.06977
      (B) DATE DE DEPOT: 05-Jun-1997
(2) INFORMATIONS POUR LA SEQ ID NO: 1:
   (i) CARACTERISTIQUES DE LA SEQUENCE:
      (A) LONGUEUR: 798 paires de bases
      (B) TYPE: nucléotide
      (C) NOMBRE DE BRINS: double
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT : 1.. 798
   (xi) DESCRIPTION DE LA SEQUENCE : SEQ ID NO: 1:
   ATG GTG AGC AAG GGC GAG GAG CTG TTC ACC GGG GTG GTG CCC ATC CTG         48
   Met Val Ser Lys Gly Glu Glu Leu Phe Thr Gly Val Val Pro Ile Leu
     1               5                  10                  15
   GTC GAG CTG GAC GGC GAC GTA AAC GGC CAC AAG TTC AGC GTG TCC GGC         96
   Val Glu Leu Asp Gly Asp Val Asn Gly His Lys Phe Ser Val Ser Gly
                20                  25                  30
   GAG GGC GAG GGC GAT GCC ACC TAC GGC AAG CTG ACC CTG AAG TTC ATC        144
   Glu Gly Glu Gly Asp Ala Thr Tyr Gly Lys Leu Thr Leu Lys Phe Ile
            35                  40                  45
   TGC ACC ACC GGC AAG CTG CCC GTG CCC TGG CCC ACC CTC GTG ACC ACC 192
   Cys Thr Thr Gly Lys Leu Pro Val Pro Trp Pro Thr Leu Val Thr Thr
   50 55 60
   CTG ACC TAC GGC GTG CAG TGC TTC AGC CGC TAC CCC GAC CAC ATG AAG 240
   Leu Thr Tyr Gly Val Gln Cys Phe Ser Arg Tyr Pro Asp His Met Lys
   65 70 75 80
   CAG CAC GAC TTC TTC AAG TCC GCC ATG CCC GAA GGC TAC GTC CAG GAG 288
   Gln His Asp Phe Phe Lys Ser Ala Met Pro Glu Gly Tyr Val Gln Glu
   85 90 95
   CGC ACC ATC TTC TTC AAG GAC GAC GGC AAC TAC AAG ACC CGC GCC GAG 336
   Arg Thr Ile Phe Phe Lys Asp Asp Gly Asn Tyr Lys Thr Arg Ala Glu
   100 105 110
   GTG AAG TTC GAG GGC GAC ACC CTG GTG AAC CGC ATC GAG CTG AAG GGC 384
   Val Lys Phe Glu Gly Asp Thr Leu Val Asn Arg Ile Glu Leu Lys Gly
   115 120 125
   ATC GAC TTC AAG GAG GAC GGC AAC ATC CTG GGG CAC AAG CTG GAG TAC 432
   Ile Asp Phe Lys Glu Asp Gly Asn Ile Leu Gly His Lys Leu Glu Tyr
   130 135 140
   AAC TAC AAC AGC CAC AAC GTC TAT ATC ATG GCC GAC AAG CAG AAG AAC 480
   Asn Tyr Asn Ser His Asn Val Tyr Ile Met Ala Asp Lys Gln Lys Asn
   145 150 155 160
   GGC ATC AAG GTG AAC TTC AAG ATC CGC CAC AAC ATC GAG GAC GGC AGC 528
   Gly Ile Lys Val Asn Phe Lys Ile Arg His Asn Ile Glu Asp Gly Ser
   165 170 175
   GTG CAG CTC GCC GAC CAC TAC CAG CAG AAC ACC CCC ATC GGC GAC GGC 576
   Val Gln Leu Ala Asp His Tyr Gln Gln Asn Thr Pro Ile Gly Asp Gly
   180 185 190
   CCC GTG CTG CTG CCC GAC AAC CAC TAC CTG AGC ACC CAG TCC GCC CTG 624
   Pro Val Leu Leu Pro Asp Asn His Tyr Leu Ser Thr Gln Ser Ala Leu
   195 200 205
   AGC AAA GAC CCC AAC GAG AAG CGC GAT CAC ATG GTC CTG CTG GAG TTC 672
   Ser Lys Asp Pro Asn Glu Lys Arg Asp His Met Val Leu Leu Glu Phe
   210 215 220
   GTG ACC GCC GCC GGG ATC ACT CTC GGC ATG GAC GAG CTG TAC AAG TAC 720
   Val Thr Ala Ala Gly Ile Thr Leu Gly Met Asp Glu Leu Tyr Lys Tyr
   225 230 235 240
   TCA GAT CTC GAG CTC AAG CTT CGA ATT CTG CAG TCG ACG GTA CCG CGG 768
   Ser Asp Leu Glu Leu Lys Leu Arg Ile Leu Gln Ser Thr Val Pro Arg
   245 250 255
   GCC CGG GAT CCA CCG GAT CTA GAT AAC TGA 798
   Ala Arg Asp Pro Pro Asp Leu Asp Asn *
   260 265
(2) INFORMATIONS POUR LA SEQ ID NO: 2:
   (i) CARACTERISTIQUES DE LA SEQUENCE:
      (A) LONGUEUR: 266 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Tyr
   225                 230                 235                 240
   Ser Asp Leu Glu Leu Lys Leu Arg Ile Leu Gln Ser Thr Val Pro Arg
                   245                 250                 255
   Ala Arg Asp Pro Pro Asp Leu Asp Asn  *
               260                 265